(19) Europäisches Patentamt
European Patent Office
Office européen des brevets

(11) Veröffentlichungsnummer : **0 338 331 B1**

(12) # EUROPÄISCHE PATENTSCHRIFT

(45) Veröffentlichungstag der Patentschrift : **21.10.92 Patentblatt 92/43**

(21) Anmeldenummer : **89106023.8**

(22) Anmeldetag : **06.04.89**

(51) Int. Cl.[5] : **C07D 417/06**, C07D 401/06, C07D 207/12, C07D 207/08, C07D 207/09, C07D 405/06, C07D 403/12, A61K 31/40, A61K 31/41, A61K 31/44

(54) **1,3-Disubstituierte Pyrrolidine.**

Die Akte enthält technische Angaben, die nach dem Eingang der Anmeldung eingereicht wurden und die nicht in dieserPatentschrift enthalten sind.

(30) Priorität : **19.04.88 DE 3812989**
**15.10.88 DE 3835291**

(43) Veröffentlichungstag der Anmeldung : **25.10.89 Patentblatt 89/43**

(45) Bekanntmachung des Hinweises auf die Patenterteilung : **21.10.92 Patentblatt 92/43**

(84) Benannte Vertragsstaaten : **AT BE CH DE ES FR GB GR IT LI NL SE**

(56) Entgegenhaltungen :
**DE-A- 1 964 510**
**DE-A- 1 964 511**
**DE-A- 2 315 092**
**US-A- 3 642 803**
**CHEMICAL ABSTRACTS, Band 71, Nr. 3, 21. Juli 1969, Columbus, Ohio, US; R.L. DUNCAN et al.: "Synthesis and biologicalproperties of some 1-substituted 3-(o-methoxyphenoxy)-pyrrolidines", Seite 311, Spalte 1, Zusammenfassung Nr. 12 930g**
**CHEMICAL ABSTRACTS, Band 102, Nr. 16, 22. April 1985, Columbus, Ohio, US; M. PERSHOTTAM PRASAD et al.:"Aryloxy-N-(aminoalkyl)-1-pyrrolidine and piperidine carboxamides and carbothioamides having antiarrhythmic activity", Seite 376,Spalte 2, Zusammenfassung Nr. 137 816q**
**CHEMICAL ABSTRACTS, Band 82, Nr. 21, 26. Mai 1975, Columbus, Ohio, US; H. YAMAMOTO et al.: "Pyrrolidinobutyrophenonederivative", Seite 611, Spalte 1, Zusammenfassung Nr. 139 951x**

(56) Entgegenhaltungen :
**CHEMICAL ABSTRACTS, Band 81, Nr. 25, 23. Dezember 1974, Columbus, Ohio, US; R.F. BOSWELL et al.: "Synthesis of someN-carboxylic acid derivatives of 3-phenoxypyrrolidines, 4-phenoxypiperidines, and 3-phenoxynortropanes with muscle relaxant andanticonvulsant activities", Seite 36, Spalte 2, Zusammenfassung Nr. 163 495b**
**CHEMICAL ABSTRACTS, Band 77, Nr. 25, 18. Dezember 1972, Columbus, Ohio, US; M. NAKAO et al.: "Derivatives of1-(benzoylalkyl)pyrrolidine", Seite 400, Spalte 2, Zusammenfassung Nr. 164 517q**
**CHEMICAL ABSTRACTS, Band 70, Nr. 15, 14. April 1969, Columbus, Ohio, US; C.D. LUNSFORD et al.: "Tranquilizing and analgesic1,3-disubstituted-pyrrolidines", Seite 356, Spalte 1, Zusammenfassung Nr. 68 126w**
**CHEMICAL ABSTRACTS, Band 67, Nr. 25, 18. Dezember 1967, Columbus, Ohio, US; W.J. WELSTEAD et al.: "Aminoalkyl indoles withcentral nervous system activity", Seite 10884, Spalte 2, Zusammenfassung Nr. 115 635m**

(73) Patentinhaber : **BAYER AG**
**W-5090 Leverkusen 1 Bayerwerk (DE)**

(72) Erfinder : **Schohe, Rudolf, Dr.**
**Pahlkestrasse 5**
**W-5600 Wuppertal 1 (DE)**
Erfinder : **Seidel, Peter-Rudolf, Dr.**
**Alte Heide 5D**
**W-5000 Köln 90 (DE)**
Erfinder : **Traber, Jörg, Dr.**
**Löwenburgstrasse 12**
**W-5204 Lohmar 21 (DE)**
Erfinder : **Glaser, Thomas, Dr.**
**Köslinerstrasse 21a**
**W-5064 Rösrath (DE)**

EP 0 338 331 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft 1,3-disubstituierte Pyrrolidine, Verfahren zu ihrer Herstellung und ihre Verwendung in Arzneimitteln.

Spezielle Pyrrolidine mit Wirkung auf das zentrale Nervensystem sind aus der DE-A 1 964 510 bekannt. Insbesondere werden in der DE-A 1 964 511 1-substituierte 3-Phenoxypyrrolidine, die eine pharmakologische Wirkung auf das zentrale Nervensystem haben, beschrieben. Die Verbindungen haben muskelerschlaffende Eigenschaften (Seite 3, 1. Absatz).

Aus der US 36 42 803 ist 1-[2-(Indol-3-yl)-ethyl]-3-(2-methoxy-phenoxy)pyrrolidin bekannt, das ebenfalls eine Wirkung auf das zentrale Nervensystem hat.

In der DE-A 2 315 092 werden ebenfalls Pyrrolidine mit antipsychotischer und muskelrelaxierender Wirkung beschrieben.

Es wurden neue 1,3-disubstituierte Pyrrolidine der allgemeinen Formel (I)

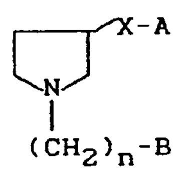

(I)

worin

A für Phenyl oder einen monocyclischen fünf- oder sechsgliedriger Hetarylrest, der ein oder zwei Stickstoff- und/oder Sauerstoff- und/oder Schwefelatome enthält, steht, an die gegebenenfalls ein bis drei aromatische gesättigte oder ungesättigte cyclische Kohlenwasserstoffe mit 5 bis 8 Kohlenstoffatomen oder fünf- oder sechs-gliedrige Heterocyclen mit einem oder zwei Stickstoff- und/oder Sauerstoff- und/oder Schwefelatomen kondensiert sind, wobei dieser Rest gegebenenfalls ein- oder zweifach durch $C_1$-$C_6$-Alkyl, $C_1$-$C_6$-Alkoxy, $C_1$-$C_6$-Alkoxycarbonyl, $C_1$-$C_6$-Alkenyloxy, Acyloxy, Benzoyloxy, Cyano, Phenyl, Benzyl, Fluor, Chlor, Brom, Trifluormethyl, Trifluormethoxy, Difluor- methoxy oder einen Rest der Formel -CO-NYZ, -NH-$SO_2$-Y', -$SO_2$-NYZ oder -NH-CO-Y substituiert ist, wobei

Y,Z gleich oder verschieden sind und für Wasserstoff oder $C_1$-$C_6$-Alkyl stehen, und

Y' für $C_1$-$C_6$-Alkyl oder Aryl mit 6 bis 12 Kohlenstoffatomen steht,

und im Fall von Stickstoffheterocyclen gegebenenfalls als N-Oxid vorliegt,

X - für -$CH_2$-O-, -O-$CH_2$- oder -O- steht,

B - für Cyano oder eine Gruppe der Formel -$COOR^1$, $CONR^2R^3$, -$SO_2NR^2R^3$, -$SO_mR^4$, $NR^5R^6$ oder -C≡C-$CH_2$-$NR^5R^6$ steht, wobei

$R^1$ - für Wasserstoff, $C_1$-$C_{11}$-Alkyl, $C_5$-$C_8$-Cycloalkyl, $C_2$-$C_{12}$-Alkenyl, $C_6$-$C_{12}$Aryl oder $C_7$-$C_{14}$-Aralkyl steht,

$R_2$ und $R_3$ gleich oder verschieden sind und

- für Wasserstoff, $C_1$-$C_{12}$-Alkyl, $C_5$-$C_8$-Cycloalkyl, $C_6$-$C_{12}$-Aryl oder $C_7$-$C_{14}$-Aralkyl stehen, wobei die Arylreste durch Halogen, $C_1$-$C_6$-Alkoxy oder $C_1$-$C_6$-Alkoxycarbonyl substituiert sein können,

$R^4$ - für $C_1$-$C_{12}$-Alkyl, $C_5$-$C_8$-Cycloalkyl, $C_6$-$C_{12}$-Aryl oder $C_7$-$C_{14}$-Aralkyl steht, wobei die Arylreste bis zu 3-fach gleich oder verschieden durch Halogen, Cyano, $C_1$-$C_6$-Alkyl, $C_1$-$C_6$-Alkoxy, Trifluormethyl oder Trifluormethoxy substituiert sein können, oder

- für eine Gruppe der Formel

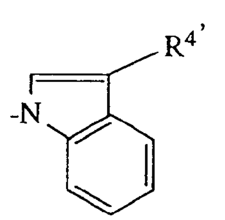

steht,

worin $R^{4'}$ Wasserstoff oder $C_1$-$C_6$-Alkyl bedeutet

m - für eine Zahl 0, 1 oder 2 steht,

$R^5$ und $R^6$ gleich oder verschieden sind und

- für Wasserstoff, $C_1$-$C_{12}$-Alkyl, $C_5$-$C_{-8}$ Cycloalkyl, $C_6$-$C_{12}$-Aryl oder $C_7$-$C_{14}$-Aralkyl stehen, wobei die Arylreste durch Halogen, Cyano, $C_1$-$C_6$-Alkyl, $C_1$-$C_6$-Alkoxy oder Trifluormethyl substituiert sein können, oder

- für eine Gruppe der Formel $-COR^7$ oder $-SO_2R^8$ stehen,

worin

$R^7$ - Wasserstoff oder

- eine Gruppe $NHR^9$ oder

- $C_1$-$C_{11}$-Alkyl, $C_5$-$C_8$-Cycloalkyl oder $C_1$-$C_{12}$-Alkoxy, oder

- $C_6$-$C_{12}$-Aryl, $C_6$-$C_{12}$-Aryloxy, $C_7$-$C_{14}$-Aralkyl, $C_6$-$C_{12}$-Aralkoxy oder einen fünf-oder sechsgliedrigen Heteroaryl-Ring mit ein oder zwei Sauerstoff- und/oder Stickstoff- und/oder Schwefelatomen bedeutet, wobei die genannten Reste bis zu 3-fach gleich oder verschieden durch $C_1$-$C_6$-Alkyl, $C_1$-$C_6$-Alkoxy, $C_1$-$C$-$_6$-Alkylthio, Halogen, Cyano, Trifluormethyl, Trifluormethoxy, Trifluormethylthio, Amino, $C_1$-$C_6$-Alkyl-amino oder $C_1$-$C_6$-Dialkyl-amino substituiert sein können,

$R^8$ - $C_5$-$C_8$-Cycloalkyl, oder

- $C_1$-$C_{12}$-Alkyl, das durch Cyano, Halogen, $C_1$-$C_{12}$-Alkoxy oder $C_1$-C-$_{12}$Alkoxy-carbonyl substituiert sein kann, oder

- $C_6$-$C_{12}$-Aryl, $C_7$-$C_{14}$-Aralkyl oder einen fünf- oder sechsgliedrigen Heteroaryl-Ring mit ein oder zwei Sauerstoff- und/oder Stickstoffund/oder Schwefelatomen bedeutet, wobei die genannten Reste bis zu 3-fach gleich oder verschieden durch $C_1$-$C_6$-Alkyl, $C_1$-$C_6$-Alkoxy, $C_1$-$C_6$-Alkylthio, Halogen, Cyano, Trifluormethyl, Trifluormethoxy, Trifluormethylthio, Nitro, Amino, $C_1$-$C_6$-Alkyl-amino oder $C_1$-$C_6$-Dialkyl-amino substituiert sein können, oder

- eine Gruppe $NR^2R^3$ bedeutet, wobei

$R^2$ und $R^3$ die oben angegebene Bedeutung haben und

$R^9$ - Wasserstoff, oder

- $C_5$-$C_8$-Cycloalkyl, oder

- gegebenenfalls durch Cyano, Fluor, Chlor oder Brom substituiertes $C_1$-$C_{12}$-Alkyl, oder

- $C_6$-$C_{12}$-Aryl, $C_7$-$C_{14}$-Aralkyl, einen fünf- oder sechsgliedrigen Heteroaryl- Ring mit ein oder zwei Sauerstoff- und/oder Stickstoff- und/oder Schwefelatomen bedeutet, wobei die Arylreste bis zu 3-fach gleich oder verschieden durch $C_1$-$C_6$-Alkyl, $C_1$-$C_6$-Alkoxy, $C_1$-$C_6$-Alkylthio, Halogen, Cyano, Trifluormethyl, Trifluormethoxy, Trifluormethylthio, Nitro, Amino, $C_1$-$C_6$-Alkyl-amino oder $C_1$-$C_6$-Dialkylamino substituiert sein können,

oder wobei

$R^5$ und $R^6$ zusammen mit dem Stickstoffatom einen Ring der Reihe

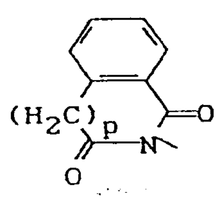 , 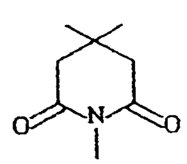 , 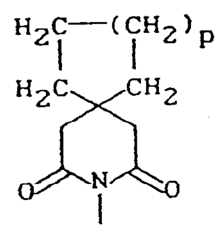 ,

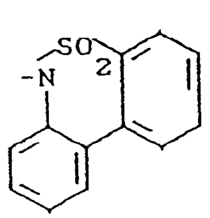 , 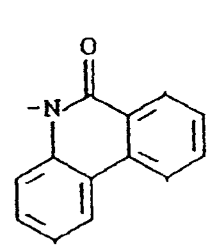 , 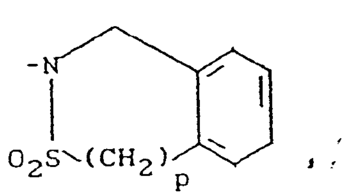 ,

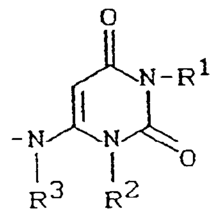, 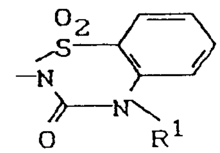, 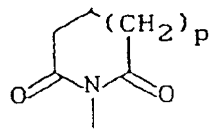, 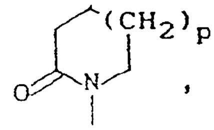,

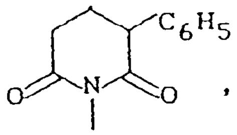, 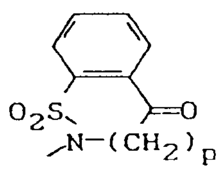, 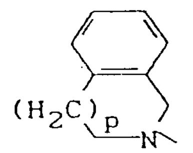, 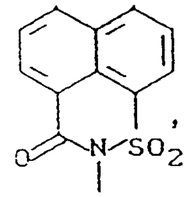,

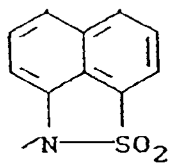, 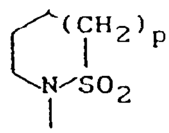 oder 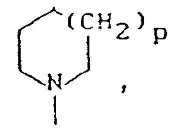,

bilden, worin

p - eine Zahl 0, 1 oder 2 bedeutet,

$R^1$, $R^2$ und $R^3$ die oben angegebene Bedeutung haben, und

n - eine Zahl von 1 bis 10 bedeutet und deren Salze gefunden.

Die erfindungsgemäßen Stoffe zeigen überraschenderweise eine überlegene Wirkung auf das Zentralnervensystem und können zur therapeutischen Behandlung bei Menschen und Tieren verwendet werden.

Die erfindungsgemäßen Stoffe haben mehrere asymmetrische Kohlenstoffatome und können daher in verschiedenen stereochemischen Formen vorliegen. Darüberhinaus können Verbindungen mit einer Sulfoxidgruppe ebenfalls in unterschiedlichen stereochemischen Formen vorliegen, Sowohl die einzelnen Isomeren, als auch deren Mischungen sind Gegenstand der Erfindung. Beispielsweise seien folgende isomeren Formen der 1,3-disubstituierten Pyrrolidine genannt:

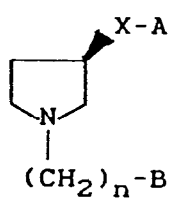     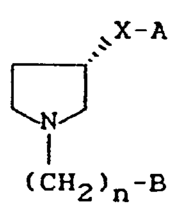

Die erfindungsgemäßen 1,3-disubstituierten Pyrrolidine können auch in Form ihrer Salze vorliegen. Im allgemeinen seien hier Salze mit anorganischen oder organischen Säuren genannt.

Im Rahmen der vorliegenden Erfindung werden physiologisch unbedenkliche Salze bevorzugt. Physiologisch unbedenkliche Salze der 1,3-disubstituierten Pyrrolidine können Salze der erfindungsgemäßen Stoffe mit Mineralsäuren, Carbonsäuren oder Sulfonsäuren sein. Bevorzugt sind beispielsweise Salzsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Oxalsäure, Malonsäure, Bernsteinsäure, Maleinsäure, Fumarsäure, Äpfelsäure, Weinsäure, Zitronensäure oder Benzoesäure.

Der Rest A kann ein Phenyl- oder Hetarylrest sein, der gegebenenfalls mit aromatischen, gesättigten, ungesättigten oder cyclischen Kohlenwasserstoffen oder Heterocyclen kondensiert ist. Ein Hetarylrest im Rahmen der Erfindung ist im allgemeinen ein monocyclischer fünfoder sechsgliedriger Ring mit einem oder zwei, bevorzugt einem Stickstoff-, Sauerstoff- oder Schwefelatom als Heteroatom. Beispielsweise seien Pyrrolyl,

Furyl, Thienyl, Oxazolyl, Thiazolyl, Isoxazolyl, Isothiazolyl, Pyridyl, Pyranyl, Thiopyranyl, Pyridazinyl, Pyrimidinyl, Pyrazinyl genannt.

An die Phenyl- und Hetarylreste können weitere Ringe kondensiert sein.

Als aromatische Ringe seien Aryl ($C_6$ bis $C_{12}$), bevorzugt Phenyl genannt.

Als ungesättigte Ringe seien fünf bis acht, bevorzugt fünf- oder sechsgliedrige, Kohlenwasserstoffe mit einer oder zwei, bevorzugt einer, Doppelbindung genannt. Beispielsweise sei Cyclopenten genannt.

Als gesättigte Ringe seien 4- bis 8-gliedrige, cyclische Kohlenwasserstoffe, bevorzugt Cyclopentyl und Cyclohexyl, genannt.

Als Reste A seien beispielsweise genannt:

Phenyl, 2-Fluorphenyl, 2-Methylphenyl, 2-Methoxyphenyl, 3-Methoxyphenyl, 4-Methoxyphenyl, 2-Cyano-6-methoxyphenyl, Alkenyloxyphenyl, 2-Aminocarbonylphenyl, 1-Naphthyl, 2-Naphthyl, 1-Tetralyl, 4-Indolyl, 1-Isochinolyl, 2-Chinolyl und 8-Chinolyl.

Bevorzugt werden 1,3-disubstituierte Pyrrolidine der allgemeinen Formel (I),

worin

A - für Phenyl oder einen monocyclischen fünf- oder sechsgliedrigen Hetarylrest, der ein oder zwei Stickstoff- und/oder Sauerstoff- und/oder Schwefelatome enthält, steht, an die gegebenenfalls ein bis drei aromatische, gesättigte oder ungesättigte cyclische Kohlenwasserstoffe mit 5 bis 8 Kohlenstoffatomen oder fünf- oder sechsgliedrige Heterocyclen mit einem oder zwei Stickstoff- und/oder Sauerstoff- und/oder Schwefelstomen kondensiert sind, wobei dieser Rest gegebenenfalls ein- oder zweifach durch $C_1$-$C_6$-Alkyl, $C_1$-$C_6$-Alkoxy, $C_1$-$C_6$-Alkoxycarbonyl, $C_1$-$C_6$-Alkenyloxy, Acyloxy, Benzoyloxy, Cyano, Phenyl, Benzyl, Fluor, Chlor, Brom, Trifluormethyl, Trifluormethoxy, Difluormethoxy oder einen Rest der Formel -CO-NYZ, -NH-$OS_2$-Y′, -$SO_2$-NYZ oder -NH-CO-Y substituiert ist,

wobei

Y,Z gleich oder verschieden sind und für Wasserstoff, oder $C_1$-$C_6$-Alkyl stehen und

Y′ für $C_1$-$C_6$-Alkyl oder Aryl mit 6 bis 12 und im Fall von Stickstoffheterocyclen gegebenenfalls als N-Oxid vorliegt,

X - für -$CH_2$-O-, -O-$CH_2$- oder -O- steht,

B - für Cyano oder eine Gruppe der Formel -$COOR^1$, $CONR^2R^3$, -$SO_2NR^2R^3$, -$SO_mR^4$, $NR^5R^6$ oder -C≡C-$CH_2$-$NR^5R^6$ steht, wobei

$R^1$ - für Wasserstoff, $C_1$-$C_6$-Alkyl oder Phenyl steht,

$R^2$ und $R^3$ gleich oder verschieden sind und

- für Wasserstoff, $C_1$-$C_6$-Alkyl oder Phenyl stehen, das seinerseits durch Fluor, Chlor, Brom, $C_1$-$C_6$-Alkoxy oder $C_1$-$C_6$-Alkoxycarbonyl substituiert sein kann,

$R^4$ - für $C_1$-$C_6$-Alkyl, oder

- für Phenyl steht, das bis zu zweifach gleich oder verschieden durch Fluor, Chlor, Brom, $C_1$-$C_6$-Alkyl oder $C_1$-$C_6$-Alkoxy substituiert sein Kann, oder

- für eine Gruppe der Formel

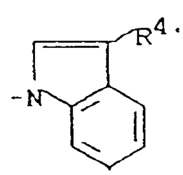

steht,

worin $R^{4\prime}$ Wasserstoff oder $C_1$-$C_6$-Alkyl bedeutet,

m - für eine Zahl 0, 1 oder 2 steht,

$R^5$ und $R^6$ gleich oder verschieden sind und

- für Wasserstoff, $C_1$-$C_6$-Alkyl, Phenyl oder Benzyl stehen, wobei die Phenylreste durch Fluor, Chlor, Brom, $C_1$-$C_6$-Alkyl, $C_1$-$C_6$-Alkoxy oder Trifluormethyl substituiert sein können, oder

- für eine Gruppe der Formel -$COR^7$ oder -$SO_2R^8$ stehen,

worin

$R^7$ - Wasserstoff bedeutet, oder

- eine Gruppe $NHR^9$ bedeutet, oder

- $C_1$-$C_6$-Alkyl oder $C_1$-$C_6$-Alkoxy bedeutet, oder

- gegebenenfalls durch $C_1$-$C_6$-Alkyl, $C_1$-$C_6$-Alkoxy, Fluor, Chlor, Brom, Trifluormethyl, Dimethylamino oder Diethylamino substituiertes Phenyl, Benzyl, Benzyloxy, Thienyl, Furyl, Pyridyl, Pyrimidyl,

Chinolyl, Isochinolyl, Benzothiazolyl, Benzoxazolyl, Thiazolyl, Oxazolyl, Isoxazolyl oder Isothiazolyl bedeutet,

$R^8$ - Cyclopropyl, Cyclopentyl, Cyclohexyl, oder

- gegebenenfalls durch Cyano, Fluor, Chlor, Brom, Trifluormethyl oder $C_1$-$C_6$-Alkoxy-carbonyl substituiertes $C_1$-$C_6$-Alkyl bedeutet, oder

- gegebenenfalls durch $C_1$-$C_6$-Alkyl, $C_1$-$C_6$-Alkoxy, Fluor, Chlor, Brom, Nitro, Trifluormethyl, Dimethylamino oder Diethylamino substituiertes Phenyl, Naphthyl, Benzyl, Thienyl, Furyl, Pyrimidyl, Pyridyl, Chinolyl, Isochinolyl, Benzothiazolyl, Benzoxazolyl, Thiazolyl, Oxazolyl, Isoxazolyl oder Isothiazolyl bedeutet, oder

- eine Gruppe $NR^2R^3$ bedeutet,

wobei

$R^2$ und $R^3$ die oben angegebene Bedeutung haben, und

$R^9$ - gegebenenfalls durch Cyano, Fluor, Chlor oder Brom substituiertes $C_1$-$C_6$-Alkyl bedeutet, oder

- gegebenenfalls durch $C_1$-$C_6$-Alkyl, $C_1$-$C_6$-Alkoxy, Fluor, Chlor, Brom, Trifluormethyl, Dimethylamino oder Diethylamino substituiertes Phenyl, Benzyl, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,

oder

$R^5$ und $R^6$ zusammen mit dem Stickstoffatom einen Ring der Reihe

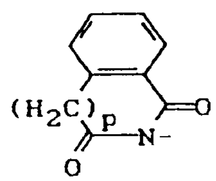 , 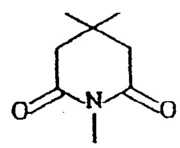 , 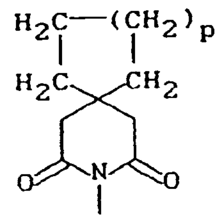 ,

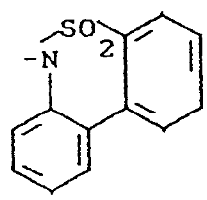 , 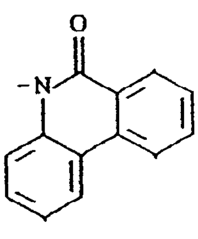 , 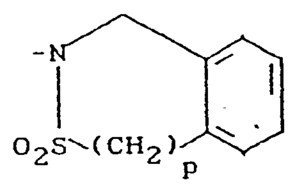 ,

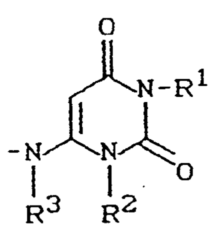 , 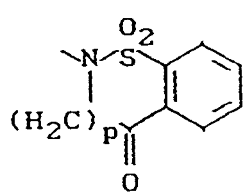 , 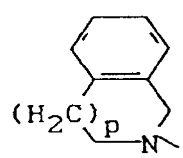 , 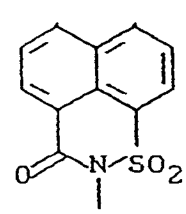

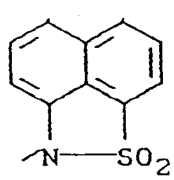 , 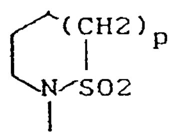 , 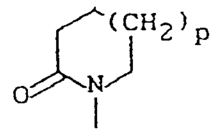 ,

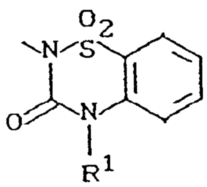 oder 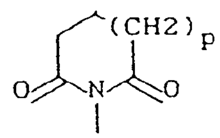 bilden,

worin

P - eine Zahl 0, 1 oder 2 bedeutet,

$R^1$, $R^2$ und $R^3$ die angegebene Bedeutung haben und

n - eine Zahl 1 bis 8 bedeutet, und deren Salze.

Im besonderen bevorzugt sind solche Verbindungen der allgemeinen Formel (I),

bei denen

A - für gegebenenfalls gleich oder verschieden durch $C_1$-$C_6$-Alkyl, $C_1$-$C_6$-Alkoxy, $C_1$-$C_6$-Alkoxycarbonyl, $C_1$-$C_6$-Alkenyloxy, Acyloxy, Benzoyloxy, Cyano, Phenyl, Benzyl, Sulfonylamino, Sulfamoyl ($C_1$ bis $C_6$), Carbamoyl, Carbonylamino, Fluor, Chlor, Brom, Trifluormethyl, Trifluormethoxy, Difluormethoxy, substituiertes Phenyl oder Naphthyl, Furan, Thiophen, Isoxazol, Pyridin, Pyrimidin, Indol, Indazol, Benzofuran, Benzisoxazol, Chinolin, Isochinolin, Tetralin, Inden, Chroman, Dihydrobenzodioxin, Dihydroindol, Tetrahydrochinolin oder Dihydrobenzofuran steht,

X - für $-O-CH_2-$, $-CH_2-O-$ oder -O- steht,

B - für Cyano, oder

- eine Gruppe der Formel $-CONR^2R^3$, $-NR^5R^6$, $-SO_mR^4$, $-C\equiv C-CH_2-NR^5R^6$, $-SO_2NR^2R^3$, $COOR^1$ steht, wobei

$R^1$ - Wasserstoff, Methyl, Ethyl oder Phenyl bedeutet,

$R^2$ und $R^3$ gleich oder verschieden sind und

- Wasserstoff, Methyl, Ethyl, Propyl, Isopropyl, Butyl, Isobutyl bedeuten oder Phenyl bedeuten, das durch Methoxycarbonyl substituiert sein kann

$R^4$ - für Methyl oder Ethyl steht oder

- für eine Gruppe der Formel

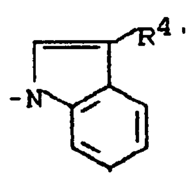

steht,

worin $R^{4'}$ Wasserstoff oder Methyl bedeutet,

$R^5$ und $R^6$ gleich oder verschieden sind, und

- Wasserstoff, Methyl, Ethyl, Propyl, Isopropyl, Butyl, Isobutyl, oder
- gegebenenfalls durch Fluor, Chlor, Methyl oder Methoxy substituiertes Phenyl, oder
- eine Gruppe $-COR^7$ oder $-SO_2R^8$ bedeuten,

worin

$R^7$ - für eine Gruppe $NHR^9$ steht, oder

- für Methyl, Ethyl, Propyl, Isopropyl, Methoxy, Ethoxy, Propoxy, Isopropoxy, tert.Butoxy oder
- für gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl steht,

$R^8$ - für gegebenenfalls durch Fluor, Chlor, Methoxycarbonyl, Ethoxycarbonyl, Propoxycarbonyl, Isopropoxycarbonyl, Butoxycarbonyl, Isobutoxycarbonyl oder tert.-Butoxycarbonyl, substituiertes Methyl, Ethyl, Propyl, Isopropyl, Butyl oder Isobutyl steht, oder

- für gegebenenfalls durch Methyl, Ethyl, Propyl, Isopropyl, Methoxy, Fluor, Chlor, Nitro substituiertes Phenyl, Napthyl, Thienyl, Furyl, Pyridyl, Pyrimidyl, Chinolyl oder Isochinolyl steht, oder
- für eine Gruppe $NR^2R^3$ steht, wobei

$R^2$ und $R^3$ die oben angegebene Bedeutung haben,

$R^9$ - gegebenenfalls durch Fluor oder Chlor substituiertes Methyl, Ethyl, Propyl, Isopropyl, Butyl, Isobutyl, Pentyl, Isopentyl, Hexyl oder Isohexyl bedeutet, oder

- Phenyl bedeutet, das durch Fluor, Chlor, Methyl oder Methoxy substituiert sein kann, oder
$R^5$ und $R^6$ gemeinsam mit dem Stickstoffatom einen Ring der Reihe

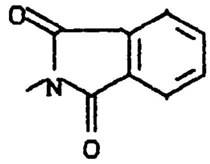 , 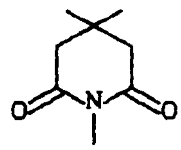 , 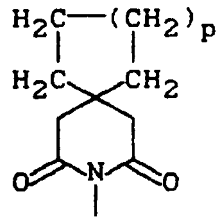 ,

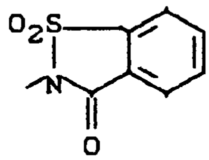 , 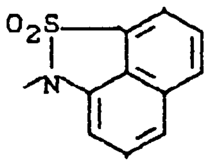 , 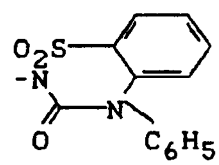 ,

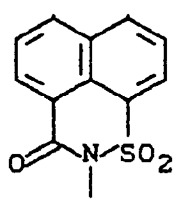 , 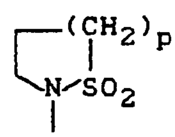 , 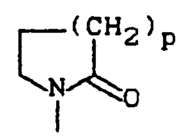 ,

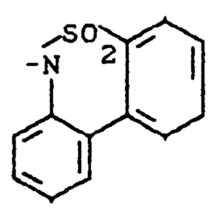 , 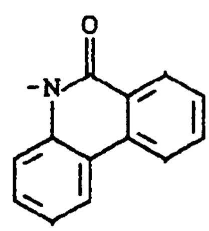 , 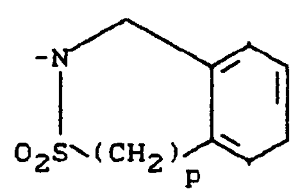 ,

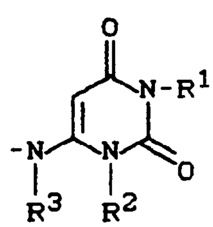 oder 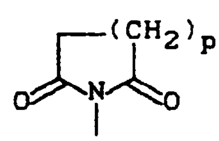

bilden, worin

p - eine Zahl 1 oder 2 bedeutet

$R^1$, $R^2$ und $R^3$ die oben angegebene Bedeutung haben und

n - eine Zahl 1 bis 6 bedeutet und deren Salze.

Beispielsweise seien die folgenden 1,3-disubstituierten Pyrrolidine genannt:

3-(2-Methoxyphenoxy)-1-[5-(1,1-dioxido-3-oxo-2,3-dihydro-benzisothiazol-2-yl)pentyl]-pyrrolidin

3-(2-Methoxyphenoxy)-1-[4-(1,1-dioxido-3-oxo-2,3-dihydro-benzisothiazol-2-yl)butyl]-pyrrolidin

3-(2-Methoxyphenoxy)-1-[3-(1,1-dioxido-3-oxo-2,3-dihydro-benzisothiazol-2-yl)propyl]-pyrrolidin

3-(2-Methoxyphenoxy)-1-[2-(1,1-dioxido-3-oxo-2,3-dihydro-benzisothiazol-2-yl)ethyl]-pyrrolidin
3-(3-Methoxyphenoxy)-1-[4-(1,1-dioxido-3-oxo-2,3-dihydro-benzisothiazol-2-yl)butyl]-pyrrolidin
3-(3-Methoxyphenoxy)-1-[5-(1,1-dioxido-3-oxo-2,3-dihydro-benzisothiazol-2-yl)pentyl]-pyrrolidin
3-(3-Methoxyphenoxy)-1-[3-(1,1-dioxido-3-oxo-2,3-dihydro-benzisothiazol-2-yl)propyl]-pyrrolidin
3-(3-Methoxyphenoxy)-1-[2-(1,1-dioxido-3-oxo-2,3-dihydro-benzisothiazol-2-yl)ethyl]-pyrrolidin
3-(4-Methoxyphenoxy)-1-[4-(1,1-dioxido-3-oxo-2,3-dihydro-benzisothiazol-2-yl)butyl]-pyrrolidin
3-(4-Methoxyphenoxy)-1-[5-(1,1-dioxido-3-oxo-2,3-dihydro-benzisothiazol-2-yl)pentyl]-pyrrolidin
3-(4-Methoxyphenoxy)-1-[3-(1,1-dioxido-3-oxo-2,3-dihydro-benzisothiazol-2-yl)propyl]-pyrrolidin
3-(4-Methoxyphenoxy)-1-[2-(1,1-dioxido-3-oxo-2,3-dihydro-benzisothiazol-2-yl)ethyl]-pyrrolidin
3-(1-Naphthyloxy)-1-[4-(1,1-dioxido-3-oxo-2,3-dihydro-benzisothiazol-2-yl)butyl]-pyrrolidin
3-(1-Naphthyloxy)-1-[5-(1,1-dioxido-3-oxo-2,3-dihydro-benzisothiazol-2-yl)pentyl]-pyrrolidin
3-(1-Naphthyloxy)-1-[3-(1,1-dioxido-3-oxo-2,3-dihydro-benzisothiazol-2-yl)propyl]-pyrrolidin
3-(1-Naphthyloxy)-1-[2-(1,1-dioxido-3-oxo-2,3-dihydro-benzisothiazol-2-yl)ethyl]-pyrrolidin
3-(2-Naphthyloxy)-1-[4-(1,1-dioxido-3-oxo-2,3-dihydro-benzisothiazol-2-yl)butyl]-pyrrolidin
3-(2-Naphthyloxy)-1-[5-(1,1-dioxido-3-oxo-2,3-dihydro-benzisothiazol-2-yl)pentyl]-pyrrolidin
3-(2-Naphthyloxy)-1-[3-(1,1-dioxido-3-oxo-2,3-dihydro-benzisothiazol-2-yl)propyl]-pyrrolidin
3-(2-Naphthyloxy)-1-[2-(1,1-dioxido-3-oxo-2,3-dihydro-benzisothiazol-2-yl)ethyl]-pyrrolidin
3-(2-Cyanophenoxy)-1-[4-(1,1-dioxido-3-oxo-2,3-dihydro-benzisothiazol-2-yl)butyl]-pyrrolidin
3-(2-Carbamoylphenoxy)-1-[4-(1,1-dioxido-3-oxo-2,3-dihydro-benzisothiazol-2-yl)butyl]-pyrrolidin
3-(2-Cyano-6-methoxy-phenoxy)-1-[4-(1,1-dioxido-3-oxo-2,3-dihydro-benzisothiazol-2-yl)butyl]-pyrrolidin
3-[(2-Fluorphenyl)-methoxy]-1-[4-(1,1-dioxido-3-oxo-2,3-dihydro-benzisothiazol-2-yl)butyl]-pyrrolidin
3-(2-Methoxy-phenoxymethyl)-1-[4-(1,1-dioxido-3-oxo-2,3-dihydro-benzisothiazol-2-yl)butyl]-pyrrolidin
3-(1-Naphthylmethoxy)-1-[4-(1,1-dioxido-3-oxo-2,3-dihydro-benzisothiazol-2-yl)butyl]-pyrrolidin
3-(2-Naphthylmethoxy)-1-[4-(1,1-dioxido-3-oxo-2,3-dihydro-benzisothiazol-2-yl)butyl]-pyrrolidin
3-(1-Carbamoyl-2-naphthyloxy)-1-[4-(1,1-dioxido-3-oxo-2,3-dihydro-benzisothiazol-2-yl)butyl]-pyrrolidin
3-(2-Carbamoyl-1-naphthyloxy)-1-[4-(1,1-dioxido-3-oxo-2,3-dihydro-benzisothiazol-2-yl)butyl]-pyrrolidin
3-[4-(1-Methyl)indolyl-oxy]-1-[4-(1,1-dioxido-3-oxo-2,3-dihydro-benzisothiazol-2-yl)butyl]-pyrrolidin
3-(2-Chinolinyl-oxy)-1-[4-(1,1-dioxido-3-oxo-2,3-dihydro-benzisothiazol-2-yl)butyl]-pyrrolidin
3-(1-Isochinolinyl-oxy)-1-[4-(1,1-dioxido-3-oxo-2,3-dihydro-benzisothiazol-2-yl)butyl]-pyrrolidin
3-(5,6,7,8-Tetrahydro-1-naphthyloxy)-1-[4-(1,1-dioxido-3-oxo-2,3-dihydro-benzisothiazol-2-yl)butyl]-pyrrolidin
1-[3-(4-Fluorphenyl)sulfonamido-propyl]-3-(2-methoxyphenoxy)-pyrrolidin
1-[2-(4-Fluorphenyl)sulfonamido-ethyl]-3-(2-methoxyphenoxy)-pyrrolidin
1-[4-(4-Fluorphenyl)sulfonamido-butyl]-3-(2-methoxyphenoxy)-pyrrolidin
1-[3-N-Phenyl-(methyl)sulfonamido-propyl]-3-(2-methoxyphenoxy)-pyrrolidin
1-[3-N-Methyl-(4-fluorphenyl)sulfonamido-propyl]-3-(2-methoxyphenoxy)-pyrrolidin
1-[4-N-Methyl-(4-fluorphenyl)sulfonamido-butyl]-3-(2-methoxyphenoxy)-pyrrolidin
1-[3-(4-Fluorphenyl)sulfonamido-propyl]-3-(1-naphthyloxy)-pyrrolidin
1-[2-(4-Fluorphenyl)sulfonamido-ethyl]-3-(1-naphthyloxy)-pyrrolidin
1-[4-(4-Fluorphenyl)sulfonamido-butyl]-3-(1-naphthyloxy)-pyrrolidin
1-[3-N-methyl-(4-fluorphenyl)sulfonamido-propyl]-3-(1-naphthyloxy)-pyrrolidin
1-[2-N-Methyl-(4-fluorphenyl)sulfonamido-ethyl]-3-(1-naphthyloxy)-pyrrolidin
1-[4-N-Methyl-(4-fluorphenyl)sulfonamido-butyl]-3-(1-naphthyloxy)-pyrrolidin
1-[3-(1,1-Dioxido-2H-naphth[1,8-cd]isothiazol-2-yl)propyl]-3-(2-methoxyphenoxy)-pyrrolidin
1-[2-(1,1-Dioxido-2H-naphth[1,8-cd]isothiazol-2-yl)ethyl]-3-(2-methoxyphenoxy)-pyrrolidin
1-[4-(1,1-Dioxido-2H-naphth[1,8-cd]isothiazol-2-yl)butyl]-3-(2-methoxyphenoxy)-pyrrolidin
1-(3-Aminopropyl)-3-(2-methoxyphenoxy)-pyrrolidin
1-(2-Aminoethyl)-3-(2-methoxyphenoxy)-pyrrolidin
1-[3-(1,1-Dioxido-2H-naphth[1,8-cd]isothiazol-2-yl)propyl]-3-(1-naphthyloxy)-pyrrolidin
1-[2-(1,1-Dioxido-2H-naphth[1,8-cd]isothiazol-2-yl)ethyl]-3-(1-naphthyloxy)-pyrrolidin
1-[4-(1,1-Dioxido-2H-naphth[1,8-cd]isothiazol-2-yl)butyl]-3-(2-naphthyloxy)-pyrrolidin
1-[3-(4,4-Dimethyl-2,6-dioxo-piperidin-1-yl)propyl]-3-(2-methoxyphenoxy)-pyrrolidin
1-[2-(4,4-Dimethyl-2,6-dioxo-piperidin-1-yl)ethyl]-3-(2-methoxyphenoxy)-pyrrolidin
1-[4-(4,4-Dimethyl-2,6-dioxo-piperidin-1-yl)butyl]-3-(2-methoxyphenoxy)-pyrrolidin
1-[3-(1,1-Dioxido-3,4-dihydro-4-phenyl-2H-1,2,4-benzothiadiazin-3-on-2-yl)propyl]-3-(2-methoxyphenoxy)-pyrrolidin
1-[2-(1,1-Dioxido-3,4-dihydro-4-phenyl-2H-1,2,4-benzothiadiazin-3-on-2-yl)-ethyl]-3-(2-methoxyphenoxy)-pyrrolidin
1-[4-(1,1-Dioxido-3,4-dihydro-4-phenyl-2H-1,2,4-benzothiadiazin-3-on-2-yl)butyl]-3-(2-methoxyphenoxy)-pyrrolidin
1-(2-Cyanoethyl)-3-(2-methoxyphenoxy)-pyrrolidin
1-Cyanomethyl-3-(2-methoxyphenoxy)-pyrrolidin
3-(1-Naphthyloxy)-1-[4-(2-oxopyrrolidin-1-yl)but-2-in-1-yl]-pyrrolidin
1-[2-(N,N-Dimethylsulfamoyl)ethyl]-3-(2-methoxyphenoxy)-pyrrolidin
1-[2-(N,N-Diethylsulfamoyl)ethyl]-3-(2-methoxyphenoxy)-pyrrolidin
1-[2-(N-(2-methoxycarbonyl)phenylsulfamoyl)ethyl]-3-(2-methoxyphenoxy)-pyrrolidin
R(-)-3-(2-Methoxyphenoxy)-1-[4-(1,1-dioxido-3-oxo-2,3-dihydro-benzisothiazol-2-yl)butyl]-pyrrolidin
S(+)-3-(2-Methoxyphenoxy)-1-[4-(1,1-dioxido-3-oxo-2,3-dihydrobenzisothiazol-2-yl)butyl]-pyrrolidin
3-(2-Methoxyphenoxy)-1-[4-(1,1-dioxido-2,3-dihydrobenzisothiazol-2-yl)butyl]-pyrrolidin
3-(2-Methoxyphenoxy)-1-[2-(3-methylindol-1-yl)-sulfonylethyl]-pyrrolidin
3-(2-Methoxyphenoxy)-1-[4-(6(5H)-phenanthridinon-5-yl)butyl]-pyrrolidin
1-[4-(5,5-Dioxido-6H-dibenzo[c,e][1,2]thiazin-6-yl)butyl]-3-(2-methoxyphenoxy)-pyrrolidin
1-[3-(1,3-Dimethyl-uracil-6-yl)aminopropyl]-3-(2-methoxyphenoxy)-pyrrolidin
1-[2-(Prop-2-enyloxy)phenoxy]-1-[4-(1,1-dioxido-3-oxo-2,3-dihydrobenzisothiazol-2-yl)butyl]-pyrrolidin-

Besonders bevorzugt werden:

3-(2-Methoxyphenoxy)-1-[4-(1,1-dioxido-3-oxo-2,3-dihydro-benzisothiazol-2-yl)butyl]-pyrrolidin
3-(3-Methoxyphenoxy)-1-[4-(1,1-dioxido-3-oxo-2,3-dihydro-benzisothiazol-2-yl)butyl]-pyrrolidin
3-(1-Naphthyl)-1-[4-(1,1-dioxido-3-oxo-2,3-dihydro-benzisothiazol-2-yl)butyl]-pyrrolidin
3-(2-Cyano-6-methoxy-phenoxy)-1-[4-(1,1-dioxido-3-oxo-2,3-dihydro-benzisothiazol-2-yl)butyl]-pyrrolidin
1-[2-(4-Fluorphenyl)sulfonamido-ethyl]-3-(2-methoxyphenoxy)-pyrrolidin
1-[3-(4-Fluorphenyl)sulfonamido-propyl]-3-(2-methoxyphenoxy)-pyrrolidin
1-[4-(1,1-Dioxido-2H-naphth[1,8-cd]isothiazol-2-yl}butyl]-3-(2-methoxyphenoxy)-pyrrolidin
1-[4-(1,1-Dioxido-2H-naphth[1,8-cd]isothiazol-2-yl}butyl]-3-(1-naphthyloxy)-pyrrolidin
R(-)-3-(2-Methoxyphenoxy)-1-[4-(1,1-dioxido-3-oxo-2,3-dihydrobenzisothiazol-2-yl)butyl]pyrrolidin
3-(2-Methoxyphenoxy)-1-[4-(2,3-dihydro-benzisothiazol-2-yl)butyl]-pyrrolidin

Weiterhin wurde ein Verfahren zur Herstellung der erfindungsgemäßen 1,3-disubstituiertern Pyrrolidine der allgemeinen Formel (I)

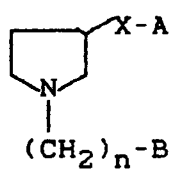

(I) ,

worin
A, B, X und n die oben genannte Bedeutung haben,
gefunden
das dadurch gekennzeichnet ist, daß man
3-substituierte Pyrrolidine der allgemeinen Formel (II)

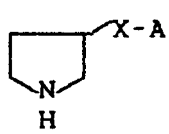

(II)

in welcher A und X die oben angegebene Bedeutung besitzen,
oder ihre Salze
in einer ersten Stufe mit Alkylderivaten der allgemeinen Formel (III)

$$R-(CH_2)_n-B' \qquad (III)$$

worin
n - die oben angegebene Bedeutung hat,
B′ dem Bedeutungsumfang von B entspricht, wobei jedoch $R^5$ und $R^6$ nicht gleichzeitig für Wasserstoff oder gleichzeitig für Wasserstoff und Alkyl oder Aryl stehen,

R - für Chlor, Brom, Iod, Methylsulfonyloxy, Phenylsulfonyloxy, Tolylsulfonyloxy, Trifluoracetoxy oder Trifluormethylsulfonyloxy steht,

in inerten Lösemitteln, gegebenenfalls in Anwesenheit von Basen, umsetzt,

dann gegebenenfalls funktionelle Gruppen durch Reduktion, Oxidation, Hydrolyse oder Umsetzung mit elektrophilen oder nucleophilen Reagentien in andere funktionelle Gruppen überführt

und dann, im Fall der Herstellung der Salze, gegebenenfalls mit der entsprechenden Säure umsetzt.

Die erste Stufe des erfindungsgemäßen Verfahrens (im folgenden Methode M 1 genannt) kann beispielhaft durch das folgende Reaktionsschema beschrieben werden:

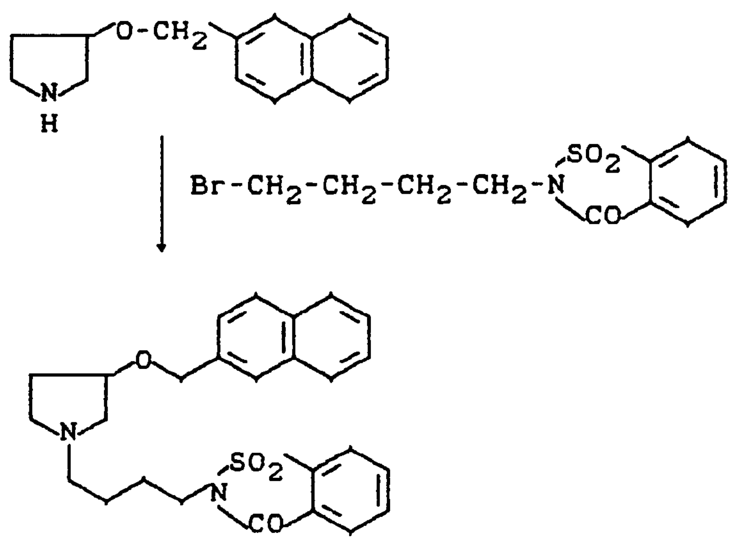

Eine gegebenenfalls nachfolgende Umsetzung (im folgenden Methode M 2 genannt) sei durch folgendes Reaktionsschema beispielhaft erläutert, wobei das Nitril nach M 1 hergestellt werden kann:

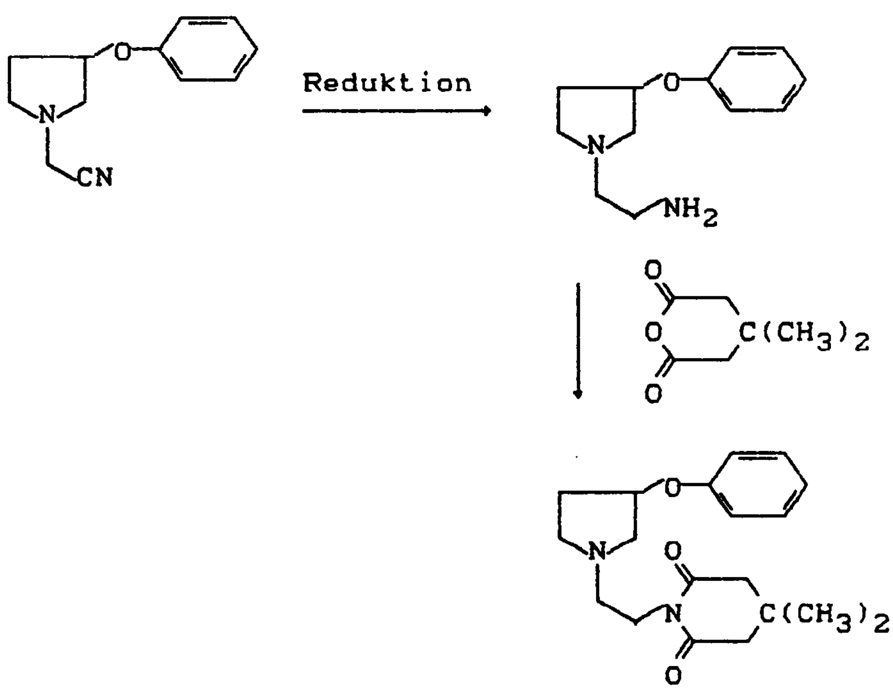


Die eingesetzten Alkylverbindungen der allgemeinen Formel (III) sind bekannt oder können nach bekannten Methoden hergestellt werden (z.B. J. March; "Advanced Organic Chemistry", Second edition, s. 1170, 1189).

Als inerte Lösemittel eignen sich für das Verfahren nach Methode M 1 die üblichen organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Butylmethylether, Dioxan, Tetrahydrofuran, Glykoldimethylether oder Diethylenglykoldimethylether, oder Halogenkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen, oder Amide wie Dimethylformamid oder Hexamethylphosphorsäuretriamid. Außerdem ist es möglich, Gemische der genannten Lösemittel zu verwenden.

Die sich bei der Reaktion bildenden Säuren können durch den Einsatz überschüssigen Pyrrolidins (II) oder durch die Verwendung von Basen gebunden werden. Als Basen können Alkali- und Erdalkalicarbonate wie Natriumcarbonat, Kaliumcarbonat, Calciumcarbonat, Hydrogencarbonate wie Natriumhydrogencarbonat, Metalloxide wie Silberoxid, oder andere anorganische Verbindungen wie Silbercarbonat dienen. Auch organische Amine können eingesetzt werden. Beispielhaft seien Triethylamin, Diisopropylamin, 1,8-Bis-(dimethylamino)naphthalin genannt. Bevorzugt werden als Basen Triethylamin und Kaliumcarbonat.

Die Reaktion kann in Ab- und in Anwesenheit von Katalysatoren durchgeführt werden. Insbesondere eignen sich hierfür Alkalimetalliodide wie Natriumiodid oder Kaliumiodid, die dem Reaktionsansatz in Mengen zwischen 0,5 und 150 Molprozent, bevorzugt 5 bis 50 Molprozent, zugesetzt werden.

Die Reaktion wird im allgemeinen in einem Temperaturbereich von -20°C bis +150°C, bevorzugt von +20°C bis +100°C durchgeführt. Die Umsetzung läßt sich bei normalem, erhöhtem und erniedrigtem Druck durchführen. Im allgemeinen arbeitet man bei Normaldruck.

Bei der Durchführung der Reaktion werden im allgemeinen die Ausgangsstoffe in einem molaren Verhältnis von Pyrrolidin (II) zu Alkylverbindung (III) von 0,5 : 1 bis 1,1 : 1 eingesetzt. Die zur Aufnahme des Reaktionsproduktes HR zugesetzte Base wird dabei equimolar oder im bis zu 20-fachen Überschuß eingesetzt. Bevorzugt wird mit einem Verhältnis von Pyrrolidin : Alkylverbindung : Base von 1:1:1 bis 1:1:4 gearbeitet. Wird die Reaktion ohne Base durchgeführt, beträgt das molare Verhältnis von Pyrrolidin (II) zu Alkylverbindung (III) 1:1 bis 5:1. Bevorzugt wird mit einem Molverhältnis von 2:1 gearbeitet.

Das Überführen von funktionellen Gruppen nach Methode M 2 in andere funktionelle Gruppen erfolgt je nach Art der funktionellen Gruppen durch Oxidation, Reduktion, Hydrolyse oder durch Umsetzung mit elektrophilen oder nucleophilen Reagenzien und soll im folgenden erläutert werden.

M 2.1.

Die Reduktion der Nitrilgruppe zur Aminogruppe erfolgt im allgemeinen mit Metallhydriden, bevorzugt mit Lithiumaluminiumhydrid, Aluminiumhydrid (hergestellt z.B. durch Umsetzung von Lithiumaluminiumhydrid mit 100%iger Schwefelsäure oder mit Aluminiumchlorid) oder deren Gemischen in inerten Lösemitteln wie Ethern oder Chlorkohlenwasserstoffen, bevorzugt in Ethern wie beispielsweise Tetrahydrofuran, Diethylether oder Dioxan in einem Temperaturbereich von -20°C bis +100°C, bevorzugt von 0°C bis +50°C bei Normaldruck.

Die Reduktion ist außerdem durch Hydrieren der Nitrile in inerten Lösemitteln wie Alkoholen z.B. Methanol, Ethanol, Propanol oder Isopropanol in Gegenwart eines Edelmetallkatalysators wie Platin, Palladium, Palladium auf Tierkohle oder Raney-Nickel, in einem Temperaturbereich von 0°C bis +150°C, bevorzugt von Raumtemperatur bis +100°C bei Normaldruck oder bei Überdruck möglich.

Die Reaktion kann durch folgendes Schema verdeutlicht werden:

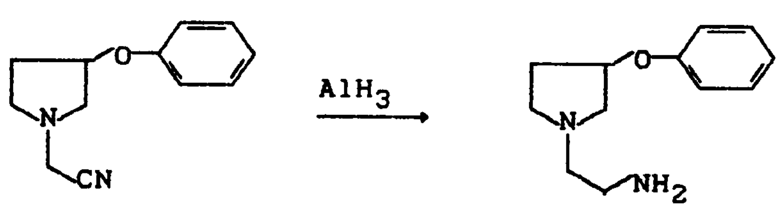


M 2.2.

Die Umsetzung von Verbindungen mit $R^5$ = Bedeutung wie oben, $R^6$ = H, mit Acylierungsmitteln wie Säurechloriden erfolgt im allgemeinen in inerten Lösemitteln wie Ethern, Kohlenwasserstoffen oder Halogenkohlenwasserstoffen oder deren Gemische, bevorzugt in Ethern wie beispielsweise Diethylether, Tetrahydrofuran, oder Halogenkohlenwasserstoffen wie Methylenchlorid oder Chloroform, gegebenenfalls in Anwesenheit von Basen wie Alkalicarbonaten beispielsweise Natriumcarbonat oder Kaliumcarbonat, oder organischen Aminen

wie beispielsweise Triethylamin oder Pyridin, in einem Temperaturbereich von -20°C bis +100°C, bevorzugt von 0°C bis +60°C bei Normaldruck.

Die Reaktion kann durch folgendes Schema verdeutlicht werden:

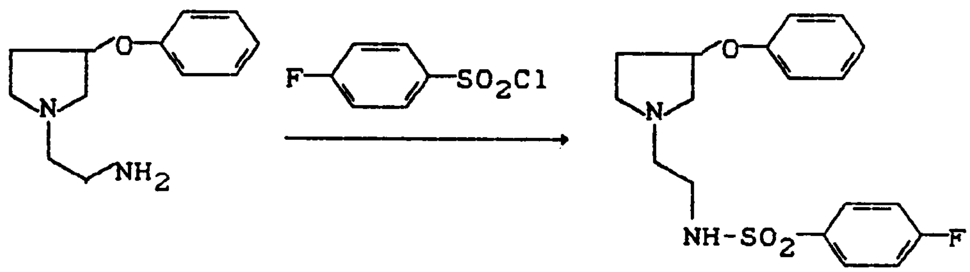

Erfolgt die Umsetzung ohne organische oder anorganische Basen, so erhält man die erfindungsgemäßen Verbindungen in Form ihrer Salze, aus denen z.B. durch Behandeln mit Bicarbonat die freie Base zugänglich ist.

M 2.3.

Cyclische Imide der allgemeinen Formel (I) werden im allgemeinen durch die Umsetzung von Aminoverbindungen des Typs I ($R^5$ = H, $R^6$ = H) mit cyclischen Anhydriden in inerten Lösemitteln wie Ethern, Kohlenwasserstoffen oder Halogenkohlenwasserstoffen oder deren Gemische, bevorzugt in Kohlenwasserstoffen wie Benzol, Toluol, Xylol, gegebenenfalls in Gegenwart organischer Basen, bevorzugt Triethylamin oder Tributylamin, gegebenenfalls unter Entfernung des Reaktionswassers, bevorzugt durch azeotrope Destillation oder den Zusatz aktivierten Molekularsiebs, in einem Temperaturbereich von +20°C bis +150°C, bevorzugt +20°C bis zur Siedetemperatur des Lösemittels hergestellt. Eine Durchführung ohne Lösemittel bei erhöhter Temperatur ist ebenfalls möglich.

Das Verfahren sei durch das folgende Beispiel erläutert:

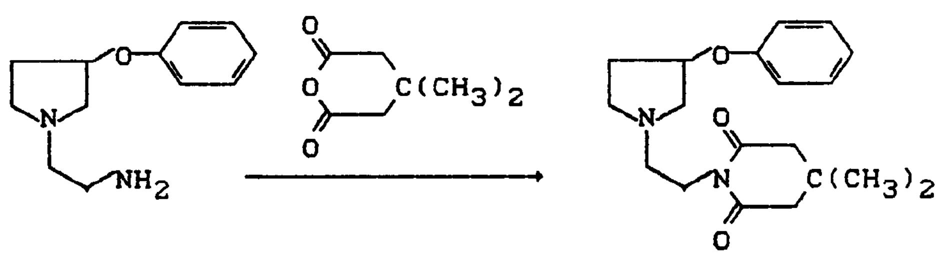

Verbindungen, bei denen n gleich 2 ist und B für Cyano oder eine Gruppe der Formel $-SO_2NR^2R^3$ oder $-SO_mR^4$ wobei $R^2$, $R^3$, $R^4$ und m die oben angegebene Bedeutung haben steht, lassen sich nach einer besonderen Verfahrensvariante (im folgenden Methode M 3 genannt) herstellen.

Es wurde ein Verfahren zur Herstellung von 1,3-disubstituierten Pyrrolidinen der allgemeinen Formel (I)

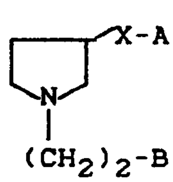

(I),

worin

A und X die oben genannte Bedeutung haben, und B für Cyano oder eine Gruppe der Formel $-SO_2NR^2R^3$ oder $-SO_mR^4$ steht, worin $R^2$, $R^3$, $R^4$ und m die oben angegebene Bedeutung haben gefunden,

dadurch gekennzeichnet, daß man 3-substituierte Pyrrolidine der allgemeinen Formel (II)

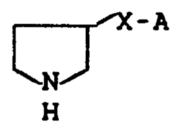

(II),

worin

A und X die oben genannte Bedeutung haben,

oder deren Salze, mit Acrylnitril oder mit einer Gruppe der Formel $CH_2=CH-SO_2NR^2R^3$ oder $CH_2=CH-SO_mR^4$, worin $R^2$, $R^3$, $R^4$ und m die oben angegebene Bedeutung haben, in Gegenwart von Katalysatoren umsetzt (Methode M 3).

Selbstverständlich ist es möglich, aus den Nitrilen gemäß Methode M 2 weitere erfindungsgemäße Pyrrolidine herzustellen.

Das folgende Reaktionsschema soll dieses Verfahren (Methode M 3) verdeutlichen:

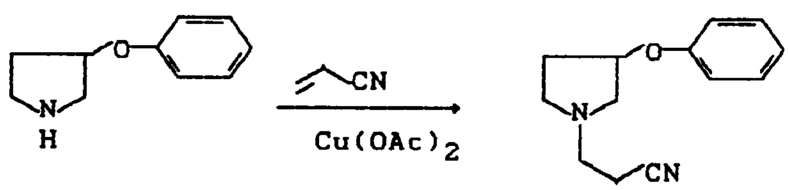


Salze der Pyrrolidine der Formel II für das erfindungsgemäße Verfahren sind beispielsweise Hydrohalogenide (wie Hydrochloride) oder Trifluoracetate.

Katalysatoren für die erfindungsgemäße Verfahrensvariante sind beispielsweise Kupfersalze, bevorzugt Kupfer(II)acetat.

Die Variante wird im allgemeinen im Temperaturbereich von +50°C bis +150°C, bevorzugt von +90°C bis +110°C, bei normalem, erhöhten oder erniedrigten Druck, bevorzugt bei Normaldruck durchgeführt.

Die Pyrrolidine der Formel II und das Olefin werden im allgemeinen im Verhältnis 0,5 zu 20, bevorzugt 1 zu 5, Mol-Äquivalent eingesetzt.

Die eingesetzte Menge an Kupfersalzen beträgt 0,5 bis 10, bevorzugt 1 bis 5, Mol-%, bezogen auf das Pyrrolidin der Formel II.

Gelangen Salze der Pyrrolidine II zum Einsatz, kann man 1 bis 10, bevorzugt 1 bis 3 Molequivalente Base, bezogen auf II, einsetzen. Als Basen dienen anorganische und organische Basen, bevorzugt Triethylamin.

Die Verfahrensvariante kann selbstverständlich in inerten Lösungsmitteln durchgeführt werden.

Die zur Darstellung der erfindungsgemäßen Verbindungen (I) gemäß den Methoden M 1 bis M 3 verwendeten 3-substituierten Pyrrolidine vom Typ II sind entweder bekannt [J. med. Chem. 12, 435 bis 441 (1969)] oder können gemäß Methode P aus Pyrrolidinen der Formel IV hergestellt werden.

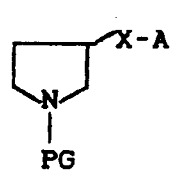

(IV)

worin

A und X die oben genannte Bedeutung haben,

und PG für eine nach üblichen Methoden der organischen Chemie abspaltbare Stickstoff-Schutzgruppe (Th. Greene, "Protective Groups in Organic Synthesis", 1. Aufl., J. Wiley and Sons, New York, 1981) steht. Die Schutzgruppen können z.B. durch Hydrolyse oder Hydrogenolyse abgespalten werden.

Für den Fall, daß der Rest X-A für O-Aryl steht, sind die Pyrrolidine (IV) aus den entsprechenden Hydroxypyrrolidinen und den entsprechenden Hydroxyaromaten in an sich bekannter Weise zugänglich. Steht X-A für $-O-CH_2$-Aryl, so erhält man diese Verbindungen aus den entsprechenden Aryl-methylhalogeniden und den entsprechenden Hydroxypyrrolidinen in an sich bekannter Weise.

3-Hydroxymethyl-pyrrolidine ergeben durch Umsetzung mit entsprechenden Hydroxyaromaten in an sich bekannter Weise Pyrrolidine der Formel (IV), bei denen X-A für $-CH_2$-O-Aryl steht.

Die Erfindung umfaßt beide Enantiomeren der Pyrrolidine des Typs I (= Ia, Ib)

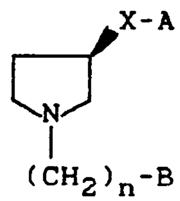

Ia

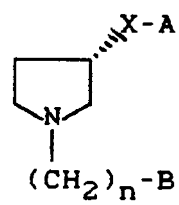

Ib

worin
A, B, X und n die oben genannte Bedeutung haben,
sowie deren Gemische.

Die vom Verhältnis Ia/Ib = 1:1 abweichenden Gemische sowie die reinen Isomere erhält man zweckmäßigerweise durch die Anwendung üblicher Methoden der Synthese optisch aktiver Verbindungen.

Als Beispiele seien die Bildung und Trennung diastereomerer Salze (mit optisch aktiven Säuren) der Verbindungen I, II, IV, (sofern PG nicht Acyl oder Alkoxycarbonyl ist), mit anschließender Freisetzung der Base und die Verwendung optisch aktiver Startmaterialien [D. Flanagan + M Joullie, Heterocycles 26, 2247 (1987)] genannt.

Die erfindungsgemäßen Pyrrolidine haben eine überlegene pharmakologische Wirkung, insbesondere auf das zentrale Nervensystem, und können als Wirkstoffe in Arzneimitteln eingesetzt werden.

Insbesondere haben die erfindungsgemäßen Pyrrolidine eine hohe Affinität zu cerebralen 5-Hydroxytryptamin-Rezeptoren vom 5-$HT_1$-Typ. Hiermit verbunden sind agonistisch, partiell agonistisch oder antagonistische Wirkungen am Serotonin-Rezeptor.

Die in der vorliegenden Erfindung beschriebenen hochaffinen Liganden für den Serotonin-1-Rezeptor stellen somit Wirkstoffe zur Bekämpfung von Krankheiten dar, die durch Störungen des serotoninergen Systems, insbesondere bei Involvierung von Rezeptoren, die hohe Affinität zu 5-Hydroxytryptamin besitzen (5-$HT_1$-Typ), gekennzeichnet sind. Sie eignen sich daher zur Behandlung von Erkrankungen des Zentralnervensystems wie Angst-, Spannungs- und Depressionszuständen, zentralnervös bedingten Sexualdysfunktionen und Schlaf- und Nahrungsaufnahmestörungen. Weiterhin sind sie geeignet zur Beseitigung kognitiver Defizite, zur Verbesserung von Lern- und Gedächtnisleistungen und zur Behandlung der Alzheimer'schen Krankheit. Weiterhin eignen sich diese Wirkstoffe auch zur Modulierung des cardiovaskulären Systems. Sie greifen auch in die Regulation der cerebralen Durchblutung ein und stellen somit wirkungsvolle Mittel zur Bekämpfung von Migräne dar. Auch eignen sie sich als Prophylaxe und zur Bekämpfung der Folgen cerebraler Infarktgeschehen (Apoplexia cerebri) wie Schlaganfall und cerebraler Ischämien. Ebenso können die erfindungsgemäßen Verbindungen zur Bekämpfung von Schmerzzuständen eingesetzt werden. Auch eignen sie sich zur Bekämpfung von Erkrankungen der Intestinaltraktes, die durch Störungen des serotoninergen Systems gekennzeichnet sind wie auch Störungen des Kohlehydrathaushaltes.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenen falls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B.: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether (z.B. Lignin-, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellungsbeispiele

Die jeweils aufgeführten $R_f$-Werte wurden - sofern nicht anders vermerkt - durch Dünnschichtchromatographie auf Kieselgel (Alufolie, Kieselgel 60 F 254, Fa. E. Merck) ermittelt. Die Visualisierung der Substanzflecke geschah durch Betrachten unter UV-Licht und/oder durch Besprühen mit 1%iger Kaliumpermanganat-Lösung.

Die Flash-Chromatographie wurde auf Kieselgel 60, 0,040 - 0,064 mm, Fa. E. Merck, durchgeführt (siehe Still et al., J. Org. Chem. 43, 2923, 1978; für einfachere Trennprobleme siehe Aldrichimica Acta 18, 25, 1985). Elution mit Lösemittelgradienten bedeutet: Beginnend mit der reinen, unpolaren Lösemittelgemischkomponente wird in steigendem Ausmaß die polare Laufmittelkomponente zugemischt, bis das gewünschte Produkt eluiert wird (DC-Kontrolle).

Bei allen Produkten wurde bei zuletzt ca. 13 Pa (0,1 Torr) das Lösemittel abdestilliert. Salze wurden bei diesem Druck über Nacht über Kaliumhydroxid und/oder Phosphorpentoxid aufbewahrt.

Beispiel 1 (Methode M 1)

3-(2-Cyanophenoxy)-1-[4-(1,1-dioxido-3-oxo-2,3-dihydrobenzisothiazol-2-yl)butyl]-pyrrolidin-oxalat

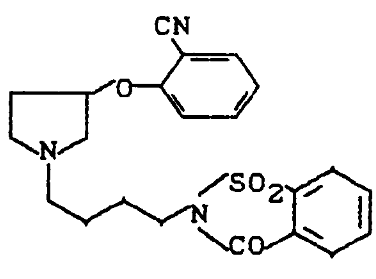

1,88 g (10 mmol) 3-(2-Cyanophenoxy)-pyrrolidin, 3,18 g (10 mmol) 2-(4-Brombutyl)-benzisothiazol-3(2H)-on-1,1-dioxid und 1,01 g (10 mmol) Triethylamin wurden in 40 ml trockenem Dimethylformamid gelöst und 20 Stunden bei 45°C unter Feuchtigkeitsausschluß gerührt. Das Lösemittel wurde im Hochvakuum entfernt, der verbleibende Rückstand durch Flashchromatographie (Toluol-Essigester und Toluol-Ethanol-Gradienten) gereinigt. Das Produkt (2,90 g) wurde als gelbes Öl erhalten.
Das $^1$H-NMR-Spektrum wies auf das Vorliegen von ca. 10% Dimethylformamid in diesem Produkt hin.
$^1$H-NMR ($CDCl_3$):

δ = 1,65 (quin, 2H); 1,95 (quin, 2H); 2,1 (m, 1H); 2,35 (m, 1H); 2,6 - 3,0 (m, enthält auch Signale von

Dimethylformamid); 3,3 (dd, 1H); 3,8 (t, 2H); 4,95 (m, 1H); 6,9 (d, 1H); 7,0 (dd, 1H); 7,5 (m, 2H); 7,8 - 8,1 (m, enthält Signal von Dimethylformamid).
Ausbeute: 61%.
$R_f$ = 0,48 (Toluol/Methanol 4:1; Kieselgelplatten)

Hieraus wurde durch Behandeln mit wasserfreier Oxalsäure in warmen, wasserfreiem Ethanol das Oxalat gewonnen. Umkristallisation aus Aceton/Essigester/tert.Butylmethylether ergab farblose Kristalle (1,68 g) vom Schmelzpunkt 135 - 139°C.

In ähnlicher Weise wurde diese Methode zur Gewinnung der Verbindungen I der Tabelle 1 (Methode M 1) eingesetzt. Gelangten Pyrrolidine vom Typ II in der Form ihrer Hydrochloride zum Einsatz, so wurde die 2 bis 3-fache Menge Triethylamin verwendet.

Beispiel 2 (Methode M 2.1.)

1-(3-Aminopropyl)-3-(2-methoxyphenoxy)-pyrrolidin 1,5-naphthalindisulfonat

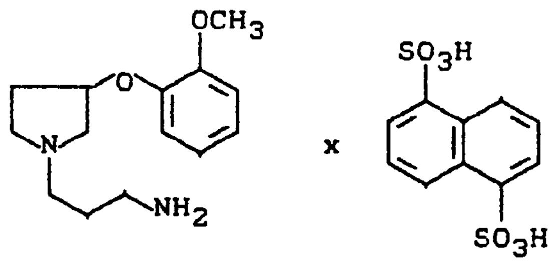

Bei 0°C wurde die Suspension von 1,6 g (42 mmol) Lithiumaluminiumhydrid in 50 ml Ether mit 2,06 g einer Mischung aus 95% Schwefelsäure und 20% Oleum im Verhältnis 1:1 vorsichtig versetzt. nach 1 h bei Raumtemperatur wurden 3,5 g (14 mmol) 1-(2-Cyanoethyl)-3-(2-methoxyphenoxy)-pyrrolidin in 70 ml Ether zugetropft. Es wurde 2 h am Rückfluß erhitzt. Unter Eiskühlung wurde mit 9,3 ml Wasser und 18,6 ml 10%iger Natronlauge versetzt. Verrühren mit Kieselgur, Absaugen über Kieselgur, Nachwaschen des Filterkuchens mit Essigester, Trocknen und Einengen ergaben 3,4 g der freien Base als gelbes Öl (96%).
MS (FAB): 251 (M + 1).
$^1$H-NMR ($CDCl_3$):
$\delta$ = 1,2 - 1,5 (breites Signal, ca. 2H; -$NH_2$); 1,65 (quin, 2H); 2,05 (m, 1H); 2,25 (m, 1H); 2,5 - 2,8 (m, 7H); 3,0 (dd, 1H); 3,75 (s, 3H); 4,85 (m, 1H); 6,75 - 6,95 (m, 4H).
$R_f$ = 0,1 (Toluol/Methanol 4:1; Kieselgelplatten)

Aus 1,2 g dieses Produktes wurde mit 1,5-Naphthalindisulfonsäure in heißem Ethanol das Salz (1:1-Addukt) in Form beiger Kristalle gewonnen (1,7 g).
Schmp.: ab 187°C unter Aufschäumen.

Beispiel 3 (Methode M 2.2.)

1-[3-(4-Fluorphenyl)sulfonamido-propyl]-3-(2-methoxyphenoxy)-pyrrolidin Hydrochlorid

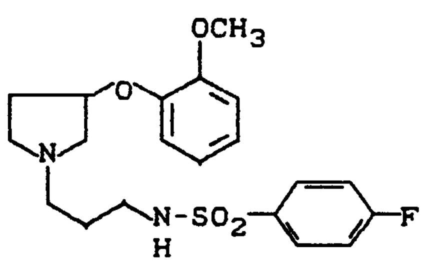

Bei 0°C wurden zur Lösung von 1,0 g (4,0 mmol) 1-(3-Aminopropyl)-3-(2-methoxyphenoxy)-pyrrolidin in 20 ml wasserfreiem Ether 0,78 g (4,0 mmol) 4-Fluorphenylsulfonsäurechlorid in 15 ml Ether getropft. Nach 2 h bei Raumtemperatur wurde vom Solvens im Vakuum befreit und der verbleibende ölige Rückstand mit Diethylether/Hexan durch Anreiben zur Kristallisation gebracht. Auf diese weise erhielt man 1,1 g (62%) Titelverbindung als leicht rötlichen, hygroskopischen Feststoff. Es schmolz ab 50°C.
MS (FAB): 409 (M + 1)
$R_f$ = 0,2 (Toluol/Methanol 4:1, Kieselgelplatten) [freie Base]

Beispiel 4 (Methode M 2.3.)

1-[3-(4,4-Dimethyl-2,6-dioxo-piperidin-1-yl)-propyl]-3-(2-methoxyphenoxy)-pyrrolidin (Salz mit 1,5-Naphthalindisulfonsäure)

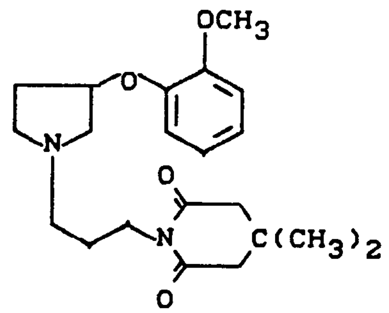

x 1/2

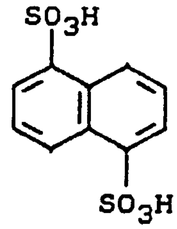

Die Lösung von 1,0 g (4,0 mmol) 1-(3-Aminopropyl)-3-(2-methoxyphenoxy)-pyrrolidin, 0,57 g (4,0 mmol) 3,3-Dimethylglutarsäureanhydrid und 4 Tropfen Tributylamin wurde in Gegenwart von 2 g Molsieb (3Å) zum Rückfluß erhitzt. Nach 2 h wurde filtriert und vom Lösemittel im Vakuum befreit. Der derart erhaltene Rückstand wurde durch Flashchromatographie (Toluol-Essigester und Essigester-Ethanol-Gradient) gereinigt.
Man erhielt 1,0 g (67%) freie Base als gelbes Öl.
$R_f$ = 0,6 (Toluol/Methanol 4:1, Kieselgelplatten)
IR ($CHCl_3$): 3008, 2965, 2814, 1727, 1672, 1593, 1503.
Hieraus wurde mit 1,5-Naphthalindisulfonsäure in heißem Ethanol das Salz (als 2:1 Verbindung) gefällt.
Schmp.: 195 - 200°C (farblose Kristalle)

Beispiel 5 (Methode M 3)

1-(2-Cyanoethyl)-3-(2-methoxyphenoxy)pyrrolidin (als Salz der 1,5-Naphthalindisulfonsäure).

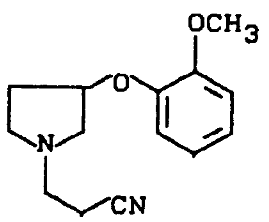

x 1/2

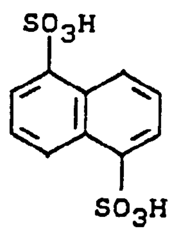

5,0 g (22 mmol) 3-(2-Methoxyphenoxy)-pyrrolidin Hydrochlorid, 2,43 g (24 mmol) Triethylamin und 5,8 g (109 mmol) Acrylnitril wurden mit 0,1 g Kupfer(II)acetat versetzt und 4 Stunden bei 110°C gerührt. Der Reaktionsansatz wurde durch Flashchromatographie (Kieselgel, Toluol/Essigester-Gradient) gereinigt. Es wurden 5,0 g (93%) der freien Base als gelbliches Öl erhalten.
MS (m/e): 246, 206, 123, 83, 82
$R_f$ = 0,3 (Toluol/Methanol 4:1, Kieselgelplatten)
IR ($CHCl_3$): 3030, 3003, 2951, 2822, 2254, 1593, 1503.
Behandeln der freien Base mit 1,5-Naphthalindisulfonsäure in heißem Ethanol ergab das 2:1 Salz der Titelverbindung als farblosen Feststoff.
Schmelzbereich: 85 - 95°C

Beispiele 6 bis 45

Die Beispiele sind in der Tabelle 1 zusammengefaßt:

Legende zu Tabelle 1:

a) Verwendete Abkürzungen in Spalte B:

SACCH = 1,1-Dioxido-2,3-dihydro-3-oxo-benzisothiazol-2-yl
FSULF = [(4-Fluorphenyl)sulfonyl]amino
NPSULF = [N-Phenyl-N-methylsulfonyl]amino
TDIAZ = 1,1-Dioxido-3-oxo-4-phenyl-3,4-dihydro-2H-1,2,4-benzothiadiazin-2-yl
NAPHT = 1,1-Dioxido-2H-naphth[1,8-cd]isothiazol-2-yl
DMP = 4,4-Dimethyl-2,6-dioxo-piperidin-2-yl
$NH_2$ = Amino
CN = Cyano
PYRR = 3-(2-Oxopyrrolidin-1-y)prop-1-inyl
DES = N,N-Diethylsulfamoyl
DMS = N,N-Dimethylsulfamoyl
BITZ = 1,1-Dioxido 2,3-Dihydro-benzisothiazol-2-yl
SANTH = N-(2-Methoxycarbonylphenyl)sulfamoyl
SIND = 3-Methyl-indol-1-yl-sulfonyl
PHEN = 6(5H)-Phenanthridinon-5-yl
DBTH = 5,5-Dioxido-6H-dibenzo[c,e][1,2]thiazin-6-yl
DMUR = (1,3-Dimethyl-uracil-6-yl)amino

b) Verwendete Abkürzungen der Laufmittel

KTM = Toluol/Methanol 4:1; Kieselgelplatten
KDM = Dichlormethan/Methanol 10:1; Kieselgelplatten
ADM = Dichlormethan/Methanol 100:1; Aluminiumoxidplatten
AE = Ethanol; Aluminiumoxidplatten
KTE = Toluol/Essigester 1:1, Kieselgelplatten

c) Verwendete Abkürzungen der Salze

OX = Salz der Oxalsäure
NS = Salz der 1,5-Naphthalindisulfonsäure (Stöchiometrie: 2 Mol Base/ 1 Mol Säure)
NDS = Salz der 1,5-Naphthalindisulfonsäure (1 Mol Base / 1 Mol Säure)
Cl = Salz der Salzsäure
W = Salz der L-Weinsäure
F = Freie Base

d) Verwendete Abkürzungen der Edukte

A = 2-(4-Brombutyl)-2H-benzisothiazol-3-on-1,1-dioxid
B = Chloracetonitril
C = Acrylnitril
D = 2-(4-Brombutyl)-2H-naphth[1,8-cd]isothiazol-1,1-dioxid
E = 2-(4-Brombutyl)-3,4-dihydro-4-phenyl-2H-1,2,4-benzothiadiazin-3-on-1,1-dioxid
F = (4-Fluorphenyl)sulfonsäurechlorid
G = 3,3-Dimethylglutarsäureanhydrid
H = Aluminiumhydrid
I = N-(4-Brombutyl)-N-phenyl-methansulfonamid
K = 2-(3-Brompropyl)-2H-benzisothiazol-3-on-1,1-dioxid
L = N,N-Dimethyl-vinylsulfonamid
M = N,N-Diethyl-vinylsulfonamid
N = 2-(4-Brombutyl)-2,3-dihydrobenzisothiazol-1,1-dioxide
O = 2-(Ethensulfonylamino)benzoesäuremethylester [aus Anthranilsäure-methylester und 2-Chlorethansulfonsäurechlorid in Gegenwart überschüssigen Triethylamins]
P = 3-Methyl-1-vinylsulfonyl-indol [aus 3-Methylindol und 2-Chlorethansulfonsäurechlorid in Gegenwart überschüssiger Triethylamine]
R = 5-(4-Brombutyl)-6(5H)-phenanthridinon
S = 6-(4-Brombutyl)-6H-dibenzo[c,e][1,2]-thiazin-5,5-dioxid
T = 6-(3-Chlorpropyl)amino-1,3-dimethyluracil

e) $^1$H-NMR ($CD_3OD$):

$\delta$ = 1,8 - 2,0 (m, 4H); 2,1 - 2,4 (m, 1H); 2,5 - 2,8 (m, 1H); 3,2 - 3,5 (m, enthält Signale des Lösemittels); 3,7 - 4,2 (m, 4H); 5,2 (m, 1H); 6,85 - 7,0 (m, 3H); 7,2 - 7,4 (m, 2H); 7,75 - 8,15 (m, 4H).

f) R(-)-Enantiomeres von Bsp. 8; $\alpha_D^{20}$ = -7,0 (c=1,$CH_3OH$)
g) sirupös, wird nach Degeration mit Diethylether fest.
h) $^1H$-NMR ($CD_3OD$):
δ = 1,05 (s, 6H); 2,1 - 2,7 (m, 6H); 3,3 - 3,6 (m, enthält Signale des Lösemittels); 3,7 - 4,2 (m, darunter: 3,75, s, $OCH_3$; 7H); 5,1 (m, 1H); 6,85 - 7,1 (m, 4H).

i) Elementaranalyse $C_{23}H_{28}N_2O_5S$ x $C_4H_6O_6$
k) $^{13}C$-NMR ($CDCl_3$):
25,4 (t), 26,5 (t), 32,1(t), 36,9 (q), 50,4 (t), 52,8 (t), 55,6 (t), 55,9 (q), 60,3 (t), 77,6 (d), 112,1 (d), 114,8 (d), 120,8 (d), 121,3 (d), 128,0 (d), 128,5 (d), 129,5 (d), 139,1 (s), 147,4 (s), 150,0 (s).
l) aus Beispiel 57 durch Umsetzung mit 1-(Prop-2-inyl)-2-pyrrolidon und Paraformaldehyd in Gegenwart von Kupfer(II)acetat
m) S(+)-Enantiomeres von Beispiel 8 $\alpha_D^{20}$ = +7,1 (c = 1, $CH_3OH$)

Tabelle 1: Verbindungen der allgemeinen Formel (I)

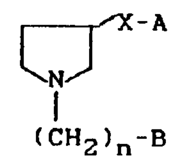

| Bsp. | -X-(A) | -A | -B[a)] | n | $R_f$/Solvens [b)] | Salz[c)]/Schmp [°C] | hergestellt aus[d)] | Methode |
|---|---|---|---|---|---|---|---|---|
| 6 | -O- | Phenyl | SACCH | 4 | 0,35 (KTM) | Cl; amorph[e)] | Bsp.48 + A | M 1 |
| 7 | $-OCH_2-$ | Phenyl | SACCH | 4 | 0,4 (KDM) | NS; 155-158 | Bsp.49 + A | M 1 |
| 8 | -O- | 2-Methoxyphenyl | SACCH | 4 | 0,35 (KTM) | OX; 134 | Bsp.50 + A | M 1 |
| 9[f)] | -O- | 2-Methoxyphenyl | SACCH | 4 | 0,52 (KDM) | F; g) | Bsp.55 + A | M 1 |
| 10 | -O- | 3-Methoxyphenyl | SACCH | 4 | 0,52 (KDM) | NS; 173-178 | Bsp.51 + A | M 1 |
| 11 | -O- | 2-Methoxyphenyl | $NH_2$ | 2 | 0,1 (KTM) | NDS; >220 | Bsp.14 + H | M 2.1. |
| 12 | -O- | 2-Methoxyphenyl | FSULF | 2 | 0,4 (KTM) | Cl; 98-110 | Bsp.11 + F | M 2.2. |
| 13 | -O- | 2-Methoxyphenyl | DMP | 2 | 0,3 (KTM) | Cl; hygr.[h)] | Bsp.11 + G | M 2.3. |
| 14 | -O- | 2-Methoxyphenyl | CN | 1 | 0,57 (KTM) | F; 73-76 | Bsp.50 + B | M 1 |
| 15 | $-CH_2O-$ | 2-Methoxyphenyl | SACCH | 4 | 0,34 (ADM) | W; amorph[i)] | Bsp.46 + A | M 1 |
| 16 | -O- | 2-Methoxyphenyl | NPSULF | 4 | 0,24 (KTM) | F; k) | Bsp.50 + I | M 1 |
| 17 | -O- | 2-Methoxyphenyl | NAPHT | 4 | 0,35 (KTM) | NS; 115-117 | Bsp.50 + D | M 1 |

Fortsetzung Tabelle 1

| Bsp. | -X-(A) | -A | $B^{a)}$ | n | $R_f$/Solvens $^{b)}$ | Salz$^{c)}$/Schmp [°C] | hergestellt aus$^{d)}$ | Methode |
|---|---|---|---|---|---|---|---|---|
| 18 | -O- | 2-Methoxyphenyl | TDIAZ | 4 | 0,38 (KDM) | Cl; amorph | Bsp.50 + E | M 1 |
| 19 | -O- | (2-Carbamoyl)-phenyl | SACCH | 4 | 0,75 (AE) | NDS; >220 | Bsp.53 + A | M 1 |
| 20 | -O- | (2-Methoxy-6-cyano)phenyl | SACCH | 4 | 0,38 (KTM) | NS; 165(Zers) | Bsp.47 + A | M 1 |
| 21 | -O- | (1-Cyano)-naphth-2-yl | SACCH | 4 | 0,59 (KDM) | F; 132-134 | Bsp.54 + A | M 1 |
| 22 | -O- | (2-Carbamoyl)-naphth-1-yl | SACCH | 4 | 0,44 (KDM) | NS; >167 (Sintern) | Bsp.56 + A | M 1 |
| 23 | -O- | Naphth-1-yl | SACCH | 4 | 0,50 (KDM) | NS; 209-215 | Bsp.57 + A | M 1 |
| 24 | -O- | Naphth-2-yl | SACCH | 4 | 0,53 (KDM) | OX; 142-149 | Bsp.58 + A | M 1 |
| 25 | $-OCH_2-$ | Naphth-1-yl | SACCH | 4 | 0,35 (KDM) | OX; amorph | Bsp.59 + A | M 1 |
| 26 | $-OCH_2-$ | Naphth-2-yl | SACCH | 4 | 0,4 (KDM) | NS; 138 | Bsp.60 + A | M 1 |
| 27 | $-OCH_2-$ | 2-Fluorphenyl | SACCH | 4 | 0,23 (KTM) | NS; 143-148 | Bsp.61 + A | M 1 |
| 28 | -O- | (1-Methyl)indol-4-yl | SACCH | 4 | 0,44 (KDM) | NS;>162(Zers) | Bsp.62 + A | M 1 |

Fortsetzung Tabelle 1

| Bsp. | -X-(A) | -A | B[a] | n | $R_f$/Solvens [b] | Salz[c]/Schmp [°C] | hergestellt aus[d] | Methode |
|---|---|---|---|---|---|---|---|---|
| 29 | -O- | Naphth-1-yl | NAPHT | 4 | 0,64 (KDM) | Cl; 192-193 | Bsp.57 + D | M 1 |
| 30 | -O- | 4-Methoxyphenyl | SACCH | 4 | 0,6 (KDM) | NS; 192-196 | Bsp.63 + A | M 1 |
| 31 | -O- | 5,6,7,8-Tetra-hydro-naphth-1-yl | SACCH | 4 | 0,38 (KDM) | NS; 190-192 | Bsp.64 + A | M 1 |
| 32 | -O- | Chinolin-2-yl | SACCH | 4 | 0,28 (KTM) | NDS;200(Zers.) | Bsp.65 + A | M 1 |
| 33 | -O- | Isochinolin-1-yl | SACCH | 4 | 0,63 (KDM) | NDS;180(Zers.) | Bsp.66 + A | M 1 |
| 34 | -O- | Naphth-1-yl | PYRR | 1 | 0,75 (KTM) | Cl; 138-140 | | 1) |
| 35 | -O- | 2-Methoxyphenyl | SACCH | 3 | 0,44 (KTM) | NS; ab 225 (Zers.) | Bsp. 50 + K | M 1 |
| 36 | -O- | 2-Methoxyphenyl | DMS | 2 | 0,24 (KTM) | NS; 162-164 | Bsp. 50 + L | M 3 |
| 37 | -O- | 2-Methoxyphenyl | DES | 2 | 0,30 (KTM) | NS; 145-150 | Bsp. 50 + M | M 3 |
| 38[m] | -O- | 2-Methoxyphenyl | SACCH | 4 | 0,50 (KDM) | F; 70-73 | Bsp. 67 + A | M 1 |
| 39 | -O- | 2-Methoxyphenyl | BITZ | 4 | 0,44 (KDM) | NS; 150-156 | Bsp. 50 + N | M 1 |
| 40 | -O- | 2-Methoxyphenyl | SANTH | 2 | 0,36 (KTE) | NS; 168-170 | Bsp. 50 + O | M 3 |

Fortsetzung Tabelle 1

| Bsp. | -X-(A) | -A | B[a] | n | $R_f$/Solvens [b] | Salz[c]/Schmp [°C] | hergestellt aus[d] | Methode |
|---|---|---|---|---|---|---|---|---|
| 41 | -O- | 2-Methoxyphenyl | SIND | 2 | 0,46 (KTE) | F; 156-158 | Bsp. 50 + P | M 3 |
| 42 | -O- | 2-Methoxyphenyl | PHEN | 4 | 0,31 (KDM) | Cl; 169-170 | Bsp. 50 + R | M 1 |
| 43 | -O- | 2-Methoxyphenyl | DBTH | 4 | 0,48 (KDM) | NS; 182-186 | Bsp. 50 + S | M 1 |
| 44 | -O- | 2-(Prop-2-enyl-oxy)phenyl | SACCH | 4 | 0,46 (KDM) | NS; 138-142 | Bsp. 68 + A | M 1 |
| 45 | -O- | 2-Methoxyphenyl | DMUR | 3 | 0,35 (KDM) | NDS;199(Zers.) | Bsp. 50 + T | M 1 |

Ausgangsverbindungen

Beispiel 46

3-(2-Methoxyphenoxy)methyl-pyrrolidin

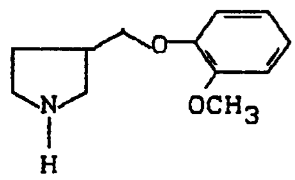

Die Lösung von 5,0 g (17 mmol) 1-Benzyl-3-(2-methoxyphenoxy)methyl-pyrrolidin und 10,6 g (0,17 mol) Ammoniumformiat in 300 ml Methanol wurde mit 5,0 g 10% Palladium auf Aktivkohle versetzt und 1 Stunde zum Rückfluß erhitzt. Nach Abkühlen wurde vom Katalysator durch Filtration abgetrennt und das Filtrat vom Lösemittel im Vakuum befreit. Der Rückstand wurde mit 1 N NaOH basisch gestellt und die Mischung mit Ether extrahiert. Nach Trocknen und Eindampfen wurden 1,5 g (43%) der Titelverbindung als Öl erhalten.
$R_f$ = 0,35 (Dichlormethan/Methanol 20:1; Aluminiumoxidplatten)
(vgl. DE-A 23 150 92)

Beispiel 47

3-(2-Cyano-6-methoxy)phenoxy-pyrrolidin-Hydrochlorid

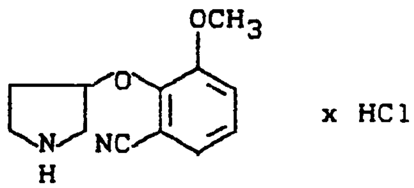

6,3 g (19,8 mmol) 1-tert.Butoxycarbonyl-3-(2-cyano-6-methoxy-phenoxy)pyrrolidin wurden bei 0°C mit 14,8 ml (59,4 mmol) 4 M Chlorwasserstoff in wasserfreiem Dioxan versetzt. Nach 2 Stunden bei Raumtemperatur wurden nochmals 7,4 ml 4 M Chlorwasserstoff in Dioxan zugegeben. Nach weiteren 2 Stunden wurde eingeengt. Der Rückstand wurde mit Toluol angerieben und über Nacht unter Toluol kristallisieren lassen. Auf diese Weise wurden 4,2 g (83%) leicht rötliche Kristalle erhalten.
Schmp.: 132°C (ab 98°C: sintern)
$R_f$ = 0,14 (Dichlormethan/Methanol 10:1, Kieselgelplatten)
[freie Base]

Beispiele 48 bis 68

Die Beispiele 48 bis 68 sind in Tabelle 2 zusammengefaßt; sie wurden analog zu Beispiel 46 und 47 erhalten.

Legende zu Tabelle 2

a) DC-System: Kieselgelplatten
Laufmittel:
TET = Toluol/Ethanol/Triethylamin 5:3:1
DM = Dichlormethan/Methanol 10:1
b) Salze:
Cl = Hydrochlorid ; F = freie Base
c) Darstellung aus Bsp.72 mit Trifluoressigsäure bei 0°C/45 min und anschließender Freisetzung der Base mit Alkali.

$^1H$-NMR ($CDCl_3$):

$\delta$ = 2,0 - 2,3 (m, ca. 4H, enthält auch Signal von -NH und Wasserspuren); 2,9 - 3,3 (m, 4H); 4,9 (m, 1H); 6,85 - 7,1 (m, 2H); 7,45 - 7,6 (m, 2H).

d) Sintern ab 98°C

e) aus Bsp. 52

f) Als Rohprodukt weiter umgesetzt zu Beispiel 9

g) amorph, hygroskopischer Feststoff

$^1H$-NMR ($CD_3OD$):

$\delta$ = 2,0 - 2,5 (m, 2H); 3,4 - 3,8 (m, 4H); 5,15 (m, 1H); 7,5 - 8,3 (m, 6H).

h) Dihydrochlorid; direkt weiter umgesetzt zu Beispiel 28.

i) 3 Äquivalente HCl in Dioxan eingesetzt;

Dihydrochlorid. amorph, hygroskopisch

$^1H$-NMR ($CD_3OD$):

$\delta$ = 2,5 (mc, 2H); 3,55 (mc, 2H); 3,75 (mc, 2H); 5,9 (m,1H); 7,4 (d, 1H); 7,6 (m, 1H); 7,8 - 8,1 (m, 3H); 8,6 (d, 1H).

k) S(+)-Enantiomeres; $\alpha_D^{20}$ (Hydrochlorid) + 22,6 (c=1; $CH_3OH$)

Tabelle 2: Verbindungen der allgemeinen Formel (II)

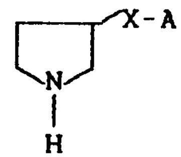

| Bsp. | -X-(A) | -A | $R_f$/Solvens a) | Salz b)/Schmp [°C] | hergestellt aus |
|---|---|---|---|---|---|
| 48 | -O- | Phenyl | [vgl. DE 19 64 511] | | |
| 49 | $-OCH_2-$ | Phenyl | 0,41 (TET) | Cl; Sirup | Bsp.71 |
| 50 | -O- | 2-Methoxyphenyl | [vgl. US 430 60 65] | | |
| 51 | -O- | 3-Methoxyphenyl | [vgl. DE 19 64 511] | | |
| 52 | -O- | 2-Cyanophenyl | 0,35 (TET) | F; Sirup c) | Bsp.72 |
| 53 | -O- e) | 2-Carbamoylphenyl | [vgl. DE 19 64 510] | | |
| 54 | -O- | (1-Cyano)naphth-2-yl | 0,11 (DM) | Cl; 204-208 | Bsp.70 |
| 55 | -O- | 2-Methoxyphenyl | f) | - | Bsp.74 |

Fortsetzung Tabelle 2

| Bsp. | -X-(A) | -A | $R_f$/Solvens a) | Salz b)/Schmp [°C] | hergestellt aus |
|---|---|---|---|---|---|
| 56 | -O- | (2-Carbamoyl-naphth-1-yl | <0,1 (TET) | Cl; g) | Bsp. 75 |
| 57 | -O- | Naphth-1-yl | 0,42 (TET) | Cl; 223-226 | Bsp. 77 |
| 58 | -O- | Naphth-2-yl | 0,5 (TET) | Cl; 168-170 | Bsp. 78 |
| 59 | $-OCH_2-$ | Naphth-1-yl | 0,25 (TET) | Cl; 117-124 | Bsp. 69 |
| 60 | $-OCH_2-$ | Naphth-2-yl | 0,28 (TET) | Cl; 128-130 | Bsp. 79 |
| 61 | $-OCH_2-$ | 2-Fluorphenyl | 0,5 (TET) | Cl; Sirup | Bsp. 80 |
| 62 | -O- | (1-Methyl)-indol-4-yl | <0,1 (TET) | Cl; Sirup h) | Bsp. 81 |
| 63 | -O- | 4-Methoxyphenyl | [vergl. J. Med. Chem. 12, 435 (1969)] | | |
| 64 | -O- | 5,6,7,8-Tetra-hydro-naphth-1-yl | 0,52 (TET) | Cl; 196-200 | Bsp. 83 |
| 65 | -O- | Chinolin-2-yl | 0,42 (TET) | i) | Bsp. 84 |
| 66 | -O- | Isochinolin-1-yl | 0,38 (TET) | Cl; Schaum | Bsp. 85 |
| 67 k) | -O- | 2-Methoxyphenyl | 0,22 (TET) | Cl; 145-147 | Bsp. 86 |
| 68 | -O- | 2-(Prop-2-en-1-yloxy)phenyl | 0,2 (TET) | Cl; Sirup | Bsp. 87 |

## Beispiel 69

1-tert.Butoxycarbonyl-3-(1-naphthyl)methoxy-pyrrolidin

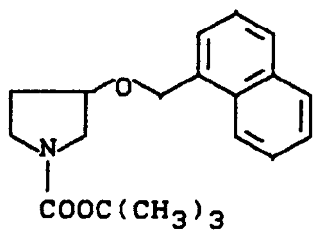

Zur Lösung von 7,49 g (40 mmol) 1-tert.Butoxycarbonyl-3-hydroxy-pyrrolidin in 40 ml trockenem Dimethylsulfoxid wurden bei 0°C 1,27 g einer 80%igen Suspension von Natriumhydrid in Paraffin (44 mmol) in kleinen Portionen gegeben. Anschließend wurden 7,77 g (44 mmol) 1-Chlormethyl-naphthalin langsam zugetropft. Nach 3 Stunden bei Raumtemperatur wurde auf 200 ml gesättigte Ammoniumchlorid-Lösung gegossen, mit Ether mehrmals extrahiert, getrocknet ($MgSO_4$) und eingeengt. Flashchromatographie (Petrolether-Toluol, dann Toluol-Essigester-Gradienten) ergab 10,5 g der Titelverbindung (76%) als gelbliches Öl.
$R_f$ = 0,46 (Toluol-Essigester 3:1)

Beispiel 70

1-tert.Butoxycarbonyl-3-[(1-cyano)-2-naphthyloxy]pyrrolidin

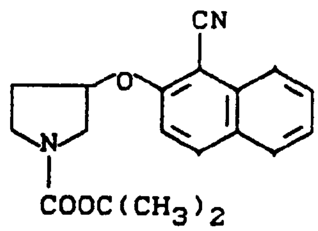

Zur Lösung von 6,2 g (37 mmol) 1-Cyano-2-hydroxy-naphthalin, 8,2 g (44 mmol) 1-tert.Butoxycarbonyl-3-hydroxypyrrolidin und 11,5 g (44 mmol) Triphenylphosphin in 100 ml trockenem Tetrahydrofuran wurden bei 0°C 7,66 g (44 mmol) Azodicarbonsäurediethylester in 20 ml trockenem Tetrahydrofuran getropft. Nach 2 Stunden bei Raumtemperatur wurde eingeengt und der Rückstand durch zweimalige Flashchromatographie (Toluol-Essigester-Gradient) gereinigt. Man erhielt 7,3 g rötliches Öl, das durch Verreiben mit Ether zur Kristallisation gebracht wurde.
Ausbeute: 4,7 g (40%)
Schmp.: 145-148°C
$R_f$ = 0,33 (Toluol/Essigester 3:1, Kieselgelplatten)

Beispiele 71 bis 87

Die Beispiele 71 bis 87 sind in der Tabelle 3 zusammengefaßt; sie werden analog zu den Beispielen 69/70 erhalten.

Legende zu Tabelle 3

a) Verwendete Abkürzungen:
BN = Phenylmethyl
BOC = tert.Butoxycarbonyl
b) Sofern nicht anders vermerkt fielen die Produkte als Öle an.
Verwendete Abkürzungen der DC-Systeme: Kieselgelplatten, Laufmittel
TE 31 = Toluol/Essigester 3:1
TE 11 = Toluol/Essigester 1:1
TM 41 = Toluol/Methanol 4:1
c) Verwendete Abkürzungen:
X = 1-tert.Butoxycarbonyl-3-hydroxy-pyrrolidin (erhalten durch Umsetzung von 3-Hydroxypyrrolidin mit Pyrokohlensäure-di-tert.butylester in Tetrahydrofuran/Methanol 5:1)
A besitzt die in der Spalte "A" angegebene Bedeutung

d) (-)-Enantiomeres; $\alpha_D^{20}$ = -6,8 ($CH_3OH$; c = 0,93)

e) (-)-1-Benzyl-3-hydroxy-pyrrolidin [J. Med. Chem. 29, 2504 (1986)].

f) Umsetzung von Beispiel 76 mit Ammoniak (gasförmig) in Ethanol bei 0°C bis Raumtemperatur.

g) Enthält noch unumgesetztes 2-Naphthol; als Gemisch weiter umgesetzt.

h) Umsetzung von Beispiel 82 mit Dimethylsulfat und Natriumhydrid in Dimethylformamid bei 0°C → Raumtemperatur

i) Schmelzpunkt 160-162°C

k) Kieselgelplatten; Laufmittel = Essigester;
MS (m/e) = 314, 257, 241, 169

l) Durchführung in THF/Acetonitril 1:2,5 bei 40° bis 50°C

m) S(+)-Enantiomer; $\alpha_D^{20}$ = +32,4 (c=1; $CH_3OH$)

n) R(-)-1-tert.-Butoxycarbonyl-3-hydroxy-pyrrolidin [$\alpha_D^{20}$ = -25,4 (c=1; $CH_3OH$)], Heterocycles 1987, 2247;

**Tabelle 3**: Verbindungen der allgemeinen Formel (IV)

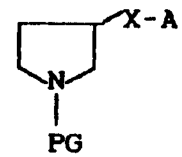

| Bsp. | -X-(A) | -A | PG[a)] | $R_f$/Solvens [b)] | hergestellt aus[c)] |
|---|---|---|---|---|---|
| 71 | $-OCH_2-$ | Phenyl | BOC | 0,49 (TE 31) | X + $A-CH_2Br$ |
| 72 | -O- | 2-Cyanophenyl | BOC | 0,27 (TE 31) | X + A-OH |
| 73 | -O- | (2-Methoxy-6-cyano)-phenyl | BOC | 0,42 (TE 31) | X + A-OH |
| 74[d)] | -O- | 2-Methoxyphenyl | BN | 0,38 (TE 11) | [e)] + A-OH |
| 75 | -O- | (2-Carbamoyl)-naphth-1-yl | BOC | 0,3 (TM 41) | Bsp.76 + $NH_3$ [f)] |
| 76 | -O- | (2-Phenoxycarbonyl)-naphth-1-yl | BOC | 0,46 (TE 31) | X + A-OH |
| 77 | -O- | Naphth-1-yl | BOC | 0,63 (TE 31) | X + A-OH |
| 78 | -O- | Naphth-2-yl | BOC | 0,6[g)] (TE 31) | X + A-OH |
| 79 | $-OCH_2-$ | Naphth-2-yl | BOC | 0,43 (TE 31) | X + $A-CH_2Cl$ |

Fortsetzung Tabelle 3

| Bsp. | -X-(A) | -A | PG[a] | $R_f$/Solvens [b] | hergestellt aus[c] |
|---|---|---|---|---|---|
| 80 | $-OCH_2-$ | 2-Fluorphenyl | BOC | 0,48 (TE 31) | X + $A-CH_2Br$ |
| 81 | -O- | (1-Methyl)indol-4-yl | BOC | 0,48 (TE 31) | Bsp.82 + $(CH_3)_2SO_4$ [h] |
| 82 | -O- | Indol-4-yl | BOC | 0,42 (TE 31)[i] | X + A-OH |
| 83 | -O- | 5,6,7,8-Tetrahydro-naphth-1-yl | BOC | 0,58 (TE 31) | X + A-OH |
| 84 | -O- | Chinolin-2-yl | BOC | 0,87[k] | X + A-OH |
| 85 | -O- | Isochinolin-1-yl | BOC | 0,58 (TM 41) | X + A-OH [l] |
| 86[m] | -O- | 2-Methoxyphenyl | BOC | 0,49 (TE 31) | n) + A-OH |
| 87 | -O- | 2-(Prop-2-en-1-yloxy)-phenyl | BOC | 0,52 (TE 31) | X + A-OH |

Anwendungsbeispiel

Beispiel 69

Affinität zum 5-$HT_1$-Rezeptor

In Tabelle 4 wird beispielhaft die hohe Affinität der erfindungsgemäßen Verbindung zu 5-Hydroxytryptamin-Rezeptoren vom Subtyp 1 dargestellt. Bei den angegebenen Werten handelt es sich um Daten, die aus Rezeptorbindungsstudien mit Kalbs-Hippocampus-Membranpräparaten ermittelt wurden. Als radioaktiv markierter Ligand wurde hierzu $^3H$-Serotonin verwendet.

Tabelle 4

| Verbindung des Beispiels-Nr. | Ki (nmol/l) |
|---|---|
| 1 | 2 |
| 6 | 9 |
| 8 | 1,3 |
| 10 | 6 |
| 19 | 7 |
| 23 | 0,3 |

Vergleich:

In der DE-A 1 964 511 wurden Pyrrolidine mit
A gleich o-Methoxy-phenyl,
X gleich Sauerstoff,
B gleich 2-Propyl und
n gleich 0
beschrieben.
Diese Verbindung hat einen Ki-Wert von 1,0 µmol/l.

## Patentansprüche

**Patentansprüche für folgende Vertragsstaaten : AT, BE, CH, DE, FR, GB, GR, IT, LI, NL, SE**

**1.** 1,3-Disubstituierte Pyrrolidine der allgemeinen Formel

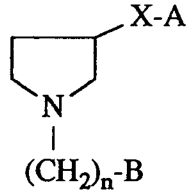

(I)

worin

A für Phenyl oder einen monocyclischen fünf- oder sechsgliedriger Hetarylrest, der ein oder zwei Stickstoff- und/oder Sauerstoff- und/oder Schwefelatome enthält, steht, an die gegebenenfalls ein bis drei aromatische gesättigte oder ungesättigte cyclische Kohlenwasserstoffe mit 5 bis 8 Kohlenstoffatomen oder fünf- oder sechs-gliedrige Heterocyclen mit einem oder zwei Stickstoff- und/oder Sauerstoff- und-/oder Schwefelatomen kondensiert sind, wobei dieser Rest gegebenenfalls ein- oder zweifach durch $C_1$-$C_6$-Alkyl, $C_1$-$C_6$-Alkoxy, $C_1$-$C_6$-Alkoxycarbonyl, $C_1$-$C_6$-Alkenyloxy, Acyloxy, Benzoyloxy, Cyano, Phenyl, Benzyl, Fluor, Chlor, Brom, Trifluormethyl, Trifluormethoxy, Difluormethoxy oder einen Rest der Formel -CO-NYZ, -NH-$SO_2$-Y', -$SO_2$-NYZ oder -NH-CO-Y substituiert ist, wobei

Y, Z gleich oder verschieden sind und für Wasserstoff oder $C_1$-$C_6$-Alkyl stehen, und

Y' für $C_1$-$C_6$-Alkyl oder Aryl mit 6 bis 12 Kohlenstoffatomen steht,

und im Fall von Stickstoffheterocyclen gegebenenfalls als N-Oxid vorliegt,

X - für -$CH_2$-O-, -O-$CH_2$- oder -O- steht,

B - für Cyano oder eine Gruppe der Formel -$COOR^1$, $CONR^2R^3$, -$SO_2NR^2R^3$, -$SO_mR^4$, $NR^5R^6$ oder -C≡C-$CH_2$-$NR^5R^6$ steht, wobei

$R^1$ - für Wasserstoff, $C_1$-$C_{11}$-Alkyl, $C_5$-$C_8$-Cycloalkyl, $C_2$-$C_{12}$-Alkenyl, $C_6$-$C_{12}$Aryl oder $C_7$-$C_{14}$-Aralkyl steht,

$R_2$ und $R_3$ gleich oder verschieden sind und

- für Wasserstoff, $C_1$-$C_{12}$-Alkyl, $C_5$-$C_8$-Cycloalkyl, $C_6$-$C_{12}$-Aryl oder $C_7$-$C_{14}$-Aralkyl stehen, wobei die Arylreste durch Halogen, $C_1$-$C_6$-Alkoxy oder $C_1$-$C_6$-Alkoxycarbonyl substituiert sein können,

$R^4$ - für $C_1$-$C_{12}$-Alkyl, $C_5$-$C_8$-Cycloalkyl, $C_6$-$C_{12}$-Aryl oder $C_7$-$C_{14}$-Aralkyl steht, wobei die Arylreste bis zu 3-fach gleich oder verschieden durch Halogen, Cyano, $C_1$-$C_6$-Alkyl, $C_1$-$C_6$-Alkoxy, Trifluormethyl oder Trifluormethoxy substituiert sein können, oder

- für eine Gruppe der Formel

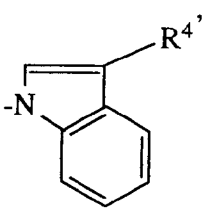

steht,

worin $R^{4'}$ Wasserstoff oder $C_1$-$C_6$-Alkyl bedeutet

m - für eine Zahl 0, 1 oder 2 steht,

$R^5$ und $R^6$ gleich oder verschieden sind und

- für Wasserstoff, $C_1$-$C_{12}$-Alkyl, $C_5$-$C_8$ Cycloalkyl, $C_6$-$C_{12}$-Aryl oder $C_7$-$C_{14}$-Aralkyl stehen, wobei die Arylreste durch Halogen, Cyano, $C_1$-$C_6$-Alkyl, $C_1$-$C_6$-Alkoxy oder Trifluormethyl substituiert sein können, oder

- für eine Gruppe der Formel -$COR^7$ oder -$SO_2R^8$ stehen,

worin

$R^7$

- Wasserstoff oder
- eine Gruppe $NHR^9$ oder
- $C_1$-$C_{11}$-Alkyl, $C_5$-$C_8$-Cycloalkyl oder $C_1$-$C_{12}$-Alkoxy, oder
- $C_6$-$C_{12}$-Aryl, $C_6$-$C_{12}$-Aryloxy, $C_7$-$C_{14}$-Aralkyl, $C_6$-$C_{12}$-Aralkoxy oder einen fünf-oder sechsgliedrigen Heteroaryl-Ring mit ein oder zwei Sauerstoff- und/oder Stickstoff- und/oder Schwefelatomen bedeutet, wobei die genannten Reste bis zu 3-fach gleich oder verschieden durch $C_1$-$C_6$-Alkyl, $C_1$-$C_6$-Alkoxy, $C_1$-$C_6$-Alkylthio, Halogen, Cyano, Trifluormethyl, Trifluormethoxy, Trifluormethylthio, Amino, $C_1$-$C_6$-Alkyl-amino oder $C_1$-$C_6$-Dialkyl-amino substituiert sein können,

$R^8$

- $C_5$-$C_8$-Cycloalkyl, oder
- $C_1$-$C_{12}$-Alkyl, das durch Cyano, Halogen, $C_1$-$C_{12}$-Alkoxy oder $C_1$-$C_{12}$Alkoxy-carbonyl substituiert sein kann, oder
- $C_6$-$C_{12}$-Aryl, $C_7$-$C_{14}$-Aralkyl oder einen fünf- oder sechsgliedrigen Heteroaryl-Ring mit ein

oder zwei Sauerstoff- und/oder Stickstoff- und/oder Schwefelatomen bedeutet, wobei die genannten Reste bis zu 3-fach gleich oder verschieden durch $C_1$-$C_6$-Alkyl, $C_1$-$C_6$-Alkoxy, $C_1$-$C_6$-Alkylthio, Halogen, Cyano, Trifluormethyl, Trifluormethoxy, Trifluormethylthio, Nitro, Amino, $C_1$-$C_6$-Alkyl-amino oder $C_1$-$C_6$-Dialkyl-amino substituiert sein können,
oder

- eine Gruppe $NR^2R^3$ bedeutet, wobei
  $R^2$ und $R^3$ die oben angegebene Bedeutung haben und

$R^9$

- Wasserstoff, oder
- $C_5$-$C_8$-Cycloalkyl, oder
- gegebenenfalls durch Cyano, Fluor, Chlor oder Brom substituiertes $C_1$-$C_{12}$-Alkyl, oder
- $C_6$-$C_{12}$-Aryl, $C_7$-$C_{14}$-Aralkyl, einen fünf- oder sechsgliedrigen Heteroaryl- Ring mit ein oder zwei Sauerstoff- und/oder Stickstoff- und/oder Schwefelatomen bedeutet, wobei die Arylreste bis zu 3-fach gleich oder verschieden durch $C_1$-$C_6$-Alkyl, $C_1$-$C_6$-Alkoxy, $C_1$-$C_6$-Alkylthio, Halogen, Cyano, Trifluormethyl, Trifluormethoxy, Trifluormethylthio, Nitro, Amino, $C_1$-$C_6$-Alkyl-amino oder $C_1$-$C_6$-Dialkylamino substituiert sein können,

oder wobei
$R^5$ und $R^6$ zusammen mit dem Stickstoffatom einen Ring der Reihe

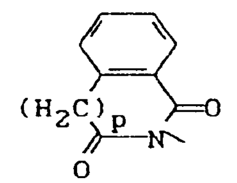 , 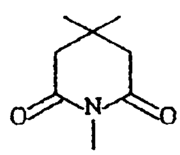 , 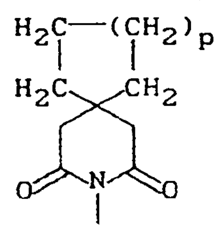 ,

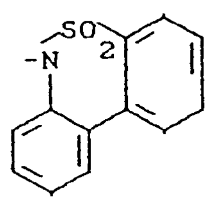 , 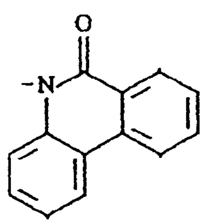 , 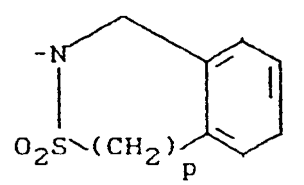 ,

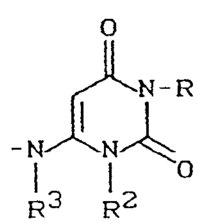 , 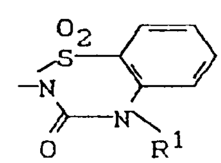 , 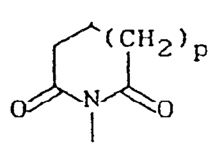 , 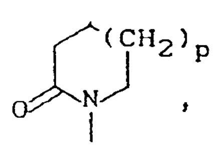 ,

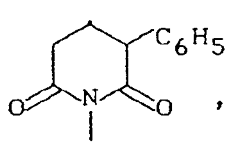 , 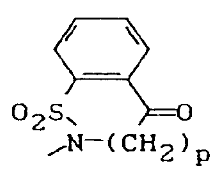 , 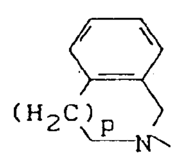 , 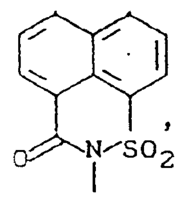 ,

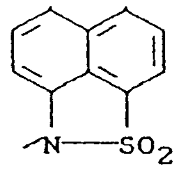 , 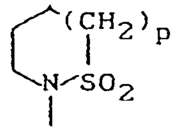 oder 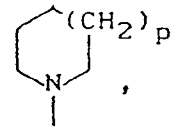 ,

bilden,

worin

p - eine Zahl 0, 1 oder 2 bedeutet,

$R^1$, $R^2$ und $R^3$ die oben angegebene Bedeutung haben,

n - eine Zahl von 1 bis 10 bedeutet

und deren Salze.

**2.** 1,3-Disubstituierte Pyrrolidine nach Anspruch 1 worin

A - für Phenyl oder einen monocyclischen fünf- oder sechsgliedrigen Hetarylrest, der ein oder zwei Stickstoff- und/oder Sauerstoff- und/oder Schwefelatome enthält, steht, an die gegebenenfalls ein bis drei aromatische, gesättigte oder ungesättigte cyclische Kohlenwasserstoffe mit 5 bis 8 Kohlenstoffatomen oder fünf- oder sechsgliedrige Heterocyclen mit einem oder zwei Stickstoffund/oder Sauerstoff- und/oder Schwefelstomen kondensiert sind, wobei dieser Rest gegebenenfalls ein- oder zweifach durch $C_1$-$C_6$-Alkyl, $C_1$-$C_6$-Alkoxy, $C_1$-$C_6$-Alkoxycarbonyl, $C_1$-$C_6$-Alkenyloxy, Acyloxy, Benzoyloxy, Cyano, Phenyl, Benzyl, Fluor, Chlor, Brom, Trifluormethyl, Trifluormethoxy, Difluormethoxy oder einen Rest der Formel -CO-NYZ, -NH-$OS_2$-Y', -$SO_2$-NYZ oder -NH-CO-Y substituiert ist,

wobei

Y,Z gleich oder verschieden sind und für Wasserstoff, oder $C_1$-$C_6$-Alkyl stehen und

Y' für $C_1$-$C_6$-Alkyl oder Aryl mit 6 bis 12 Kohlenstoffatomen steht,

und im Fall von Stickstoffheterocyclen gegebenenfalls als N-Oxid vorliegt,

X - für -$CH_2$-O-, -O-$CH_2$- oder -O- steht,

B - für Cyano oder eine Gruppe der Formel -$COOR^1$, $CONR^2R^3$, -$SO_2NR^2R^3$, -$SO_mR^4$, $NR^5R^6$ oder -C≡C-$CH_2$-$NR^5R^6$ steht, wobei

$R^1$ - für Wasserstoff, $C_1$-$C_6$-Alkyl oder Phenyl steht,

$R^2$ und $R^3$ gleich oder verschieden sind und

- für Wasserstoff, $C_1$-$C_6$-Alkyl oder Phenyl stehen, das seinerseits durch Fluor, Chlor, Brom, $C_1$-$C_6$-Alkoxy oder $C_1$-$C_6$-Alkoxycarbonyl substituiert sein kann,

$R^4$ - für $C_1$-$C_6$-Alkyl, oder

- für Phenyl steht, das bis zu zweifach gleich oder verschieden durch Fluor, Chlor, Brom, $C_1$-$C_6$-Alkyl oder $C_1$-$C_6$-Alkoxy substituiert sein kann, oder

- für eine Gruppe der Formel

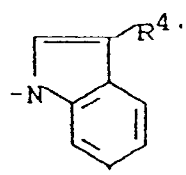

steht, worin $R^{4'}$ Wasserstoff oder $C_1$-$C_6$-Alkyl bedeutet,

m - für eine Zahl 0, 1 oder 2 steht,

$R^5$ und $R^6$ gleich oder verschieden sind und

- für Wasserstoff, $C_1$-$C_6$-Alkyl, Phenyl oder Benzyl stehen, wobei die Phenylreste durch Fluor, Chlor, Brom, $C_1$-$C_6$-Alkyl, $C_1$-$C_6$-Alkoxy oder Trifluormethyl substituiert sein können, oder

- für eine Gruppe der Formel -$COR^7$ oder -$SO_2R^8$ stehen,

worin

$R^7$

- Wasserstoff bedeutet, oder
- eine Gruppe $NHR^9$ bedeutet, oder
- $C_1$-$C_6$-Alkyl oder $C_1$-$C_6$-Alkoxy bedeutet, oder
- gegebenenfalls durch $C_1$-$C_6$-Alkyl, $C_1$-$C_6$-Alkoxy, Fluor, Chlor, Brom, Trifluormethyl,

Dimethylamino oder Diethylamino substituiertes Phenyl, Benzyl, Benzyloxy, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,

$R^8$ - Cyclopropyl, Cyclopentyl, Cyclohexyl, oder

- gegebenenfalls durch Cyano, Fluor, Chlor, Brom, Trifluormethyl oder $C_1$-$C_6$-Alkoxycarbonyl substituiertes $C_1$-$C_6$-Alkyl bedeutet, oder

- gegebenenfalls durch $C_1$-$C_6$-Alkyl, $C_1$-$C_6$-Alkoxy, Fluor, Chlor, Brom, Nitro, Trifluormethyl, Dimethylamino oder Diethylamino substituiertes Phenyl, Naphthyl, Benzyl, Thienyl, Furyl, Pyrimidyl, Pyridyl, Chinolyl, Isochinolyl, Benzothiazolyl, Benzoxazolyl, Thiazolyl, Oxazolyl, Isoxazolyl oder Isothiazolyl bedeutet, oder

- eine Gruppe $NR^2R^3$ bedeutet,

wobei

$R^2$ und $R^3$ die oben angegebene Bedeutung haben, und

$R^9$

- gegebenenfalls durch Cyano, Fluor, Chlor oder Brom substituiertes $C_1$-$C_6$-Alkyl bedeutet, oder

- gegebenenfalls durch $C_1$-$C_6$-Alkyl, $C_1$-$C_6$-Alkoxy, Fluor, Chlor, Brom, Trifluormethyl, Dimethylamino oder Diethylamino substituiertes Phenyl, Benzyl, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,

oder

$R^5$ und $R^6$ zusammen mit dem Stickstoffatom einen Ring der Reihe

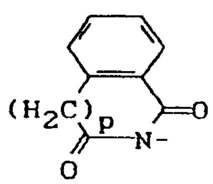 , 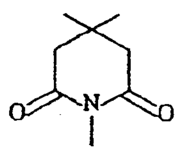 , 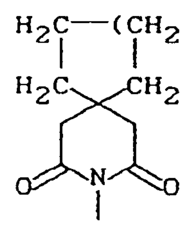 ,

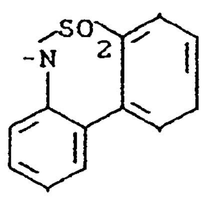 , 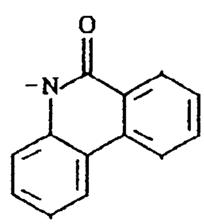 , 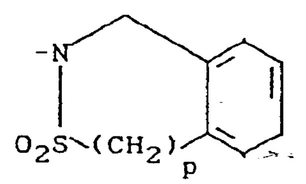 ,

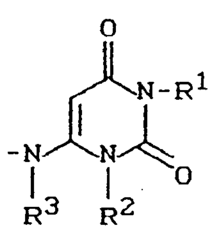 , 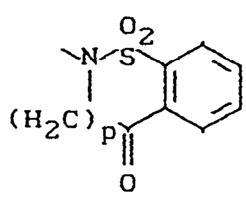 , 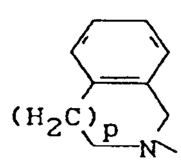 , 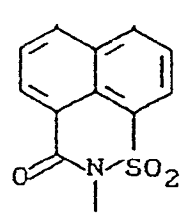

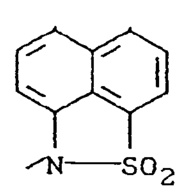 , 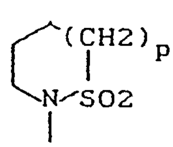 , 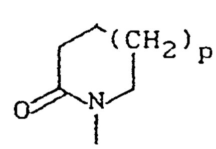 ,

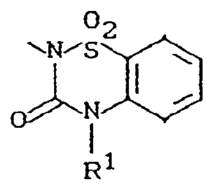 oder 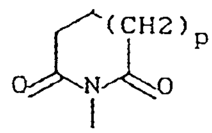 bilden,

worin
P - eine Zahl 0, 1 oder 2 bedeutet,
$R^1$, $R^2$ und $R^3$ die oben angegebene Bedeutung haben und
n - eine Zahl 1 bis 8 bedeutet,
und deren Salze.

**3.** 1,3-Disubstituierte Pyrrolidine nach Anspruch 1 wobei
A - für gegebenenfalls gleich oder verschieden durch $C_1$-$C_6$-Alkyl, $C_1$-$C_6$-Alkoxy, $C_1$-$C_6$-Alkoxycarbonyl, $C_1$-$C_6$-Alkenyloxy, Acyloxy, Benzoyloxy, Cyano, Phenyl, Benzyl, Sulfonylamino, Sulfamoyl ($C_1$ bis $C_6$), Carbamoyl, Carbonylamino, Fluor, Chlor, Brom, Trifluormethyl, Trifluormethoxy, Difluormethoxy, substituiertes Phenyl oder Naphthyl, Furan, Thiophen, Isoxazol, Pyridin, Pyrimidin, Indol, Indazol, Benzofuran, Benzisoxazol, Chinolin, Isochinolin, Tetralin, Inden, Chroman, Dihydrobenzodioxin, Dihydroindol, Tetrahydrochinolin oder Dihydrobenzofuran steht,
X - für $-O-CH_2-$, $-CH_2-O-$ oder -O- steht,
B - für Cyano, oder
- eine Gruppe der Formel $-CONR^2R^3$, $-NR^5R^6$, $-SO_mR^4$, $-C\equiv C-CH_2-NR^5R^6$, $-SO_2NR^2R^3$, $COOR^1$ steht, wobei
$R^1$ - Wasserstoff, Methyl, Ethyl oder Phenyl bedeutet,
$R^2$ und $R^3$ gleich oder verschieden sind und
- Wasserstoff, Methyl, Ethyl, Propyl, Isopropyl, Butyl, Isobutyl bedeuten oder Phenyl bedeuten, das durch Methoxycarbonyl substituiert sein kann
$R^4$
- für Methyl oder Ethyl steht oder
- für eine Gruppe der Formel

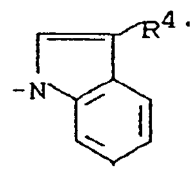

steht, worin $R^{4'}$ Wasserstoff oder Methyl bedeutet,
$R^5$ und $R^6$ gleich oder verschieden sind, und
- Wasserstoff, Methyl, Ethyl, Propyl, Isopropyl, Butyl, Isobutyl, oder
- gegebenenfalls durch Fluor, Chlor, Methyl oder Methoxy substituiertes Phenyl, oder
- eine Gruppe $-COR^7$ oder $-SO_2R^8$ bedeuten,
worin
$R^7$
- für eine Gruppe $NHR^9$ steht, oder
- für Methyl, Ethyl, Propyl, Isopropyl, Methoxy, Ethoxy, Propoxy, Isopropoxy, tert.Butoxy oder
- für gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl steht,
$R^8$
- für gegebenenfalls durch Fluor, Chlor, Methoxycarbonyl, Ethoxycarbonyl, Propoxycarbonyl, Isopropoxycarbonyl, Butoxycarbonyl, Isobutoxycarbonyl oder tert.-Butoxycarbonyl, substituiertes Methyl, Ethyl, Propyl, Isopropyl, Butyl oder Isobutyl steht, oder
- für gegebenenfalls durch Methyl, Ethyl, Propyl, Isopropyl, Methoxy, Fluor, Chlor, Nitro substituiertes Phenyl, Naphthyl, Thienyl, Furyl, Pyridyl, Pyrimidyl, Chinolyl oder Isochinolyl steht, oder
- für eine Gruppe $NR^2R^3$ steht, wobei

$R^2$ und $R^3$ die oben angegebene Bedeutung haben,

$R^9$

- gegebenenfalls durch Fluor oder Chlor substituiertes Methyl, Ethyl, Propyl, Isopropyl, Butyl, Isobutyl, Pentyl, Isopentyl, Hexyl oder Isohexyl bedeutet, oder
- Phenyl bedeutet, das durch Fluor, Chlor, Methyl oder Methoxy substituiert sein kann, oder

$R^5$ und $R^6$ gemeinsam mit dem Stickstoffatom einen Ring der Reihe

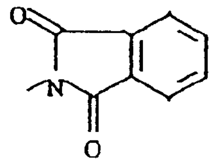 , 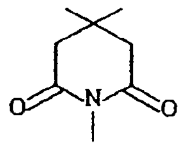 , 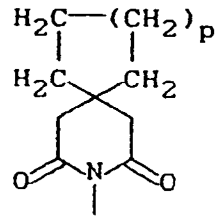 ,

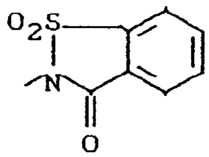 , 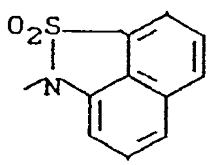 , 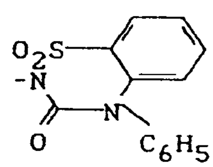 ,

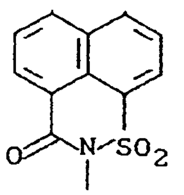 , 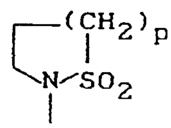 , 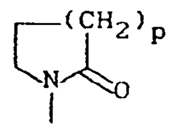 ,

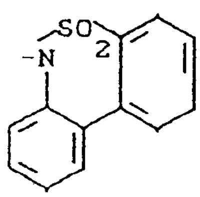 , 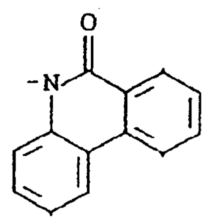 , 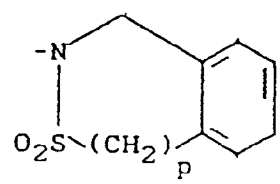 ,

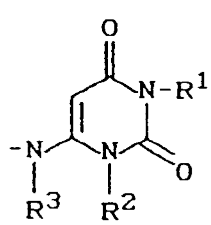 oder 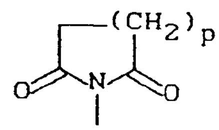

bilden, worin

p - eine Zahl 1 oder 2 bedeutet

$R^1$, $R^2$ und $R^3$ die oben angegebene Bedeutung haben und

n - eine Zahl 1 bis 6 bedeutet

und deren Salze.

**4.** 1,3-Disubstituierte Pyrrolidine nach den Ansprüchen 1 bis 3, zur therapeutischen Behandlung.

**5.** Verfahren zur Herstellung von 1,3-disubstituierten Pyrrolidinen der allgemeinen Formel

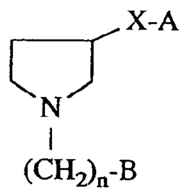

(I)

worin

A für Phenyl oder ein monocyclischer fünf- oder sechsgliedriger Hetarylrest, der ein oder zwei Stickstoff- und/oder Sauerstoff- und/oder Schwefelatome enthält, steht, an die gegebenenfalls ein bis drei aromatische gesättigte oder ungesättigte cyclische Kohlenwasserstoffe mit 5 bis 8 Kohlenstoffatomen oder fünf- oder sechs-gliedrige Heterocyclen mit einem oder zwei Stickstoff- und/oder Sauerstoff- und/oder Schwefelatomen kondensiert sind, wobei dieser Rest gegebenenfalls ein- oder zweifach durch $C_1$-$C_6$-Alkyl, $C_1$-$C_6$-Alkoxy, $C_1$-$C_6$-Alkoxycarbonyl, $C_1$-$C_6$-Alkenyloxy, Acyloxy, Benzoyloxy, Cyano, Phenyl, Benzyl, Fluor, Chlor, Brom, Trifluormethyl, Trifluormethoxy, Difluormethoxy oder einen Rest der Formel -CO-NYZ, -NH-$SO_2$-Y', -$SO_2$-NYZ oder -NH-CO-Y substituiert ist, wobei

Y,Z gleich oder verschieden sind und für Wasserstoff oder $C_1$-$C_6$-Alkyl stehen, und

Y' für $C_1$-$C_6$-Alkyl oder Aryl mit 6 bis 12 Kohlenstoffatomen steht,

und im Fall von Stickstoffheterocyclen gegebenenfalls als N-Oxid vorliegt,

X - für -$CH_2$-O-, O-$CH_2$- oder -O- steht,

B - für Cyano oder eine Gruppe der Formel -$COOR^1$, $CONR^2R^3$, -$SO_2NR^2R^3$, -$SO_mR^4$, $NR^5R^6$ oder -C≡C-$CH_2$-$NR^5R^6$ steht, wobei

$R^1$ - für Wasserstoff, $C_1$-$C_{11}$-Alkyl, $C_5$-$C_8$-Cycloalkyl, $C_2$-$C_{12}$-Alkenyl, $C_6$-$C_{12}$-Aryl oder $C_7$-$C_{14}$-Aralkyl steht,

$R_2$ und $R_3$ gleich oder verschieden sind und

- für Wasserstoff, $C_1$-$C_{12}$-Alkyl, $C_5$-$C_8$-Cycloalkyl, $C_6$-$C_{12}$-Aryl oder $C_7$-$C_{14}$-Aralkyl stehen, wobei die Arylreste durch Halogen, $C_1$-$C_6$-Alkoxy oder $C_1$-$C_6$-Alkoxycarbonyl substituiert sein können,

$R^4$ - für $C_1$-$C_{12}$-Alkyl, $C_5$-$C_8$-Cycloalkyl, $C_6$-$C_{12}$-Aryl oder $C_7$-$C_{14}$-Aralkyl steht, wobei die Arylreste bis zu 3-fach gleich oder verschieden durch Halogen, Cyano, $C_1$-$C_6$-Alkyl, $C_1$-$C_6$-Alkoxy, Trifluormethyl oder Trifluormethoxy substituiert sein können, oder

- für eine Gruppe der Formel

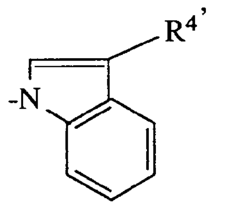

steht,

worin $R^{4'}$ Wasserstoff oder $C_1$-$C_6$-Alkyl bedeutet

m - für eine Zahl 0, 1 oder 2 steht,

$R^5$ und $R^6$ gleich oder verschieden sind und

- für Wasserstoff, $C_1$-$C_{12}$-Alkyl, $C_5$-$C_8$ Cycloalkyl, $C_6$-$C_{12}$-Aryl oder $C_7$-$C_{14}$- Aralkyl stehen, wobei die Arylreste durch Halogen, Cyano, $C_1$-$C_6$-Alkyl, $C_1$-$C_6$-Alkoxy oder Trifluormethyl substituiert sein können, oder

- für eine Gruppe der Formel -$COR^7$ oder -$SO_2R^8$ stehen,

worin

$R^7$

- Wasserstoff oder
- eine Gruppe $NHR^9$ oder
- $C_1$-$C_{11}$-Alkyl, $C_5$-$C_8$-Cycloalkyl oder $C_1$-$C_{12}$-Alkoxy, oder

- $C_6$-$C_{12}$-Aryl, $C_6$-$C_{12}$-Aryloxy, $C_7$-$C_{14}$-Aralkyl, $C_6$-$C_{12}$-Aralkoxy oder einen fünf-oder sechsgliedrigen Heteroaryl-Ring mit ein oder zwei Sauerstoff- und/oder Stickstoffund/oder Schwefelatomen bedeutet, wobei die genannten Reste bis zu 3-fach gleich oder verschieden durch $C_1$-$C_6$-Alkyl, $C_1$-$C_6$-Alkoxy, $C_1$-C-$_6$-Alkylthio, Halogen, Cyano, Trifluormethyl, Trifluormethoxy, Trifluormethylthio, Amino, $C_1$-$C_6$-Alkylamino oder $C_1$-$C_6$-Dialkyl-amino substituiert sein können,

$R^8$

- $C_5$-$C_8$-Cycloalkyl, oder
- $C_1$-$C_{12}$-Alkyl, das durch Cyano, Halogen, $C_1$-$C_{12}$-Alkoxy oder $C_1$-C-$_{12}$Alkoxycarbonyl substituiert sein kann, oder
- $C_6$-$C_{12}$-Aryl, $C_7$-$C_{14}$-Arylkyl oder einen fünf- oder sechsgliedrigen Heteroaryl-Ring mit ein oder zwei Sauerstoff- und/oder Stickstoff- und/oder Schwefelatomen bedeutet, wobei die genannten Reste bis zu 3-fach gleich oder verschieden durch $C_1$-$C_6$-Alkyl, $C_1$-$C_6$-Alkoxy, $C_1$-$C_6$-Alkylthio, Halogen, Cyano, Trifluormethyl, Trifluormethoxy, Trifluormethylthio, Nitro, Amino, $C_1$-$C_6$-Alkyl-amino oder $C_1$-$C_6$-Dialkyl-amino substituiert sein können, oder
- eine Gruppe $NR^2R^3$ bedeutet, wobei

  $R^2$ und $R^3$ die oben angegebene Bedeutung haben und

$R^9$

- Wasserstoff, oder
- $C_5$-$C_8$-Cycloalkyl, oder
- gegebenenfalls durch Cyano, Fluor, Chlor oder Brom substituiertes $C_1$-$C_{12}$-Alkyl,

oder

- $C_6$-$C_{12}$-Aryl, $C_7$-$C_{14}$-Aralkyl, einen fünf- oder sechsgliedrigen Heteroaryl-Ring mit ein oder zwei Sauerstoff- und/oder Stickstoff- und/oder Schwefelatomen bedeutet, wobei die Arylreste bis zu 3 - fach gleich oder verschieden durch $C_1$-$C_6$-Alkyl, $C_1$-$C_6$-Alkoxy, $C_1$-$C_6$-Alkylthio, Halogen, Cyano, Trifluormethyl, Trifluormethoxy, Trifluormethylthio, Nitro, Amino, substituiert sein können,

oder wobei

$R^5$ und $R^6$ zusammen mit dem Stickstoffatom einen Ring der Reihe

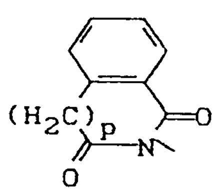 , 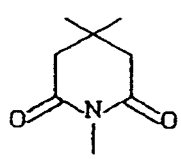 , 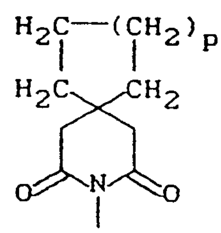 ,

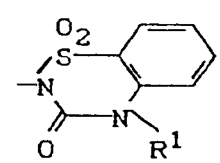 , 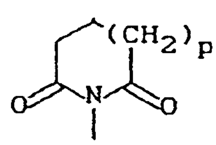 , 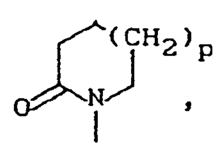 , 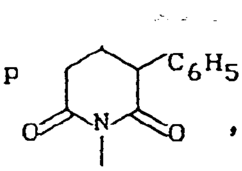 ,

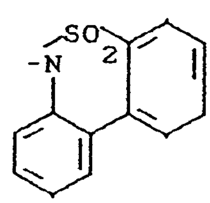 , 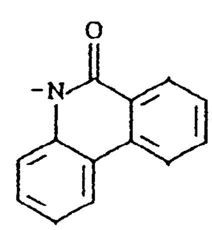 , 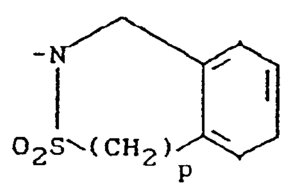 ,

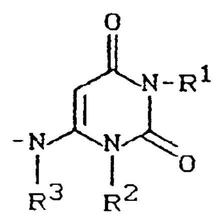 , 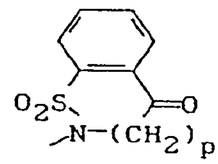 , 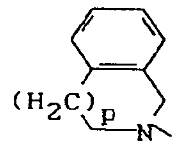 ,

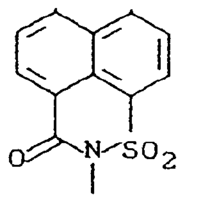 , 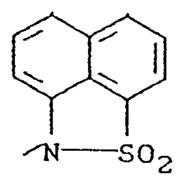 , 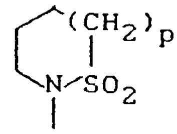 oder 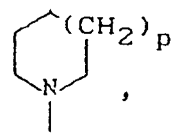 ,

bilden,
worin
p - eine Zahl 0, 1 oder 2 bedeutet,
$R^1$, $R^2$ und $R^3$ die oben angegebene Bedeutung haben,
n - eine Zahl von 1 bis 10 bedeutet
und deren Salze, dadurch gekennzeichnet, daß man
3-substituierte Pyrrolidine der allgemeinen Formel (II)

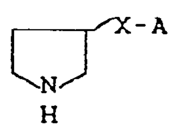 (II)

in welcher A und X die oben angegebene Bedeutung besitzen,
oder ihre Salze
in einer ersten Stufe mit Alkylderivaten der allgemeinen Formel (III)

$$R\text{-}(CH_2)_n\text{-}B' \qquad (III)$$

worin
n - die oben angegebene Bedeutung hat,

B' dem Bedeutungsumfang von B entspricht, wobei jedoch $R^5$ und $R^6$ nicht gleichzeitig für Wasserstoff oder gleichzeitig für Wasserstoff und Alkyl oder Aryl stehen,

R - für Chlor, Brom, Jod, Methylsulfonyloxy, Phenylsulfonyloxy, Tolylsulfonyloxy, Trifluoracetoxy oder Trifluormethylsulfonyloxy steht,

in inerten Lösemitteln, gegebenenfalls in Anwesenheit von Basen, umsetzt,

dann gegebenenfalls funktionelle Gruppen durch Reduktion, Oxidation, Hydrolyse oder Umsetzung mit nucleophilen oder elektrophilen Reagentien in andere funktionelle Gruppen überführt und dann, im Fall der Herstellung der Salze, gegebenenfalls mit der entsprechenden Säure umsetzt.

6. Verfahren nach Anspruch 5, dadurch gekennzeichnet, daß man die Umsetzung im Temperaturbereich von -20°C bis + 150°C durchführt.

7. Verfahren zur Herstellung von 1,3-disubstituierten Pyrrolidinen der allgemeinen Formel

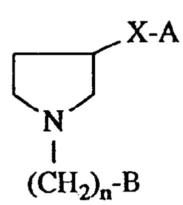 (I)

worin

A für Phenyl oder ein monocyclischer fünf- oder sechsgliedriger Hetarylrest, der ein oder zwei Stickstoff- und/oder Sauerstoff- und/oder Schwefelatome enthält, steht, an die gegebenenfalls ein bis drei aromatische gesättigte oder ungesättigte cyclische Kohlenwasserstoffe mit 5 bis 8 Kohlenstoffatomen oder fünf- oder sechs-gliedrige Heterocyclen mit einem oder zwei Stickstoff- und/oder Sauerstoff- und/oder Schwefelatomen kondensiert sind, wobei dieser Rest gegebenenfalls ein- oder zweifach durch $C_1$-$C_6$-Alkyl, $C_1$-$C_6$-Alkoxy, $C_1$-$C_6$-Alkoxycarbonyl, $C_1$-$C_6$-Alkenyloxy, Acyloxy, Benzoyloxy, Cyano, Phenyl, Benzyl, Fluor, Chlor, Brom, Trifluormethyl, Trifluormethoxy, Difluormethoxy oder einen Rest der Formel -CO-NYZ, -NH-$SO_2$-Y′, -$SO_2$-NYZ oder -NH-CO-Y substituiert ist, wobei

Y,Z gleich oder verschieden sind und für Wasserstoff oder $C_1$-$C_6$-Alkyl stehen, und
Y′ für $C_1$-$C_6$-Alkyl oder Aryl mit 6 bis 12
Kohlenstoffatomen steht,

und im Fall von Stickstoffheterocyclen gegebenenfalls als N-Oxid vorliegt,
X - für -$CH_2$-O-, O-$CH_2$- oder -O- steht,
und
B - für Cyano oder eine Gruppe der Formel -$SO_2NR^2R^3$ oder -$SO_mR^4$ bedeutet,
wobei
$R^2$, $R^3$, $R^4$ und m die in Anspruch 1 angegebene Bedeutung hat
dadurch gekennzeichnet, daß man 3-substituierte Pyrrolidine der allgemeinen Formel (II)

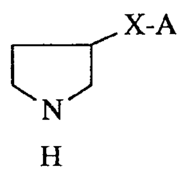

(II)

worin

A und X die obengenannte Bedeutung haben mit Acrylnitril oder einer Verbindung der Formel $CH_2$=CH-$SO_2NR^2R^3$ oder -$CH_2$=$CH_2$-$SO_mR^4$ in Gegenwart von Katalysatoren umsetzt.

**8.** Verfahren nach Anspruch 7, dadurch gekennzeichnet, daß man die Umsetzung im Temperaturbereich von + 50°C bis + 150°C durchführt.

**9.** Arzneimittel, enthaltend 1,3-disubstituierte Pyrrolidine nach den Ansprüchen 1 - 3.

**10.** Arzneimittel nach Anspruch 9, enthaltend 0,5 bis 90 Gew.-% an 1,3-disubstituierten Pyrrolidinen, bezogen auf die Gesamtmischung.

**11.** Verwendung von 1,3-disubstituierten Pyrrolidinen nach den Ansprüchen 1 - 3 zur Herstellung von Arzneimitteln.

**12.** Verwendung nach Anspruch 12, zur Herstellung von Arzneimitteln zur Behandlung von Erkrankungen des zentralen Nervensystems.

**Patentansprüche für folgenden Vertragsstaat : ES**

**1.** Verfahren zur Herstellung von 1,3-disubstituierten Pyrrolidinen der allgemeinen Formel

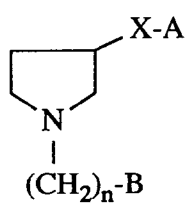

(I)

worin

A für Phenyl oder ein monocyclischer fünf- oder sechsgliedriger Hetarylrest, der ein oder zwei Stickstoff- und/oder Sauerstoff- und/oder Schwefelatome enthält, steht, an die gegebenenfalls ein bis drei aromatische gesättigte oder ungesättigte cyclische Kohlenwasserstoffe mit 5 bis 8 Kohlenstoffatomen oder fünf- oder sechs-gliedrige Heterocyclen mit einem oder zwei Stickstoff- und/oder Sauerstoff- und/oder Schwefelatomen kondensiert sind, wobei dieser Rest gegebenenfalls ein- oder zweifach durch $C_1$-$C_6$-Alkyl, $C_1$-$C_6$-Alkoxy, $C_1$-$C_6$-Alkoxycarbonyl, $C_1$-$C_6$-Alkenyloxy, Acyloxy, Benzoyloxy, Cyano, Phenyl, Benzyl, Fluor, Chlor, Brom, Trifluormethyl, Trifluormethoxy, Difluor- methoxy oder einen Rest der Formel -CO-NYZ, -NH-$SO_2$-Y′, -$SO_2$-NYZ oder -NH-CO-Y substituiert ist, wobei

Y,Z gleich oder verschieden sind und für Wasserstoff oder $C_1$-$C_6$-Alkyl stehen, und

Y′ für $C_1$-$C_6$-Alkyl oder Aryl mit 6 bis 12 Kohlenstoffatomen steht,

und im Fall von Stickstoffheterocyclen gegebenenfalls als N-Oxid vorliegt,

X - für -$CH_2$-O-, O-$CH_2$- oder -O- steht,

B - für Cyano oder eine Gruppe der Formel -$COOR^1$, $CONR^2R^3$, -$SO_2NR^2R^3$, -$SO_mR^4$, $NR^5R^6$ oder -C≡C-$CH_2$-$NR^5R^6$ steht, wobei

$R^1$ - für Wasserstoff, $C_1$-$C_{11}$-Alkyl, $C_5$-$C_8$-Cycloalkyl, $C_2$-$C_{12}$-Alkenyl, $C_6$-$C_{12}$-Aryl oder $C_7$-$C_{14}$-Aralkyl steht,

$R_2$ und $R_3$ gleich oder verschieden sind und

- für Wasserstoff, $C_1$-$C_{12}$-Alkyl, $C_5$-$C_8$-Cycloalkyl, $C_6$-$C_{12}$-Aryl oder $C_7$-$C_{14}$-Aralkyl stehen, wobei die Arylreste durch Halogen, $C_1$-$C_6$-Alkoxy oder $C_1$-$C_6$-Alkoxycarbonyl substituiert sein können,

$R^4$ - für $C_1$-$C_{12}$-Alkyl, $C_5$-$C_8$-Cycloalkyl, $C_6$-$C_{12}$-Aryl oder $C_7$-$C_{14}$-Aralkyl steht, wobei die Arylreste bis zu 3-fach gleich oder verschieden durch Halogen, Cyano, $C_1$-$C_6$-Alkyl, $C_1$-$C_6$-Alkoxy, Trifluormethyl oder Trifluormethoxy substituiert sein können, oder

- für eine Gruppe der Formel

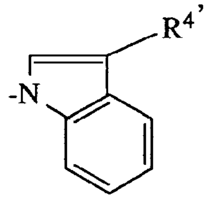

steht,

worin $R^{4'}$ Wasserstoff oder $C_1$-$C_6$-Alkyl bedeutet

m - für eine Zahl 0, 1 oder 2 steht,

$R^5$ und $R^6$ gleich oder verschieden sind und

- für Wasserstoff, $C_1$-$C_{12}$-Alkyl, $C_5$-$C_8$ Cycloalkyl, $C_6$-$C_{12}$-Aryl oder $C_7$-$C_{14}$-Aralkyl stehen, wobei die Arylreste durch Halogen, Cyano, $C_1$-$C_6$-Alkyl, $C_1$-$C_6$-Alkoxy oder Trifluormethyl substituiert sein können, oder

- für eine Gruppe der Formel -$COR^7$ oder -$SO_2R^8$ stehen,

worin

$R^7$ - Wasserstoff oder

- eine Gruppe $NHR^9$ oder

- $C_1$-$C_{11}$-Alkyl, $C_5$-$C_8$-Cycloalkyl oder $C_1$-$C_{12}$-Alkoxy, oder

- $C_6$-$C_{12}$-Aryl, $C_6$-$C_{12}$-Aryloxy, $C_7$-$C_{14}$-Aralkyl, $C_6$-$C_{12}$-Aralkoxy oder einen fünf-oder sechsgliedrigen Heteroaryl-Ring mit ein oder zwei Sauerstoff- und/oder Stickstoff- und/oder Schwefelatomen bedeutet, wobei die genannten Reste bis zu 3-fach gleich oder verschieden durch $C_1$-$C_6$-Alkyl, $C_1$-$C_6$-Alkoxy, $C_1$-$C_6$-Alkylthio, Halogen, Cyano, Trifluormethyl, Trifluormethoxy, Trifluormethylthio, Amino, $C_1$-$C_6$-Alkyl-amino oder $C_1$-$C_6$-Dialkyl-amino substituiert sein können,

$R^8$ - $C_5$-$C_8$-Cycloalkyl, oder

- $C_1$-$C_{12}$-Alkyl, das durch Cyano, Halogen, $C_1$-$C_{12}$-Alkoxy oder $C_1$-$C_{12}$Alkoxycarbonyl substituiert sein kann, oder

- $C_6$-$C_{12}$-Aryl, $C_7$-$C_{14}$-Arylkyl oder einen fünf- oder sechsgliedrigen Heteroaryl-Ring mit ein oder zwei Sauerstoff- und/oder Stickstoffund/oder Schwefelatomen bedeutet, wobei die genannten Reste bis zu 3-fach gleich oder verschieden durch $C_1$-$C_6$-Alkyl, $C_1$-$C_6$-Alkoxy, $C_1$-$C_6$-Alkylthio, Halogen, Cyano, Trifluormethyl, Trifluormethoxy, Trifluormethylthio, Nitro, Amino, $C_1$-$C_6$-Alkyl-amino oder $C_1$-$C_6$-Dialkyl-amino substituiert sein können, oder

- eine Gruppe $NR^2R^3$ bedeutet, wobei

$R^2$ und $R^3$ die oben angegebene Bedeutung haben und

$R^9$ - Wasserstoff, oder

- $C_5$-$C_8$-Cycloalkyl, oder
- gegebenenfalls durch Cyano, Fluor, Chlor oder Brom substituiertes $C_1$-$C_{12}$-Alkyl, oder
- $C_6$-$C_{12}$-Aryl, $C_7$-$C_{14}$-Aralkyl, einen fünf- oder sechsgliedrigen Heteroaryl-Ring mit ein oder zwei Sauerstoffund/oder Stickstoff- und/oder Schwefelatomen bedeutet, wobei die Arylreste bis zu 3-fach gleich oder verschieden durch $C_1$-$C_6$-Alkyl, $C_1$-$C_6$-Alkoxy, $C_1$-$C_6$-Alkylthio, Halogen, Cyano, Trifluormethyl, Trifluormethoxy, Trifluormethylthio, Nitro, Amino, $C_1$-$C_6$-Alkyl-amino oder $C_1$-$C_6$-Dialkylamino substituiert sein können,

oder wobei

$R^5$ und $R^6$ zusammen mit dem Stickstoffatom einen Ring der Reihe

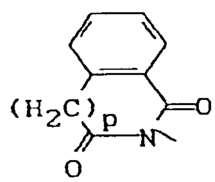 , 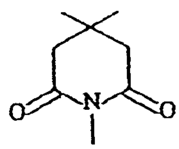 , 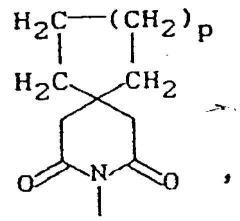 ,

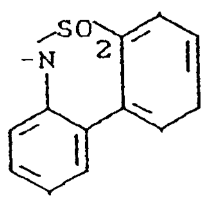 , 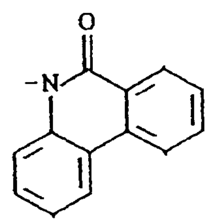 , 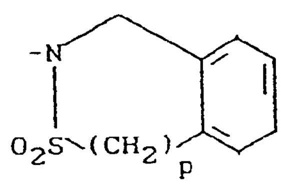 ,

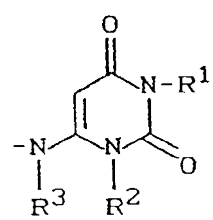 , 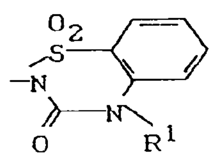 , 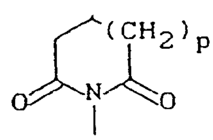 , 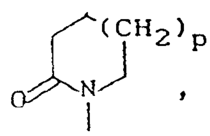 ,

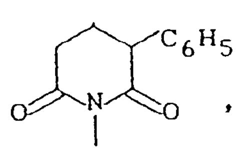 , 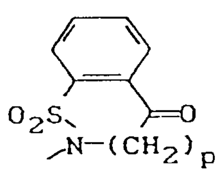 , 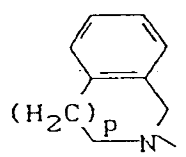 , 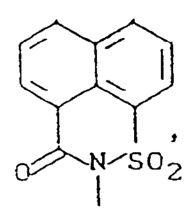 ,

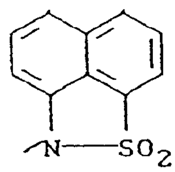 , 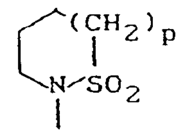 oder 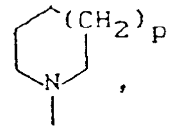 ,

bilden,

worin

p - eine Zahl 0, 1 oder 2 bedeutet,
$R^1$, $R^2$ und $R^3$ die oben angegebene Bedeutung haben,
n - eine Zahl von 1 bis 10 bedeutet
und deren Salze, dadurch gekennzeichnet, daß man
3-substituierte Pyrrolidine der allgemeinen Formel (II)

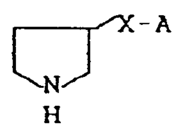

(II)

in welcher A und X die oben angegebene Bedeutung besitzen,
oder ihre Salze
in einer ersten Stufe mit Alkylderivaten der allgemeinen Formel (III)

$$R\text{-}(CH_2)_n\text{-}B' \qquad (III)$$

worin
n - die oben angegebene Bedeutung hat,
B' dem Bedeutungsumfang von B entspricht, wobei jedoch $R^5$ und $R^6$ nicht gleichzeitig für Wasserstoff oder gleichzeitig für Wasserstoff und Alkyl oder Aryl stehen,
R - für Chlor, Brom, Jod, Methylsulfonyloxy, Phenylsulfonyloxy, Tolylsulfonyloxy, Trifluoracetoxy oder Trifluormethylsulfonyloxy steht,
in inerten Lösemitteln, gegebenenfalls in Anwesenheit von Basen, umsetzt,
dann gegebenenfalls funktionelle Gruppen durch Reduktion, Oxidation, Hydrolyse oder Umsetzung mit nucleophilen oder elektrophilen Reagentien in andere funktionelle Gruppen überführt und dann, im Fall der Herstellung der Salze, gegebenenfalls mit der entsprechenden Säure umsetzt.

2. Verfahren nach Anspruch 1 zur Herstellung von Verbindungen der Formel (I), in welcher
A - für Phenyl oder einen monocyclischen fünf- oder sechsgliedrigen Hetarylrest, der ein oder zwei Stickstoff- und/oder Sauerstoff- und/oder Schwefelatome enthält, steht, an die gegebenenfalls ein bis drei aromatische, gesättigte oder ungesättigte cyclische Kohlenwasserstoffe mit 5 bis 8 Kohlenstoffatomen oder fünf- oder sechsgliedrige Heterocyclen mit einem oder zwei Stickstoffund/oder Sauerstoff- und/oder Schwefelstomen kondensiert sind, wobei dieser Rest gegebenenfalls ein- oder zweifach durch $C_1$-$C_6$-Alkyl, $C_1$-$C_6$-Alkoxy, $C_1$-$C_6$-Alkoxycarbonyl, $C_1$-$C_6$-Alkenyloxy, Acyloxy, Benzoyloxy, Cyano, Phenyl, Benzyl, Fluor, Chlor, Brom, Trifluormethyl, Trifluormethoxy, Difluormethoxy oder einen Rest der Formel -CO-NYZ, $-NH-OS_2-Y'$, $-SO_2-NYZ$ oder -NH-CO-Y substituiert ist,
wobei
Y,Z gleich oder verschieden sind und für Wasserstoff, oder $C_1$-$C_6$-Alkyl stehen und
Y' für $C_1$-$C_6$-Alkyl oder Aryl mit 6 bis 12 Kohlenstoffatomen,
und im Fall von Stickstoffheterocyclen gegebenenfalls als N-Oxid vorliegt,
X - für $-CH_2-O-$, $-O-CH_2-$ oder -O- steht,
B - für Cyano oder eine Gruppe der Formel $-COOR^1$, $CONR^2R^3$, $-SO_2NR^2R^3$, $-SO_mR^4$, $NR^5R^6$ oder $-C{\equiv}C-CH_2-NR^5R^6$ steht, wobei
$R^1$ - für Wasserstoff, $C_1$-$C_6$-Alkyl oder Phenyl steht,
$R^2$ und $R^3$ gleich oder verschieden sind und
- für Wasserstoff, $C_1$-$C_6$-Alkyl oder Phenyl stehen, das seinerseits durch Fluor, Chlor, Brom, $C_1$-$C_6$-Alkoxy oder $C_1$-$C_6$-Alkoxycarbonyl substituiert sein kann,
$R^4$ - für $C_1$-$C_6$-Alkyl, oder
- für Phenyl steht, das bis zu zweifach gleich oder verschieden durch Fluor, Chlor, Brom, $C_1$-$C_6$-Alkyl oder $C_1$-$C_6$-Alkoxy substituiert sein kann, oder
- für eine Gruppe der Formel

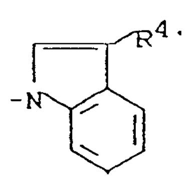

steht, worin $R^{4'}$ Wasserstoff oder $C_1$-$C_6$-Alkyl bedeutet,

m - für eine Zahl 0, 1 oder 2 steht,

$R^5$ und $R^6$ gleich oder verschieden sind und

- für Wasserstoff, $C_1$-$C_6$-Alkyl, Phenyl oder Benzyl stehen, wobei die Phenylreste durch Fluor, Chlor, Brom, $C_1$-$C_6$-Alkyl, $C_1$-$C_6$-Alkoxy oder Trifluormethyl substituiert sein können, oder
- für eine Gruppe der Formel $-COR^7$ oder $-SO_2R^8$ stehen,

worin

$R^7$ - Wasserstoff bedeutet, oder

- eine Gruppe $NHR^9$ bedeutet, oder
- $C_1$-$C_6$-Alkyl oder $C_1$-$C_6$-Alkoxy bedeutet, oder
- gegebenenfalls durch $C_1$-$C_6$-Alkyl, $C_1$-$C_6$-Alkoxy, Fluor, Chlor, Brom, Trifluormethyl, Dimethylamino oder Diethylamino substituiertes Phenyl, Benzyl, Benzyloxy, Thienyl, furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,

$R^8$ - Cyclopropyl, Cyclopentyl, Cyclohexyl, oder

- gegebenenfalls durch Cyano, Fluor, Chlor, Brom, Trifluormethyl oder $C_1$-$C_6$-Alkoxycarbonyl substituiertes $C_1$-$C_6$-Alkyl bedeutet, oder
- gegebenenfalls durch $C_1$-$C_6$-Alkyl, $C_1$-$C_6$-Alkoxy, Fluor, Chlor, Brom, Nitro, Trifluormethyl, Dimethylamino oder Diethylamino substituiertes Phenyl, Naphthyl, Benzyl, Thienyl, Furyl, Pyrimidyl, Pyridyl, Chinolyl, Isochinolyl, Benzothiazolyl, Benzoxazolyl, Thiazolyl, Oxazolyl, Isoxazolyl oder Isothiazolyl bedeutet, oder
- eine Gruppe $NR^2R^3$ bedeutet,

wobei

$R^2$ und $R^3$ die oben angegebene Bedeutung haben, und

$R^9$ - gegebenenfalls durch Cyano, Fluor, Chlor oder Brom substituiertes $C_1$-$C_6$-Alkyl bedeutet, oder

- gegebenenfalls durch $C_1$-$C_6$-Alkyl, $C_1$-$C_6$-Alkoxy, Fluor, Chlor, Brom, Trifluormethyl, Dimethylamino oder Diethylamino substituiertes Phenyl, Benzyl, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,

oder

$R^5$ und $R^6$ zusammen mit dem Stickstoffatom einen Ring der Reihe

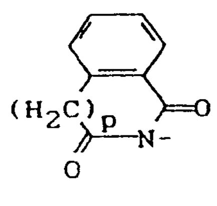,
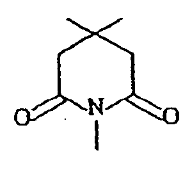,
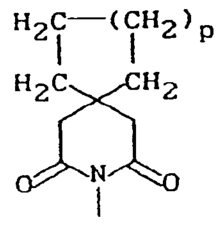,

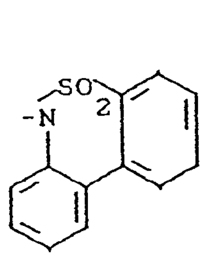,
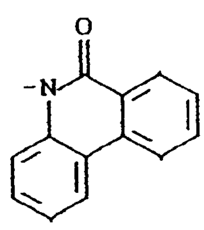,
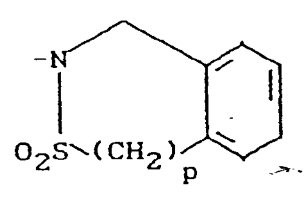,

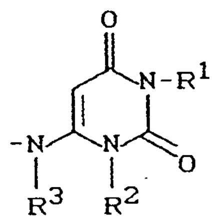,
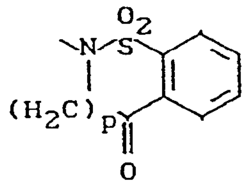,
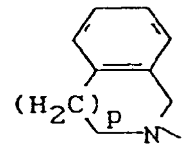,
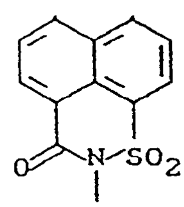

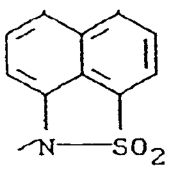,
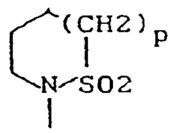,
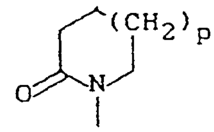,

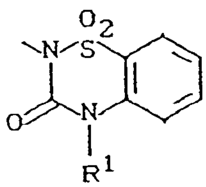
oder
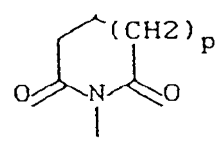

bilden, worin
P - eine Zahl 0, 1 oder 2 bedeutet,
$R^1$, $R^2$ und $R^3$ die oben angegebene Bedeutung haben und
n - eine Zahl 1 bis 8 bedeutet,
und deren Salze.

**3.** Verfahren nach Anspruch 1 zur Herstellung von Verbindungen der Formel (I), in welcher
A - für gegebenenfalls gleich oder verschieden durch $C_1$-$C_6$-Alkyl, $C_1$-$C_6$-Alkoxy, $C_1$-$C_6$-Alkoxycarbonyl, $C_1$-$C_6$-Alkenyloxy, Acyloxy, Benzoyloxy, Cyano, Phenyl, Benzyl, Sulfonylamino, Sulfamoyl ($C_1$ bis $C_6$), Carbamoyl, Carbonylamino, Fluor, Chlor, Brom, Trifluormethyl, Trifluormethoxy, Difluormethoxy, substituiertes Phenyl oder Naphthyl, Furan, Thiophen, Isoxazol, Pyridin, Pyrimidin, Indol, Indazol, Benzofuran, Benzisoxazol, Chinolin, Isochinolin, Tetralin, Inden, Chroman, Dihydrobenzodioxin, Dihydroindol, Tetrahydrochinolin oder Dihydrobenzofuran steht,
X - für $-O-CH_2-$, $-CH_2-O-$ oder $-O-$ steht,
B - für Cyano, oder
- eine Gruppe der Formel $-CONR^2R^3$, $-NR^5R^6$, $-SO_mR^4$, $-C{\equiv}C-CH_2-NR^5R^6$, $-SO_2NR^2R^3$, $COOR^1$ steht, wobei
$R^1$ - Wasserstoff, Methyl, Ethyl oder Phenyl bedeutet,
$R^2$ und $R^3$ gleich oder verschieden sind und
- Wasserstoff, Methyl, Ethyl, Propyl, Isopropyl, Butyl, Isobutyl bedeuten oder Phenyl bedeuten, das durch Methoxycarbonyl substituiert sein kann
$R^4$ - für Methyl oder Ethyl steht oder
- für eine Gruppe der Formel

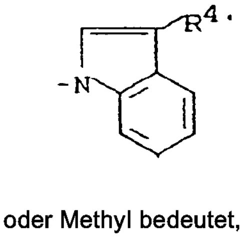

steht, worin $R^{4'}$ Wasserstoff oder Methyl bedeutet,
$R^5$ und $R^6$ gleich oder verschieden sind, und
- Wasserstoff, Methyl, Ethyl, Propyl, Isopropyl, Butyl, Isobutyl, oder

- gegebenenfalls durch Fluor, Chlor, Methyl oder Methoxy substituiertes Phenyl, oder
- eine Gruppe $-COR^7$ oder $-SO_2R^8$ bedeuten, worin

$R^7$

- für eine Gruppe $NHR^9$ steht, oder
- für Methyl, Ethyl, Propyl, Isopropyl, Methoxy, Ethoxy, Propoxy, Isopropoxy, tert.Butoxy oder
- für gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl steht,

$R^8$

- für gegebenenfalls durch Fluor, Chlor, Methoxycarbonyl, Ethoxycarbonyl, Propoxycarbonyl, Isopropoxycarbonyl, Butoxycarbonyl, Isobutoxycarbonyl oder tert.-Butoxycarbonyl, substituiertes Methyl, Ethyl, Propyl, Isopropyl, Butyl oder Isobutyl steht, oder
- für gegebenenfalls durch Methyl, Ethyl, Propyl, Isopropyl, Methoxy, Fluor, Chlor, Nitro substituiertes Phenyl, Naphthyl, Thienyl, Furyl, Pyridyl, Pyrimidyl, Chinolyl oder Isochinolyl steht, oder
- für eine Gruppe $NR^2R^3$ steht, wobei

$R^2$ und $R^3$ die oben angegebene Bedeutung haben,

$R^9$

- gegebenenfalls durch Fluor oder Chlor substituiertes Methyl, Ethyl, Propyl, Isopropyl, Butyl, Isobutyl, Pentyl, Isopentyl, Hexyl oder Isohexyl bedeutet, oder
- Phenyl bedeutet, das durch Fluor, Chlor, Methyl oder Methoxy substituiert sein kann, oder

$R^5$ und $R^6$ gemeinsam mit dem Stickstoffatom einen Ring der Reihe

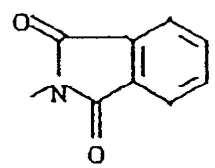 , 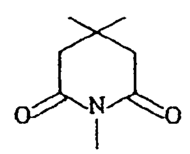 , 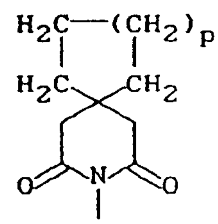 ,

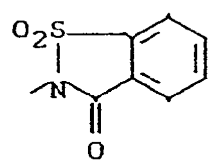 , 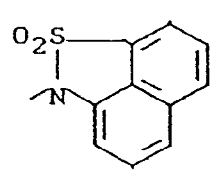 , 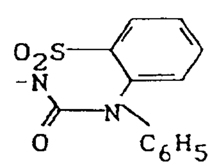 ,

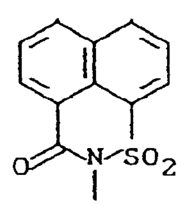 , 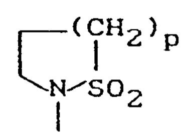 , 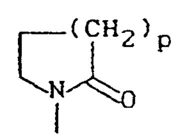 ,

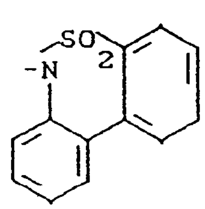 , 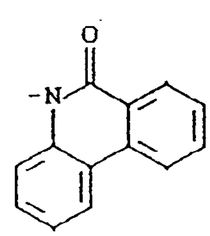 , 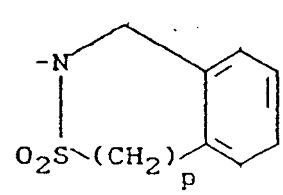 ,

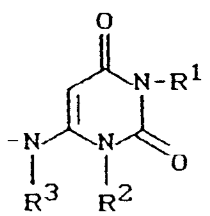

oder

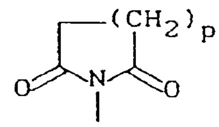

bilden, worin
p - eine Zahl 1 oder 2 bedeutet
$R^1$, $R^2$ und $R^3$ die oben angegebene Bedeutung haben und
n - eine Zahl 1 bis 6 bedeutet
und deren Salze.

4. Verfahren nach Anspruch 1, dadurch gekennzeichnet, daß man in einem Temperaturbereich von -20°C bis +150°C arbeitet.

5. Verfahren zur Herstellung von 1,3-disubstituierten Pyrrolidinen der allgemeinen Formel

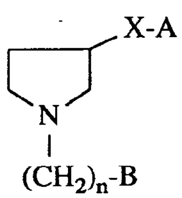

(I)

worin
A für Phenyl oder ein monocyclischer fünf- oder sechsgliedriger Hetarylrest, der ein oder zwei Stickstoff- und/oder Sauerstoff- und/oder Schwefelatome enthält, steht, an die gegebenenfalls ein bis drei aromatische gesättigte oder ungesättigte cyclische Kohlenwasserstoffe mit 5 bis 8 Kohlenstoffatomen oder fünf- oder sechs-gliedrige Heterocyclen mit einem oder zwei Stickstoff- und/oder Sauerstoff- und/oder Schwefelatomen kondensiert sind, wobei dieser Rest gegebenenfalls ein- oder zweifach durch $C_1$-$C_6$-Alkyl, $C_1$-$C_6$-Alkoxy, $C_1$-$C_6$-Alkoxycarbonyl, $C_1$-$C_6$-Alkenyloxy, Acyloxy, Benzoyloxy, Cyano, Phenyl, Benzyl, Fluor, Chlor, Brom, Trifluormethyl, Trifluormethoxy, Difluormethoxy oder einen Rest der Formel -CO-NYZ, -NH-$SO_2$-Y', -$SO_2$-NYZ oder -NH-CO-Y substituiert ist, wobei
Y,Z gleich oder verschieden sind und für Wasserstoff oder $C_1$-$C_6$-Alkyl stehen, und
Y' für $C_1$-$C_6$-Alkyl oder Aryl mit 6 bis 12 Kohlenstoffatomen steht,
und im Fall von Stickstoffheterocyclen gegebenenfalls als N-Oxid vorliegt,
X - für -$CH_2$-O-, O-$CH_2$- oder -O- steht,
und
B - für Cyano oder eine Gruppe der Formel -$SO_2NR^2R^3$ oder -$SO_mR^4$ bedeutet, wobei
$R^2$, $R^3$, $R^4$ und m die in Anspruch 1 angegebene Bedeutung hat
dadurch gekennzeichnet, daß man 3-substituierte Pyrrolidine der allgemeinen Formel (II)

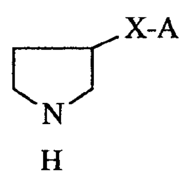

(II)

worin
A und X die obengenannte Bedeutung haben mit Acrylnitril oder einer Verbindung der Formel $CH_2$=CH-$SO_2NR^2R^3$ oder -$CH_2$=$CH_2$-$SO_mR^4$ in Gegenwart von Katalysatoren umsetzt.

6. Verfahren nach Anspruch 5, dadurch gekennzeichnet, daß man die Umsetzung in einem Temperaturbereich von +50°C bis + 150°C durchführt.

## Claims

**Claims for the following Contracting States : AT, BE, CH, DE, FR, GB, IT, LI, NL, SE**

**1.** 1,3-Disubstituted pyrrolidines of the general formula

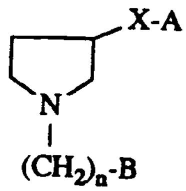

(I)

wherein

A represents phenyl or a monocyclic five- or six-membered hetaryl radical which contains one or two nitrogen and/or oxygen and/or sulphur atoms, onto which are optionally fused one to three aromatic saturated or unsaturated cyclic hydrocarbons having 5 to 8 carbon atoms or five- or six-membered heterocycles having one or two nitrogen and/or oxygen and/or sulphur atoms, where this radical is optionally monosubstituted or disubstituted by $C_1$-$C_6$-alkyl, $C_1$-$C_6$-alkoxy, $C_1$-$C_6$-alkoxycarbonyl, $C_1$-$C_6$-alkenyloxy, acyloxy, benzoyloxy, cyano, phenyl, benzyl, fluorine, chlorine, bromine, trifluoromethyl, trifluoromethoxy, difluoromethoxy or a radical of the formula -CO-NYZ, $-NH-SO_2-Y'$, $-SO_2-NYZ$ or -NH-CO-Y, where

Y and Z are identical or different and represent hydrogen or $C_1$-$C_6$-alkyl, and

Y' represents $C_1$-$C_6$-alkyl or aryl having 6 to 12 carbon atoms,

and in the case of nitrogen heterocycles is optionally present as the N-oxide,

X - represents $-CH_2-O-$, $-O-CH_2-$ or -O-,

B - represents cyano or a group of the formula $-COOR^1$, $CONR^2R^3$, $-SO_2NR^2R^3$, - $SO_mR^4$, $NR^5R^6$ or $-C\equiv C-CH_2-NR^5R^6$, where

$R^1$ - represents hydrogen, $C_1$-$C_{11}$-alkyl, $C_5$-$C_8$-cycloalkyl, $C_2$-$C_{12}$-alkenyl, $C_6$-$C_{12}$-aryl or $C_7$-$C_{14}$-aralkyl,

$R^2$ and $R^3$ are identical or different and

- represent hydrogen, $C_1$-$C_{12}$-alkyl, $C_5$-$C_8$-cycloalkyl, $C_6$-$C_{12}$-aryl or $C_7$-$C_{14}$-aralkyl, where the aryl radicals can be substituted by halogen, $C_1$-$C_6$-alkoxy or $C_1$-$C_6$-alkoxycarbonyl,

$R^4$ - represents $C_1$-$C_{12}$-alkyl, $C_5$-$C_8$-cycloalkyl, $C_6$-$C_{12}$-aryl or $C_7$-$C_{14}$-aralkyl, where the aryl radicals can be monosubstituted, disubstituted or tri, substituted by identical or different substituents from the series comprising halogen, cyano, $C_1$-$C_6$-alkyl, $C_1$-$C_6$-alkoxy, trifluoromethyl and trifluoromethoxy, or

- represents a group of the formula

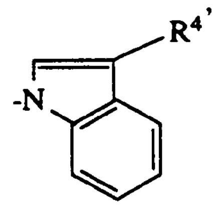

wherein $R^{4'}$ denotes hydrogen or $C_1$-$C_6$-alkyl

m - represents a number 0, 1 or 2,

$R^5$ and $R^6$ are identical or different and

- represent hydrogen, $C_1$-$C_{12}$-alkyl, $C_5$-$C_8$-cycloalkyl, $C_6$-$C_{12}$-aryl or $C_7$-$C_{14}$-aralkyl, where the aryl radicals can be substituted by halogen, cyano, $C_1$-$C_6$-alkyl, $C_1$-$C_6$-alkoxy or trifluoromethyl, or

- represent a group of the formula $-COR^7$ or $-SO_2R^8$, wherein

$R^7$ - denotes hydrogen or

- a group $NHR^9$ or

- $C_1$-$C_{11}$-alkyl, $C_5$-$C_8$-cycloalkyl or $C_1$-$C_{12}$-alkoxy, or

- $C_6$-$C_{12}$-aryl, $C_6$-$C_{12}$-aryloxy, $C_7$-$C_{14}$-aralkyl, $C_6$-$C_{12}$-aralkoxy or a five- or six-membered heteroaryl ring having one or two oxygen and/ or nitrogen and/or sulphur atoms, where the radicals mentioned can be monosubstituted, disubstituted or trisubstituted by identical or different substituents from the series comprising $C_1$-$C_6$-alkyl, $C_1$-$C_6$-alkoxy, $C_1$-$C_6$-alkylthio, halogen, cyano, trifluoromethyl, trifluoromethoxy, trifluoromethylthio, amino, $C_1$-$C_6$-alkyl-amino and $C_1$-$C_6$-dialkyl-amino

$R^8$ - denotes $C_5$-$C_8$-cycloalkyl, or

- $C_1$-$C_{12}$-alkyl which can be substituted by cyano, halogen, $C_1$-$C_{12}$-alkoxy or $C_1$-$C_{12}$-alkoxycarbonyl, or

- $C_6$-$C_{12}$-aryl, $C_7$-$C_{14}$-aralkyl or a five- or six-membered heteroaryl ring having one or two oxygen and/or nitrogen and/or sulphur atoms, where the radicals mentioned can be monosubstituted, disubstituted or trisubstituted by identical or different substituents from the series comprising $C_1$-$C_6$-alkyl, $C_1$-$C_6$-alkoxy, $C_1$-$C_6$-alkylthio, halogen, cyano, trifluoromethyl, trifluoromethoxy, trifluoromethylthio, nitro, amino, $C_1$-$C_6$-alkyl-amino and $C_1$-$C_6$-dialkyl-amino or

- denotes a group $NR^2R^3$, where

$R^2$ and $R^3$ have the abovementioned meaning and

$R^9$ - denotes hydrogen, or

- $C_5$-$C_8$-cycloalkyl, or

- $C_1$-$C_{12}$-alkyl optionally substituted by cyano, fluorine, chlorine or bromine, or

- $C_6$-$C_{12}$-aryl, $C_7$-$C_{14}$-aralkyl or a five- or six-membered heteroaryl ring having one or two oxygen and/or nitrogen and/or sulphur atoms, where the aryl radicals can be monosubstituted, disubstituted or trisubstituted by identical or different substituents from the series comprising $C_1$-$C_6$-alkyl, $C_1$-$C_6$-alkoxy, $C_1$-$C_6$-alkylthio, halogen, cyano, trifluoromethyl, trifluoromethoxy, trifluoromethylthio, nitro, amino, $C_1$-$C_6$-alkyl-amino and $C_1$-$C_6$-dialkylamino,

or where

$R^5$ and $R^6$, together with the nitrogen atom, form a ring from the series comprising

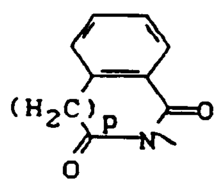 , 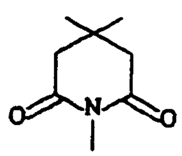 , 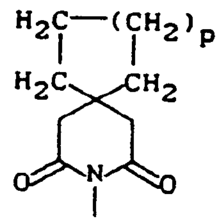 ,

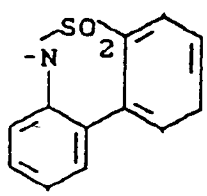 , 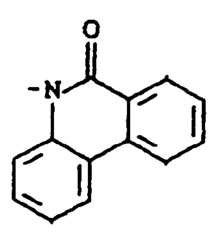 , 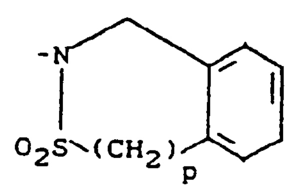 ,

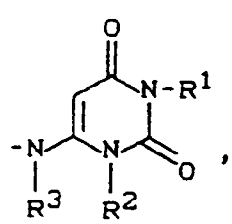 , 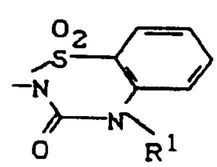 , 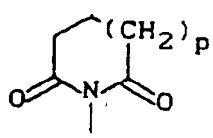 , 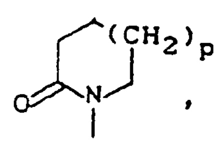 ,

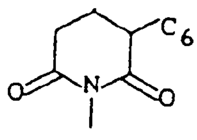 , 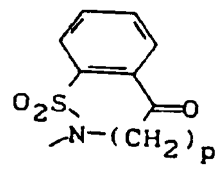 , 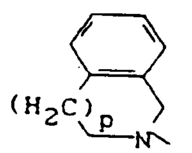 , 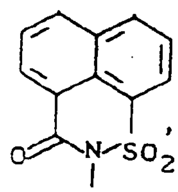 ,

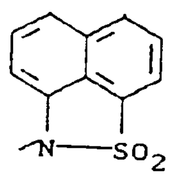 , 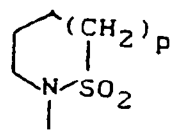 or 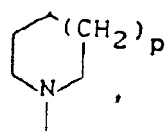 ,

wherein
p - denotes a number 0, 1 or 2,
$R^1$, $R^2$ and $R^3$ have the abovementioned meaning,
n - denotes a number from 1 to 10
and their salts.

**2.** 1,3-Disubstituted pyrrolidines according to Claim 1, wherein
A - represents phenyl or a monocyclic five- or six-membered hetaryl radical which contains one or two nitrogen and/or oxygen and/or sulphur atoms, onto which one to three aromatic, saturated or unsaturated cyclic hydrocarbons having 5 to 8 carbon atoms or five- or six-membered heterocycles having one or two nitrogen and/or oxygen and/or sulphur atoms are optionally fused, where this radical is optionally monosubstituted or disubstituted by $C_1$-$C_6$-alkyl, $C_1$-$C_6$-alkoxy, $C_1$-$C_6$-alkoxycarbonyl, $C_1$-$C_6$-alkenloxy, acyloxy, benzoyloxy, cyano, phenyl, benzyl, fluorine, chlorine, bromine, trifluoromethyl, trifluoromethoxy, difluoromethoxy or a radical of the formula -CO-NYZ, -NH-$SO_2$-Y′, -$SO_2$-NYZ or NH-CO-Y, where
Y and Z are identical or different and represent hydrogen or $C_1$-$C_6$-alkyl and
Y′ represents $C_1$-$C_6$-alkyl or aryl having 6 to 12 carbon atoms,
and in the case of nitrogen heterocycles is optionally present as the N-oxide,
X - represents -$CH_2$-O-, -O-$CH_2$-, or -O-,
B - represents cyano or a group of the formula -$COOR^1$, $CONR^2R^3$, -$SO_2NR^2R^3$, -$SO_mR^4$, $NR^5R^6$ or -C≡C-$CH_2$-$NR^5R^6$, where
$R^1$ - represents hydrogen, $C_1$-$C_6$-alkyl or phenyl,
$R^2$ and $R^3$ are identical or different and
- represent hydrogen, $C_1$-$C_6$-alkyl or phenyl which, in turn, can be substituted by fluorine, chlorine, bromine, $C_1$-$C_6$-alkoxy or $C_1$-$C_6$-alkoxycarbonyl,
$R^4$ - represents $C_1$-$C_6$-alkyl, or
- represents phenyl which can be monosubstituted or disubstituted by identical or different substituents from the series comprising fluorine, chlorine, bromine, $C_1$-$C_6$-alkyl and $C_1$-$C_6$-alkoxy, or
- represents a group of the formula

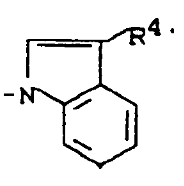

wherein $R^{4'}$ denotes hydrogen or $C_1$-$C_6$-alkyl,
m - represents a number 0, 1 or 2,
$R^5$ and $R^6$ are identical or different and
- represent hydrogen, $C_1$-$C_6$-alkyl, phenyl or benzyl, where the phenyl radicals can be substituted by fluorine, chlorine, bromine, $C_1$-$C_6$-alkyl, $C_1$-$C_6$-alkoxy or trifluoromethyl, or
- represents a group of the formula -$COR^7$ or -$SO_2R^8$,
wherein

$R^7$ - denotes hydrogen, or

- denotes a group $NHR^9$, or
- denotes $C_1$-$C_6$-alkyl or $C_1$-$C_6$-alkoxy, or
- phenyl, benzyl, benzyloxy, thienyl, furyl, pyridyl, pyrimidyl, quinolyl, isoquinolyl, benzothiazolyl, benzoxazolyl, thiazolyl, oxazolyl, isoxazolyl or isothiazolyl optionally substituted by $C_1$-$C_6$-alkyl, $C_1$-$C_6$-alkoxy, fluorine, chlorine, bromine, trifluoromethyl, dimethylamino or diethylamino,

$R^8$ - denotes cyclopropyl,cyclopentyl, cyclohexyl, or

- $C_1$-$C_6$-alkyl which is optionally substituted by cyano, fluorine, chlorine, bromine, trifluoromethyl or $C_1$-$C_6$-alkoxycarbonyl, or
- denotes phenyl, naphthyl, benzyl, thienyl, furyl, pyrimidyl, pyridyl, quinolyl, isoquinolyl, benzothiazolyl, benzoxazolyl, thiazolyl, oxazolyl, isoxazolyl or isothiazolyl optionally substituted by $C_1$-$C_6$-alkyl, $C_1$-$C_6$-alkoxy, fluorine, chlorine, bromine, nitro,trifluoromethyl, dimethylamino or diethylamino, or
- denotes a group $NR^2R^3$, where

  $R^2$ and $R^3$ have the abovementioned meaning, and

$R^9$ - denotes $C_1$-$C_6$-alkyl which is optionally substituted by cyano, fluorine, chlorine or bromine, or

- denotes phenyl, benzyl, thienyl, furyl, pyridyl, pyrimidyl, quinolyl, isoquinolyl, benzothiazolyl, benzoxazolyl, thiazolyl, oxazolyl, isoxazolyl or isothiazolyl optionally substituted by $C_1$-$C_6$-alkyl, $C_1$-$C_6$-alkoxy, fluorine, chlorine, bromine, trifluoromethyl, dimethylamino or diethylamino,

or

$R^5$ and $R^6$, together with the nitrogen atom, form a ring from the series comprising

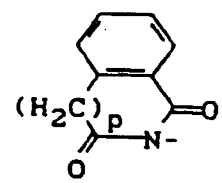 , 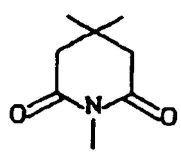 , 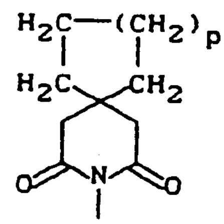 ,

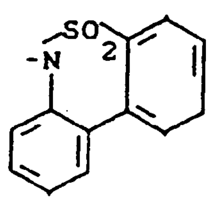 , 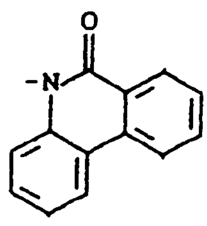 , 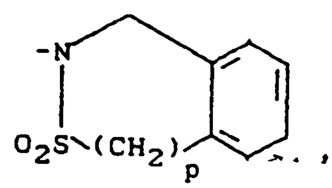 ,

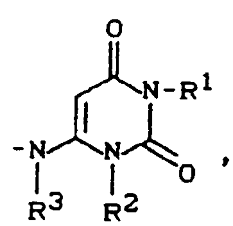 , 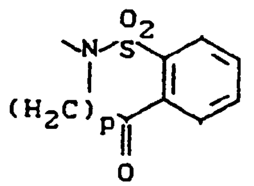 , 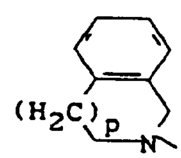 , 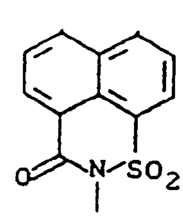

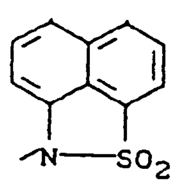 , 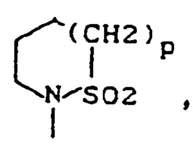 , 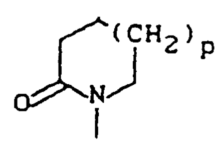 ,

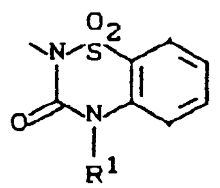

or

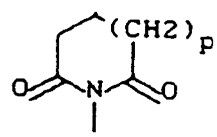

wherein

p - denotes a number 0, 1 or 2,

$R^1$, $R^2$ and $R^3$ have the abovementioned meaning, and

n - denotes a number from 1 to 8,

and their salts.

**3.** 1,3-Disubstituted pyrrolidines according to Claim 1, where

A - represents phenyl or naphthyl optionally substituted by identical or different substituents from the series comprising $C_1$-$C_6$-alkyl, $C_1$-$C_6$-alkoxy, $C_1$-$C_6$-alkoxy-carbonyl, $C_1$-$C_6$-alkenyloxy, acyloxy, benzoyloxy, cyano, phenyl, benzyl, sulphonylamino, sulphamoyl ($C_1$ to $C_6$), carbamoyl, carbonylamino, fluorine, chlorine, bromine, trifluoromethyl, trifluoromethoxy and difluoromethoxy, furan, thiophene, isoxazole, pyridine, pyrimidine, indole, indazole, benzofuran, benzisoxazole, quinoline, isoquinoline, tetralin, indene, chroman, dihydrobenzodioxin, dihydroindole, tetrahydroquinoline or dihydrobenzofuran,

X - represents $-O-CH_2-$, $-CH_2-O-$ or -O-,

B - represents cyano, or

- a group of the formula $-CONR^2R^3$, $-NR^5R^6$, $-SO_mR^4$, $-C{\equiv}C-CH_2-NR^5R^6$, $-SO_2NR^2R^3$ or $COOR^1$,

where

$R^1$ - denotes hydrogen, methyl, ethyl or phenyl,

$R^2$ and $R^3$ are identical or different and

- denote hydrogen, methyl, ethyl, propyl, isopropyl, butyl or isobutyl or denote phenyl which can be substituted by methoxycarbonyl

$R^4$ - represents methyl or ethyl or

- represents a group of the formula

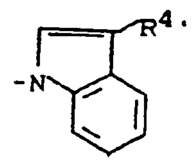

wherein $R^{4'}$, denotes hydrogen or methyl,

$R^5$ and $R^6$ are identical or different, and

- denote hydrogen, methyl, ethyl, propyl, isopropyl, butyl or isobutyl, or
- phenyl optionally substituted by fluorine, chlorine, methyl or methoxy, or
- denote a group $-COR^7$ or $-SO_2R^8$, wherein

$R^7$ - represents a group $NHR^9$, or

- represents methyl, ethyl, propyl, isopropyl, methoxy, ethoxy, propoxy, isopropoxy, tert.butoxy or
- phenyl, benzyl, benzyloxy, thienyl, furyl, pyridyl, pyrimidyl, quinolyl or isoquinolyl optionally substituted by methyl, methoxy, fluorine or chlorine,

$R^8$ - represents methyl, ethyl, propyl, isopropyl, butyl or isobutyl optionally substituted by fluorine, chlorine, methoxycarbonyl, ethoxycarbonyl, propoxycarbonyl, isopropoxycarbonyl, butoxycarbonyl, isobutoxycarbonyl or tert.-butoxycarbonyl, or

- represents phenyl, naphthyl, thienyl, furyl, pyridyl, pyrimidyl, quinolyl or isoquinolyl optionally substituted by methyl, ethyl, propyl, isopropyl, methoxy, fluorine, chlorine or nitro, or
- represents a group $NR^2R^3$, where

$R^2$ and $R^3$ have the abovementioned meaning,

$R^9$ - denotes methyl, ethyl, propyl, isopropyl, butyl, isobutyl, pentyl, isopentyl, hexyl or isohexyl optionally substituted by fluorine or chlorine, or

- denotes phenyl which can be substituted by fluorine, chlorine, methyl or methoxy, or

$R^5$ and $R^6$, together with the nitrogen atom, form a ring from the series comprising

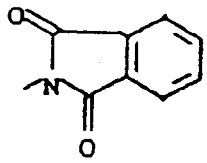, 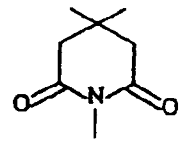, 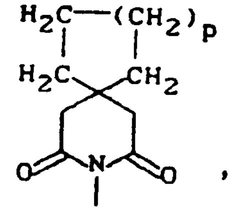,

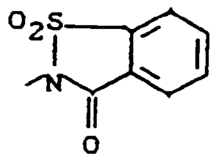, 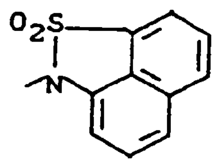, 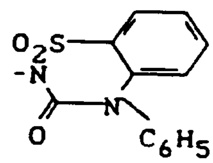,

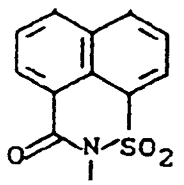, 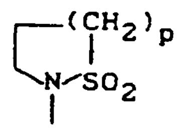, 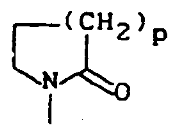,

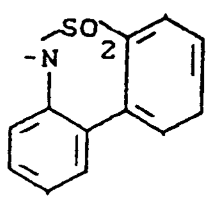, 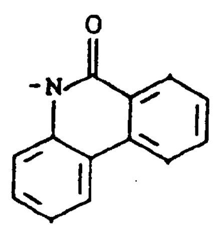, 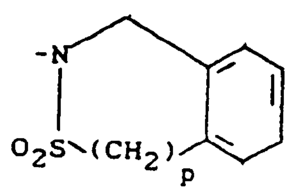,

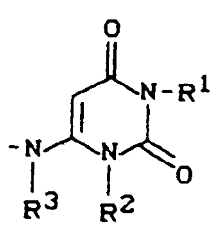 or 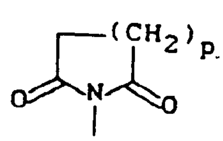

p - denotes a number 1 or 2

$R^1$, $R^2$ and $R^3$ have the abovementioned meaning and

n - denotes a number from 1 to 6,

and their salts.

**4.** 1,3-Disubstituted pyrrolidines according to Claims 1 to 3, for therapeutic treatment.

**5.** Process for the preparation of 1,3-disubstituted pyrrolidines of the general formula

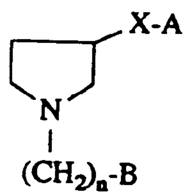

(I)

wherein

A represents phenyl or a monocyclic five- or six-membered hetaryl radical which contains one or two nitrogen and/or oxygen and/or sulphur atoms, onto which are optionally fused one to three aromatic saturated or unsaturated cyclic hydrocarbons having 5 to 8 carbon atoms or five- or six-membered heterocycles having one or two nitrogen and/or oxygen and/or sulphur atoms, where this radical is optionally monosubstituted or disubstituted by $C_1$-$C_6$-alkyl, $C_1$-$C_6$-alkoxy, $C_1$-$C_6$-alkoxycarbonyl, $C_1$-$C_6$-alkenyloxy, acyloxy, benzoyloxy, cyano, phenyl, benzyl, fluorine, chlorine, bromine, trifluoromethyl, trifluoromethoxy, difluoromethoxy or a radical of the formula -CO-NYZ, -NH-$SO_2$-Y', -$SO_2$-NYZ or -NH-CO-Y, where

Y and Z are identical - or different and represent hydrogen or $C_1$-$C_6$-alkyl, and

Y' represents $C_1$-$C_6$-alkyl or aryl having 6 to 12 carbon atoms,

and in the case of nitrogen heterocycles is optionally present as the N-oxide,

X - represents -$CH_2$-O-, O-$CH_2$- or -O-,

B - represents cyano or a group of the formula -$COOR^1$, $CONR^2R^3$, -$SO_2NR^2R^3$, -$SO_mR^4$, $NR^5R^6$ or -C≡C-$CH_2$-$NR^5R^6$, where

$R^1$ - represents hydrogen, $C_1$-$C_{11}$-alkyl, $C_5$-$C_8$-cycloalkyl, $C_2$-$C_{12}$-alkenyl, $C_6$-$C_{12}$-aryl or $C_7$-$C_{14}$-aralkyl,

$R^2$ and $R^3$ are identical or different and

- represent hydrogen, $C_1$-$C_{12}$-alkyl, $C_5$-$C_8$-cycloalkyl, $C_6$-$C_{12}$-aryl or $C_7$-$C_{14}$-aralkyl, where the aryl radicals can be substituted by halogen, $C_1$-$C_6$-alkoxy or $C_1$-$C_6$-alkoxycarbonyl,

$R^4$ - represents $C_1$-$C_{12}$-alkyl, $C_5$-$C_8$-cycloalkyl, $C_6$-$C_{12}$-aryl or $C_7$-$C_{14}$-aralkyl, where the aryl radicals can be monosubstituted, disubstituted or trisubstituted by identical or different substituents from the series comprising halogen, cyano, $C_1$-$C_6$-alkyl, $C_1$-$C_6$-alkoxy, trifluoromethyl and trifluoromethoxy, or

- represents a group of the formula

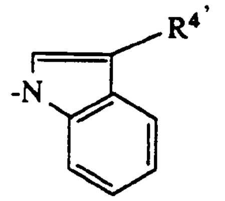

wherein $R^{4'}$ denotes hydrogen or $C_1$-$C_6$-alkyl

m - represents a number 0, 1 or 2,

$R^5$ and $R^6$ are identical or different and

- represent hydrogen, $C_1$-$C_{12}$-alkyl, $C_5$-$C_8$-cycloalkyl, $C_6$-$C_{12}$-aryl or $C_7$-$C_{14}$-aralkyl, where the aryl radicals can be substituted by halogen, cyano, $C_1$-$C_6$-alkyl, $C_1$-$C_6$-alkoxy or trifluoromethyl, or

- represent a group of the formula -$COR^7$ or -$SO_2R^8$, wherein

$R^7$ - denotes hydrogen or

- a group $NHR^9$ or

- $C_1$-$C_{11}$-alkyl, $C_5$-$C_8$-cycloalkyl or $C_1$-$C_{12}$-alkoxy, or

- $C_6$-$C_{12}$-aryl, $C_6$-$C_{12}$-aryloxy, $C_7$-$C_{14}$-aralkyl, $C_6$-$C_{12}$-aralkoxy or a five- or six-membered heteroaryl ring having one or two oxygen and/or nitrogen and/or sulphur atoms, where the radicals mentioned can be monosubstituted, disubstituted or trisubstituted by identical or different substituents from the series comprising $C_1$-$C_6$-alkyl, $C_1$-$C_6$-alkoxy, $C_1$-$C_6$-alkylthio, halogen, cyano, trifluoromethyl, trifluoromethoxy, trifluoromethylthio, amino, $C_1$-$C_6$-alkyl-amino and $C_1$-$C_6$-dialkyl-amino

$R^8$ - denotes $C_5$-$C_8$-cycloalkyl, or

- $C_1$-$C_{12}$-alkyl which can be substituted by cyano, halogen, $C_1$-$C_{12}$-alkoxy or $C_1$-$C_{12}$-alkoxycarbonyl, or

- $C_6$-$C_{12}$-aryl, $C_7$-$C_{14}$-aralkyl or a five- or six-membered heteroaryl ring having one or two

oxygen and/or nitrogen and/or sulphur atoms, where the radicals mentioned can be monosubstituted, disubstituted or trisubstituted by identical or different substituents from the series comprising $C_1$-$C_6$-alkyl, $C_1$-$C_6$-alkoxy, $C_1$-$C_6$-alkylthio, halogen, cyano, trifluoromethyl, trifluoromethoxy, trifluoromethylthio, nitro, amino, $C_1$-$C_6$-alkyl-amino and $C_1$-$C_6$-dialkyl-amino or

- denotes a group $NR^2R^3$, where

$R^2$ and $R^3$ have the above-mentioned meaning and

$R^9$ - denotes hydrogen, or

- $C_5$-$C_8$-cycloalkyl, or
- $C_1$-$C_{12}$-alkyl optionally substituted by cyano, fluorine, chlorine or bromine, or
- $C_6$-$C_{12}$-aryl, $C_7$-$C_{14}$-aralkyl or a five- or six-membered heteroaryl ring having one or two oxygen and/or nitrogen and/or sulphur atoms, where the aryl radicals can be monosubstituted, disubstituted or trisubstituted by identical or different substituents from the series comprising $C_1$-$C_6$-alkyl, $C_1$-$C_6$-alkoxy, $C_1$-$C_6$-alkylthio, halogen, cyano, trifluoromethyl, trifluoromethoxy, trifluoromethylthio, nitro, amino, $C_1$-$C_6$-alkyl-amino and $C_1$-$C_6$-dialkylamino,

or where

$R^5$ and $R^6$, together with the nitrogen atom, form a ring from the series comprising

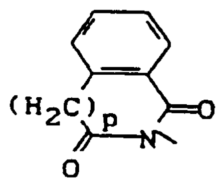 , 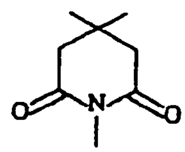 , 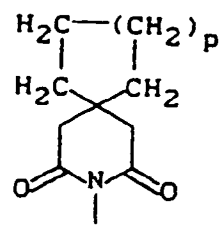 ,

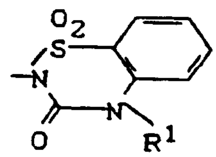 , 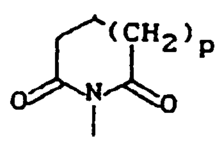 , 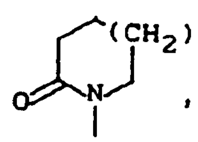 , 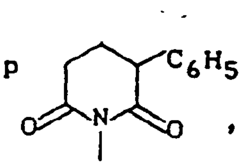 ,

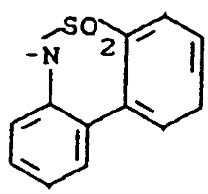 , 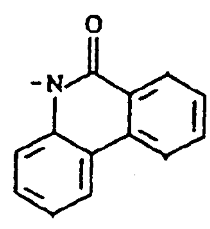 , 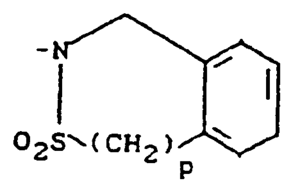 ,

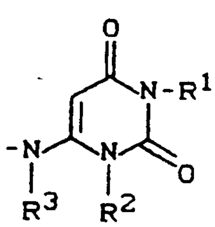 , 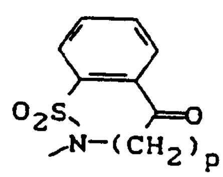 , 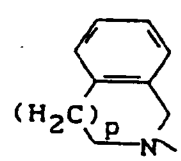 ,

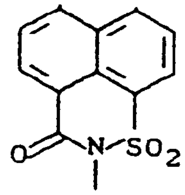,
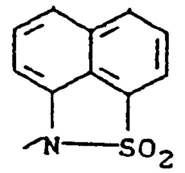,
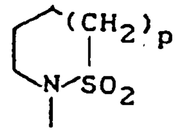 or
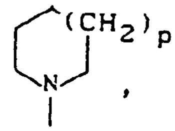,

wherein
p - denotes a number 0, 1 or 2,
$R^1$, $R^2$ and $R^3$ have the abovementioned meaning,
n - denotes a number from 1 to 10
and their salts, characterised in that
3-substituted pyrrolidines of the general formula (II)

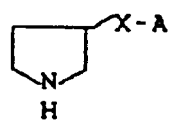
(II)

in which A and X have the abovementioned meaning,
or their salts
are reacted in a first step with alkyl derivatives of the general formula (III)

$$R-(CH_2)_n-B' \qquad (III)$$

wherein
n - has the abovementioned meaning,
B′ - corresponds to the range of meaning of B, where, however, $R^5$ and $R^6$ do not simultaneously represent hydrogen or simultaneously represent hydrogen and alkyl or aryl,
R - represents chlorine, bromine, iodine, methylsulphonyloxy, phenylsulphonyloxy, tolylsulphonyloxy, trifluoroacetoxy or trifluoromethylsulphonyloxy,
in inert solvents, if appropriate in the presence of bases,
then, if desired, functional groups are converted into other functional groups by reduction, oxidation, hydrolysis or reaction with nucleophilic or electrophilic or reagents and then, in the case of the preparation of the salts, if desired, the products are reacted with the corresponding acid.

6. Process according to Claim 5, characterised in that the reaction is carried out in the temperature range from -20°C to +150°C.

7. Process for the preparation of 1,3-disubstituted pyrrolidines of the general formula

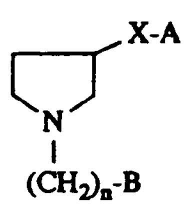
(I),

wherein
A represents phenyl or a monocyclic five- or six-membered hetaryl radical which contains one or two nitrogen and/or oxygen and/or sulphur atoms, onto which are optionally fused one to three aromatic saturated or unsaturated cyclic hydrocarbons having 5 to 8 carbon atoms or five- or six-membered heterocycles having one or two nitrogen and/or oxygen and/or sulphur atoms, where this radical is optionally monosubstituted or disubstituted by $C_1$-$C_6$-alkyl, $C_1$-$C_6$-alkoxy, $C_1$-$C_6$-alkoxycarbonyl, $C_1$-$C_6$-alkenyloxy, acyloxy, benzoyloxy, cyano, phenyl, benzyl, fluorine, chlorine, bromine, trifluoromethyl, trifluoromethoxy, difluoromethoxy or a radical of the formula $-CO-NYZ$, $-NH-SO_2-Y'$, $-SO_2-NYZ$ or $-NH-CO-Y$, where
Y and Z are identical or different and represent hydrogen or $C_1$-$C_6$-alkyl, and
Y′ represents $C_1$-$C_6$-alkyl or aryl having 6 to 12 carbon atoms,

and in the case of nitrogen heterocycles is optionally present as the N-oxide,
X - represents $-CH_2-O-$, $O-CH_2-$ or -O-, and
B - denotes cyano or a group of the formula
$-SO_2NR^2R^3$ or $-SO_mR^4$, where
$R^2$, $R^3$, $R^4$ and m have the meaning specified in Claim 1,
characterised in that 3-substituted pyrrolidines of the general formula (II)

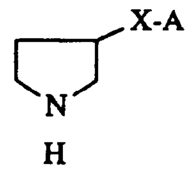

(II),

wherein
A and X have the abovementioned meaning, are reacted with acrylonitrile or a compound of the formula $CH_2=CH-SO_2NR^2R^3$ or $-CH_2=CH_2-SO_mR^4$ in the presence of catalysts.

8. Process according to Claim 7, characterised in that the reaction is carried out in the temperature range from +50°C to +150°C.

9. Medicaments, containing 1,3-disubstituted pyrrolidines according to Claims 1 - 3.

10. Medicaments according to Claim 9, containing 0.5 to 90% by weight of 1,3-disubstituted pyrrolidines, relative to the total mixture.

11. Use of 1,3-disubstituted pyrrolidines according to Claims 1 - 3 for the preparation of medicaments.

12. Use according to Claim 12 for the preparation of medicaments for the treatment of disorders of the central nervous system.

**Claims for the following Contracting State : ES**

1. Process for the preparation of 1,3-disubstituted pyrrolidines of the general formula

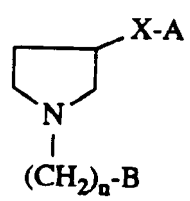

(I)

wherein
A represents phenyl or a monocyclic five- or six-membered hetaryl radical which contains one or two nitrogen and/or oxygen and/or sulphur atoms, onto which are optionally fused one to three aromatic saturated or unsaturated cyclic hydrocarbons having 5 to 8 carbon atoms or five- or six-membered heterocycles having one or two nitrogen and/or oxygen and/or sulphur atoms, where this radical is optionally monosubstituted or disubstituted by $C_1$-$C_6$-alkyl, $C_1$-$C_6$-alkoxy, $C_1$-$C_6$-alkoxycarbonyl, $C_1$-$C_6$-alkenyloxy, acyloxy, benzoyloxy, cyano, phenyl, benzyl, fluorine, chlorine, bromine, trifluoromethyl, trifluoromethoxy, difluoromethoxy or a radical of the formula -CO-NYZ, $-NH-SO_2-Y'$, $-SO_2-NYZ$ or -NH-CO-Y, where
Y and Z are identical or different and represent hydrogen or $C_1$-$C_6$-alkyl, and
Y' represents $C_1$-$C_6$-alkyl or aryl having 6 to 12 carbon atoms,
and in the case of nitrogen heterocycles is optionally present as the N-oxide,
X - represents $-CH_2-O$, $O-CH_2-$ or -O-,
B - represents cyano or a group of the formula $-COOR^1$, $CONR^2R^3$, $-SO_2NR^2R^3$, $-SO_mR^4$, $NR^5R^6$ or $-C{\equiv}C-CH_2-NR^5R^6$, where
$R^1$ - represents hydrogen, $C_1$-$C_{11}$-alkyl, $C_5$-$C_8$-cycloalkyl, $C_2$-$C_{12}$-alkenyl, $C_6$-$C_{12}$-aryl or $C_7$-$C_{14}$-

aralkyl,

$R^2$ and $R^3$ are identical or different and

- represent hydrogen, $C_1$-$C_{12}$-alkyl, $C_5$-$C_8$-cycloalkyl, $C_6$-$C_{12}$-aryl or $C_7$-$C_{14}$-aralkyl, where the aryl radicals can be substituted by halogen, $C_1$-$C_6$-alkoxy or $C_1$-$C_6$-alkoxycarbonyl,

$R^4$ - represents $C_1$-$C_{12}$-alkyl, $C_5$-$C_8$-cycloalkyl, $C_6$-$C_{12}$-aryl or $C_7$-$C_{14}$-aralkyl, where the aryl radicals can be monosubstituted, disubstituted or trisubstituted by identical or different substituents from the series comprising halogen, cyano, $C_1$-$C_6$-alkyl, $C_1$-$C_6$-alkoxy, trifluoromethyl and trifluoromethoxy, or

- represents a group of the formula

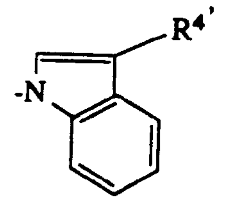

wherein $R^{4'}$ denotes hydrogen or $C_1$-$C_6$-alkyl

m - represents a number 0, 1 or 2,

$R^5$ and $R^6$ are identical or different and

- represent hydrogen, $C_1$-$C_{12}$-alkyl, $C_5$-$C_8$-cycloalkyl, $C_6$-$C_{12}$-aryl or $C_7$-$C_{14}$-aralkyl, where the aryl radicals can be substituted by halogen, cyano, $C_1$-$C_6$-alkyl, $C_1$-$C_6$-alkoxy or trifluoromethyl, or

- represent a group of the formula $-COR^7$ or $-SO_2R^8$, wherein

$R^7$ - denotes hydrogen or

- a group $NHR^9$ or

- $C_1$-$C_{11}$-alkyl, $C_5$-$C_8$-cycloalkyl or $C_1$-$C_{12}$-alkoxy, or

- $C_6$-$C_{12}$-aryl, $C_6$-$C_{12}$-aryloxy, $C_7$-$C_{14}$-aralkyl, $C_6$-$C_{12}$-aralkoxy or a five- or six-membered heteroaryl ring having one or two oxygen and/or nitrogen and/or sulphur atoms, where the radicals mentioned can be monosubstituted, disubstituted or trisubstituted by identical or different substituents from the series comprising $C_1$-$C_6$-alkyl, $C_1$-$C_6$-alkoxy, $C_1$-$C_6$-alkylthio, halogen, cyano, trifluoromethyl, trifluoromethoxy, trifluoromethylthio, amino, $C_1$-$C_6$-alkyl-amino and $C_1$-$C_6$-dialkyl -amino

$R^8$ - denotes $C_5$-$C_8$-cycloalkyl, or

- $C_1$-$C_{12}$-alkyl which can be substituted by cyano, halogen, $C_1$-$C_{12}$-alkoxy or $C_1$-$C_{12}$-alkoxycarbonyl, or

- $C_6$-$C_{12}$-aryl, $C_7$-$C_{14}$-aralkyl or a five- or six-membered heteroaryl ring having one or two oxygen and/or nitrogen and/or sulphur atoms, where the radicals mentioned can be monosubstituted, disubstituted or trisubstituted by identical or different substituents from the series comprising $C_1$-$C_6$-alkyl, $C_1$-$C_6$-alkoxy, $C_1$-$C_6$-alkylthio, halogen, cyano, trifluoromethyl, trifluoromethoxy, trifluoromethylthio, nitro, amino, $C_1$-$C_6$-alkyl-amino and $C_1$-$C_6$-dialkyl-amino or

- denotes a group $NR^2R^3$, where

$R^2$ and $R^3$ have the abovementioned meaning and

$R^9$ - denotes hydrogen, or

- $C_5$-$C_8$-cycloalkyl, or

- $C_1$-$C_{12}$-alkyl optionally substituted by cyano, fluorine, chlorine or bromine, or

- $C_6$-$C_{12}$-aryl, $C_7$-$C_{14}$-aralkyl or a five - or six-membered heteroaryl ring having one or two oxygen and/or nitrogen and/or sulphur atoms, where the aryl radicals can be monosubstituted, disubstituted or trisubstituted by identical or different substituents from the series comprising $C_1$-$C_6$-alkyl, $C_1$-$C_6$-alkoxy, $C_1$-$C_6$-alkylthio, halogen, cyano, trifluoromethyl, trifluoromethoxy, trifluoromethylthio, nitro, amino, $C_1$-$C_6$-alkyl-amino and $C_1$-$C_6$-dialkylamino,

or where

$R^5$ and $R^6$, together with the nitrogen atom, form a ring from the series comprising

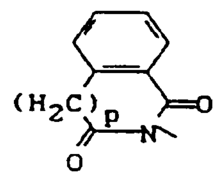 , 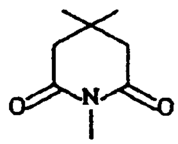 , 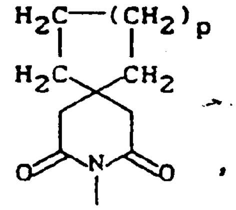 ,

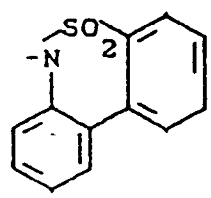 , 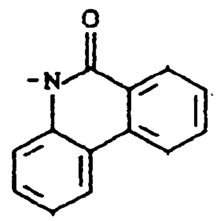 , 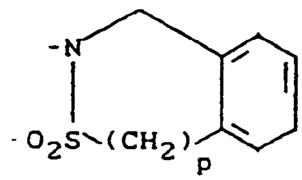 ,

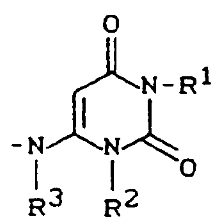 , 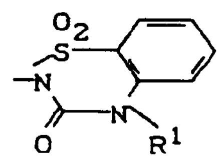 , 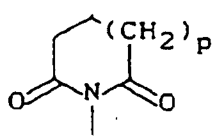 , 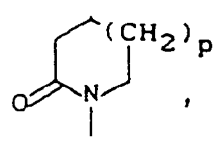 ,

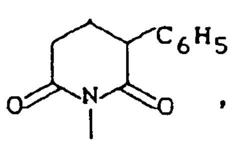 , 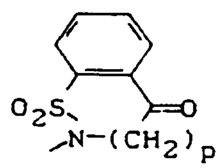 , 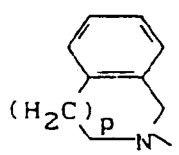 , 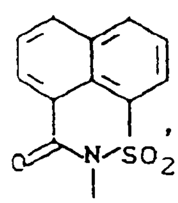 ,

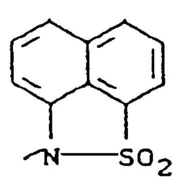 , 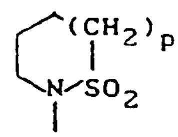 or 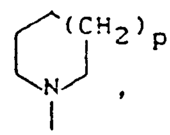 ,

wherein
p - denotes a number 0, 1 or 2,
$R^1$, $R^2$ and $R^3$ have the abovementioned meaning,
n - denotes a number from 1 to 10
and their salts, characterised in that
3-substituted pyrrolidines of the general formula (II)

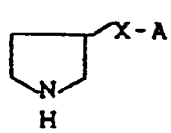 (II)

in which A and X have the abovementioned meaning,
or their salts

are reacted in a first step with alkyl derivatives of the general formula (III)

$$R-(CH_2)_n-B' \quad (III)$$

wherein

n - has the abovementioned meaning,

B′ - corresponds to the range of meaning of B, where, however, $R^5$ and $R^6$ do not simultaneously represent hydrogen or simultaneously represent hydrogen and alkyl or aryl,

R - represents chlorine, bromine, iodine, methylsulphonyloxy, phenylsulphonyloxy, tolylsulphonyloxy, trifluoroacetoxy or trifluoromethylsulphonyloxy,

in inert solvents, if appropriate in the presence of bases,

then, if desired, functional groups are converted into other functional groups by reduction, oxidation, hydrolysis or reaction with nucleophilic or electrophilic reagents and then, in the case of the preparation of the salts, if desired, the products are reacted with the corresponding acid.

**2.** Process according to Claim 1 for the preparation of compounds of the formula (I), in which

A - represents phenyl or a monocyclic five- or six-membered hetaryl radical which contains one or two nitrogen and/or oxygen and/or sulphur atoms, onto which one to three aromatic, saturated or unsaturated cyclic hydrocarbons having 5 to 8 carbon atoms or five- or six-membered heterocycles having one or two nitrogen and/or oxygen and/or sulphur atoms are optionally fused, where this radical is optionally monosubstituted or disubstituted by $C_1$-$C_6$-alkyl, $C_1$-$C_6$-alkoxy, $C_1$-$C_6$-alkoxycarbonyl, $C_1$-$C_6$-alkenyloxy, acyloxy, benzoyloxy, cyano, phenyl, benzyl, fluorine, chlorine, bromine, trifluoromethyl, trifluoromethoxy, difluoromethoxy or a radical or the formula -CO-NYZ, $-NH-SO_2-Y'$, $-SO_2-NYZ$ or -NH-CO-Y, where

Y and Z are identical or different and represent hydrogen or $C_1$-$C_6$-alkyl and

Y′ represents $C_1$-$C_6$-alkyl or aryl having 6 to 12 carbon atoms,

and in the case of nitrogen heterocycles is optionally present as the N-oxide,

X - represents $-CH_2-O-$, $-O-CH_2-$, or -O-,

B - represents cyano or a group of the formula $-COOR^1$, $CONR^2R^3$, $-SO_2NR^2R^3$, $-SO_mR^4$, $NR^5R^6$ or $-C{\equiv}C-CH_2-NR^5R^6$, where

$R^1$ - represents hydrogen, $C_1$-$C_6$-alkyl or phenyl,

$R^2$ and $R^3$ are identical or different and

- represent hydrogen, $C_1$-$C_6$-alkyl or phenyl which, in turn, can be substituted by fluorine, chlorine, bromine, $C_1$-$C_6$-alkoxy or $C_1$-$C_6$-alkoxycarbonyl,

$R^4$ - represents $C_1$-$C_6$-alkyl, or

- represents phenyl which can be monosubstituted or disubstituted by identical or different substituents from the series comprising fluorine, chlorine, bromine, $C_1$-$C_6$-alkyl and $C_1$-$C_6$-alkoxy, or

- represents a group of the formula

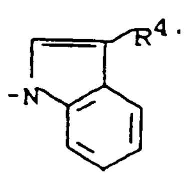

wherein $R^{4\prime}$, denotes hydrogen or $C_1$-$C_6$-alkyl,

m - represents a number 0, 1 or 2,

$R^5$ and $R^6$ are identical or different and

- represent hydrogen, $C_1$-$C_6$-alkyl, phenyl or benzyl, where the phenyl radicals can be substituted by fluorine, chlorine, bromine, $C_1$-$C_6$-alkyl, $C_1$-$C_6$-alkoxy or trifluoromethyl, or

- represents a group of the formula $-COR^7$ or $-SO_2R^8$,

wherein

$R^7$ - denotes hydrogen, or

- denotes a group $NHR^9$, or

- denotes $C_1$-$C_6$-alkyl or $C_1$-$C_6$-alkoxy, or

- phenyl, benzyl, benzyloxy, thienyl, furyl, pyridyl, pyrimidyl, quinolyl, isoquinolyl, benzothiazolyl, benzoxazolyl, thiazolyl, oxazolyl, isoxazolyl or isothiazolyl optionally substituted by $C_1$-$C_6$-alkyl, $C_1$-$C_6$-alkoxy, fluorine, chlorine, bromine, trifluoromethyl, dimethylamino or diethylamino,

$R^8$ - denotes cyclopropyl,cyclopentyl, cyclohexyl, or

- $C_1$-$C_6$-alkyl which is optionally substituted by cyano, fluorine, chlorine, bromine, trifluoro-

methyl or $C_1$-$C_6$-alkoxycarbonyl, or

- denotes phenyl, naphthyl, benzyl, thienyl, furyl, pyrimidyl, pyridyl, quinolyl, isoquinolyl, benzothiazolyl, benzoxazolyl, thiazolyl, oxazolyl, isoxazolyl or isothiazolyl optionally substituted by $C_1$-$C_6$-alkyl, $C_1$-$C_6$-alkoxy, fluorine, chlorine, bromine, nitro, trifluoromethyl, dimethylamino or diethylamino, or

- denotes a group $NR^2R^3$, where

$R^2$ and $R^3$ have the abovementioned meaning, and

$R^9$ - denotes $C_1$-$C_6$-alkyl which is optionally substituted by cyano, fluorine, chlorine or bromine, or

- denotes phenyl, benzyl, thienyl, furyl, pyridyl, pyrimidyl, quinolyl, isoquinolyl, benzothiazolyl, benzoxazolyl, thiazolyl, oxazolyl, isoxazolyl or isothiazolyl optionally substituted by $C_1$-$C_6$-alkyl, $C_1$-$C_6$-alkoxy, fluorine, chlorine, bromine, trifluoromethyl, dimethylamino or diethylamino,

or

$R^5$ and $R^6$, together with the nitrogen atom, form a ring from the series comprising

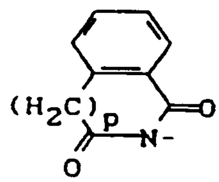 , 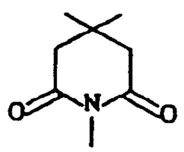 , 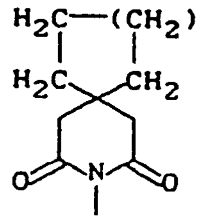 ,

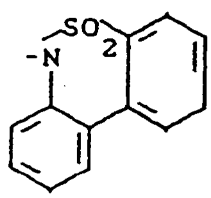 , 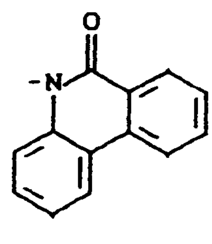 , 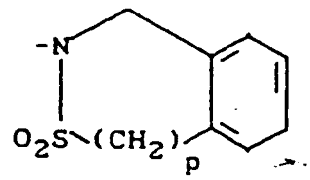 ,

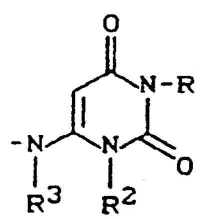 , 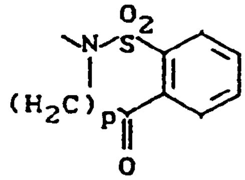 , 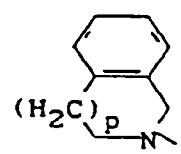 , 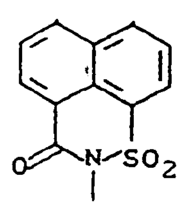

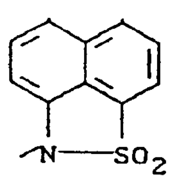 , 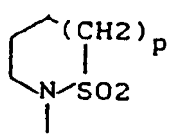 , 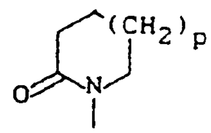 ,

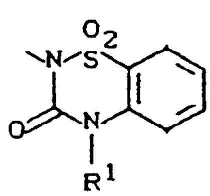 or 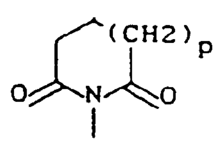

wherein

p - denotes a number 0, 1 or 2,

$R^1$, $R^2$ and $R^3$ have the abovementioned meaning, and

n - denotes a number from 1 to 8,
and their salts.

3. Process according to Claim 1 for the preparation of compounds of the formula (I), in which

A - represents phenyl or naphthyl optionally substituted by identical or different substituents from the series comprising $C_1$-$C_6$-alkyl, $C_1$-$C_6$-alkoxy, $C_1$-$C_6$-alkoxycarbonyl, $C_1$-$C_6$-alkenyloxy, acyloxy, benzoyloxy, cyano, phenyl, benzyl, sulphonylamino, sulphamoyl ($C_1$ to $C_6$), carbamoyl, carbonylamino, fluorine, chlorine, bromine, trifluoromethyl, trifluoromethoxy and difluoromethoxy, furan, thiophene, isoxazole, pyridine, pyrimidine, indole, indazole, benzofuran, benzisoxazole, quinoline, isoquinoline, tetralin, indene, chroman, dihydrobenzodioxin, dihydroindole, tetrahydroquinoline or dihydrobenzofuran,

X - represents -O-$CH_2$-, -$CH_2$-O- or -O-,

B - represents cyano, or

- a group of the formula -$CONR^2R^3$, -$NR^5R^6$, - $SO_mR^4$, -C≡C-$CH_2$-$NR^5R^6$, -$SO_2NR^2R^3$ or $COOR^1$, where

$R^1$ - denotes hydrogen, methyl, ethyl or phenyl,

$R^2$ and $R^3$ are identical or different and

- denote hydrogen, methyl, ethyl, propyl, isopropyl, butyl or isobutyl or denote phenyl which can be substituted by methoxycarbonyl

$R^4$ - represents methyl or ethyl or

- represents a group of the formula

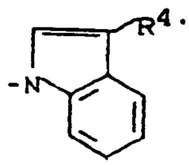

wherein $R^{4'}$, denotes hydrogen or methyl,

$R^5$ and $R^6$ are identical or different, and

- denote hydrogen, methyl, ethyl, propyl, isopropyl, butyl or isobutyl, or

- phenyl optionally substituted by fluorine, chlorine, methyl or methoxy, or

- denote a group -$COR^7$ or -$SO_2R^8$, wherein

$R^7$ - represents a group $NHR^9$, or

- represents methyl, ethyl, propyl, isopropyl, methoxy, ethoxy, propoxy, isopropoxy, tert.butoxy or

- phenyl, benzyl, benzyloxy, thienyl, furyl, pyridyl, pyrimidyl, quinolyl or isoquinolyl optionally substituted by methyl, methoxy, fluorine or chlorine,

$R^8$ - represents methyl, ethyl, propyl, isopropyl, butyl or isobutyl optionally substituted by fluorine, chlorine, methoxycarbonyl, ethoxycarbonyl, propoxycarbonyl, isopropoxycarbonyl, butoxycarbonyl, isobutoxycarbonyl or tert.butoxycarbonyl, or

- represents phenyl, naphthyl, thienyl, furyl, pyridyl, pyrimidyl, quinolyl or isoquinolyl optionally substituted by methyl, ethyl, propyl, isopropyl, methoxy, fluorine, chlorine or nitro, or

- represents a group $NR^2R^3$, where

$R^2$ and $R^3$ have the abovementioned meaning,

$R^9$ - denotes methyl, ethyl, propyl, isopropyl, butyl, isobutyl, pentyl, isopentyl, hexyl or isohexyl optionally substituted by fluorine or chlorine, or

- denotes phenyl which can be substituted by fluorine, chlorine, methyl or methoxy, or

$R^5$ and $R^6$, together with the nitrogen atom, form a ring from the series comprising

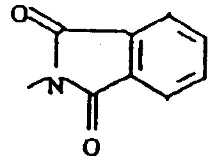 , 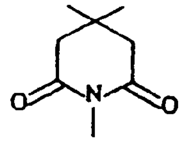 , 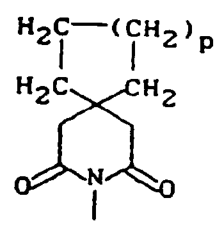 ,

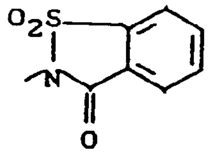 , 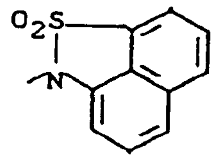 , 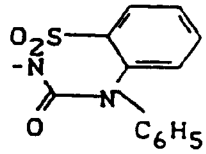 ,

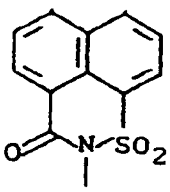 , 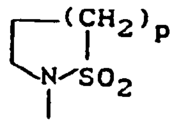 , 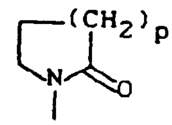 ,

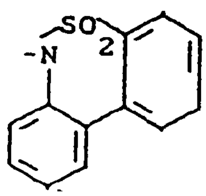 , 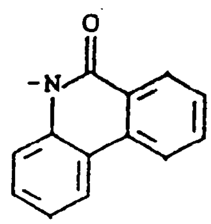 , 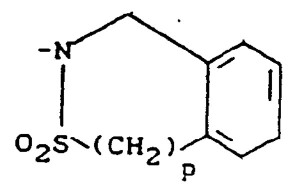 ,

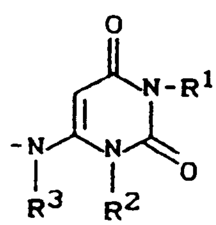 or 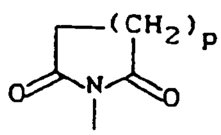

p - denotes a number 1 or 2
$R^1$, $R^2$ and $R^3$ have the abovementioned meaning and
n - denotes a number from 1 to 6,
and their salts.

4. Process according to Claim 1, characterised in that it is carried out in a temperature range from -20°C to +150°C.

5. Process for the preparation of 1,3-disubstituted pyrrolidines of the general formula

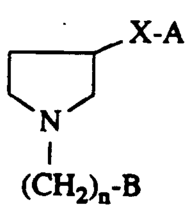 (I),

wherein

A represents phenyl or a monocyclic five- or six-membered hetaryl radical which contains one or two nitrogen and/or oxygen and/or sulphur atoms, onto which are optionally fused one to three aromatic saturated or unsaturated cyclic hydrocarbons having 5 to 8 carbon atoms or five- or six-membered heterocycles having one or two - nitrogen and/or oxygen and/or sulphur atoms, where this radical is optionally monosubstituted or disubstituted by $C_1$-$C_6$-alkyl, $C_1$-$C_6$-alkoxy, $C_1$-$C_6$-alkoxycarbonyl, $C_1$-$C_6$-alkenyloxy, acyloxy, benzoyloxy, cyano, phenyl, benzyl, fluorine, chlorine, bromine, trifluoromethyl, trifluoromethoxy, difluoromethoxy or a radical of the formula -CO-NYZ, -NH-$SO_2$-Y′, -$SO_2$-NYZ or -NH-CO-Y, where

Y and Z are identical or different and represent hydrogen or $C_1$-$C_6$-alkyl, and

Y′ represents $C_1$-$C_6$-alkyl or aryl having 6 to 12 carbon atoms,

and in the case of nitrogen heterocycles is optionally present as the N-oxide,

X - represents $-CH_2-O-$, $O-CH_2-$ or -O-, and

B - denotes cyano or a group of the formula

$-SO_2NR^2R^3$ or $-SO_mR^4$,

where

$R^2$, $R^3$, $R^4$ and m have the meaning specified in Claim 1,

characterised in that 3-substituted pyrrolidines of the general formula (II)

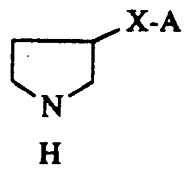

(II),

wherein

A and X have the abovementioned meaning, are reacted with acrylonitrile or a compound of the formula $CH_2=CH-SO_2NR^2R^3$ or $-CH_2=CH_2-SO_mR^4$ in the presence of catalysts.

6. Process according to Claim 5, characterised in that the reaction is carried out in a temperature range from +50°C to +150°C.

## Revendications

### Revendications pour les Etats contractants suivants : AT, BE, CH, DE, FR, GB, GR, IT, LI, NL, SE

1. Pyrrolidines substituées en positions 1,3 de formule générale

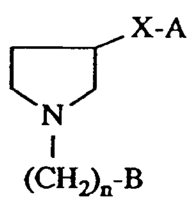

(I)

dans laquelle

A représente un reste phényle ou un reste hétéro-aryle monocyclique pentagonal ou hexagonal, qui comprend un ou deux atomes d'azote et/ou d'oxygène et/ou de soufre, auquel sont condensés le cas échéant un à trois hydrocarbures aromatiques, cycliques saturés ou non saturés ayant 5 à 8 atomes de carbone ou des hétérocycles pentagonaux ou hexagonaux comportant un ou deux atomes d'azote et/ou d'oxygène et/ou de soufre, ce reste portant éventuellement un ou deux substituants alkyle en $C_1$ à $C_6$, alkoxy en $C_1$ à $C_6$, (alkoxy en $C_1$ à $C_6$) carbonyle, alcényloxy en $C_1$ à $C_6$, acyloxy, benzoyloxy, cyano, phényle, benzyle, fluoro, chloro, bromo, trifluorométhyle, trifluorométhoxy, difluorométhoxy ou étant substitué le cas échéant par un reste de formule -CO-NYZ, $-NH-SO_2-Y'$, $-SO_2NYZ$ ou -NH-CO-Y, où

Y,Z sont identiques ou différents et représentent l'hydrogène ou un groupe alkyle en $C_1$ à $C_6$ et

Y′ est un groupe alkyle en $C_1$ à $C_6$ ou aryle ayant 6 à 12 atomes de carbone,

et se présentant le cas échéant sous forme de N-oxyde dans le cas d'hétérocycles azotés

X - représente un groupe $-CH_2-O-$, $-O-CH_2-$ ou -O-,

B - est un groupe cyano ou un groupe de formule $-COOR^1$, $CONR^2R^3$, $-SO_2NR^2R^3$, $-SO_mR^4$, $NR^5R^6$ ou

$-C{\equiv}C-CH_2-NR^5R^6$ où

$R^1$ - est l'hydrogène, un groupe alkyle en $C_1$ à $C_{11}$, cycloalkyle en $C_5$ à $C_8$, alcényle en $C_2$ à $C_{12}$,

aryle en $C_6$ à $C_{12}$ ou aralkyle en $C_7$ à $C_{14}$,

$R^2$ et $R^3$ sont identiques ou différents et représentent

- l'hydrogène, un groupe alkyle en $C_1$ à $C_{12}$, cycloalkyle en $C_5$ à $C_8$, aryle en $C_6$ à $C_{12}$ ou aralkyle en $C_7$ à $C_{14}$, les restes aryle pouvant être substitués par un halogène, un radical alkoxy en $C_1$ à $C_6$ ou (alkoxy en $C_1$ à $C_6$)-carbonyle,

$R^4$ - est un groupe alkyle en $C_1$ à $C_{12}$, cycloalkyle en $C_5$ à $C_8$, aryle en $C_6$ à $C_{12}$ ou aralkyle en $C_7$ à $C_{14}$, les restes aryle pouvant porter jusqu'à 3 substituants, identiques ou différents, halogéno, cyano, alkyle en $C_1$ à $C_6$, alkoxy en $C_1$ à $C_6$, trifluorométhyle ou trifluorométhoxy, ou bien

- un groupe de formule

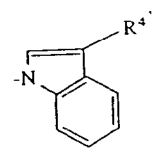

dans laquelle $R^{4'}$ est l'hydrogène ou un radical alkyle en $C_1$ à $C_6$,

m - est le nombre 0, 1 ou 2,

$R^5$ et $R^6$ sont identiques ou différents et représentent

- l'hydrogène, un groupe alkyle en $C_1$ à $C_{12}$, cycloalkyle en $C_5$ à $C_8$, aryle en $C_6$ à $C_{12}$ ou aralkyle en $C_7$ à $C_{14}$, les restes aryle pouvant être substitués par un halogène, un radical cyano, alkyle en $C_1$ à $C_6$, alkoxy en $C_1$ à $C_6$, ou trifluorométhyle, ou bien

- un groupe de formule $-COR^7$ ou $-SO_2R^8$, dans laquelle

$R^7$

- représente l'hydrogène ou
- un groupe $NHR^9$ ou
- un groupe alkyle en $C_1$ à $C_{11}$, cycloalkyle en $C_5$ à $C_8$ ou alkoxy en $C_1$ à $C_{12}$, ou
- un groupe aryle en $C_6$ à $C_{12}$, aryloxy en $C_6$ à $C_{12}$, aralkyle en $C_7$ à $C_{14}$, aralkoxy en $C_6$ à $C_{12}$ ou un noyau hétéroaryle pentagonal ou hexagonal portant un ou deux atomes d'oxygène et/ou d'azote et/ou de soufre, les restes mentionnés pouvant porter jusqu'à 3 substituants identiques ou différents, alkyle en $C_1$ à $C_6$, alkoxy en $C_1$ à $C_6$, alkylthio en $C_1$ à $C_6$, halogéno, cyano, trifluorométhyle, trifluorométhoxy, trifluorométhylthio, amino, alkylamino en $C_1$ à $C_6$ ou di(alkyle en $C_1$ à $C_6$)amino,

$R^8$

- est un groupe cycloalkyle en $C_5$ à $C_8$ ou
- alkyle en $C_1$ à $C_{12}$ qui peut être substitué par un radical cyano, halogéno, alkoxy en $C_1$ à $C_{12}$ ou (alkoxy en $C_1$ à $C_{12}$)-carbonyle, ou bien
- un groupe aryle en $C_6$ à $C_{12}$, aralkyle en $C_7$ à $C_{14}$ ou un noyau hétéroaryle pentagonal ou hexagonal portant un ou deux atomes d'oxygène et/ou d'azote et/ou de soufre, les restes mentionnés pouvant porter jusqu'à 3 substituants, identiques ou différents, alkyle en $C_1$ à $C_6$, alkoxy en $C_1$ à $C_6$, alkylthio en $C_1$ à $C_6$, halogéno, cyano, trifluorométhyle, trifluorométhoxy, trifluorométhylthio, nitro, amino, (alkyle en $C_1$ à $C_6$)-amino, di(alkyle en $C_1$ à $C_6$)-amino,

ou

- un groupe $NR^2R^3$, dans lequel $R^2$ et $R^3$ ont la définition indiquée ci-dessus et

$R^9$

- représente l'hydrogène ou
- un groupe cycloalkyle en $C_5$ à $C_8$ ou
- un groupe alkyle en $C_1$ à $C_{12}$ portant facultativement un substituant cyano, fluoro, chloro ou bromo, ou
- un groupe aryle en $C_6$ à $C_{12}$, aralkyle en $C_7$ à $C_{14}$, un noyau hétéroaryle pentagonal ou hexagonal comportant 1 ou 2 atomes d'oxygène et/ou d'azote et/ou de soufre, les restes aryle pouvant porter jusqu'à 3 substituants, identiques ou différents, alkyle en $C_1$ à $C_6$, alkoxy en $C_1$ à $C_6$, alkylthio en $C_1$ à $C_6$, halogéno, cyano, trifluorométhyle, trifluorométhoxy, trifluorométhylthio, nitro, amino, alkylamino en $C_1$ à $C_6$ ou di(alkyle en $C_1$ à $C_6$)-amino,

ou bien

$R^5$ et $R^6$ forment conjointement avec l'atome d'azote un noyau de la série

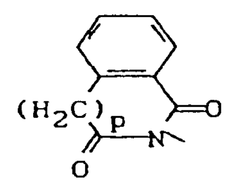 , 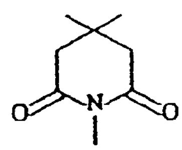 , 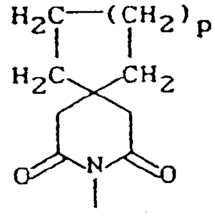 ,

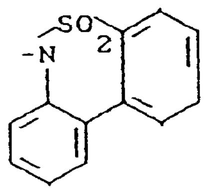 , 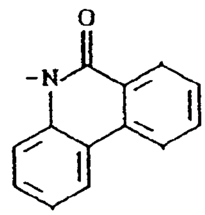 , 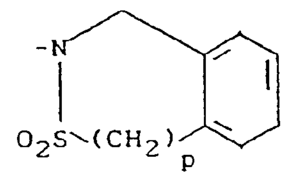 ,

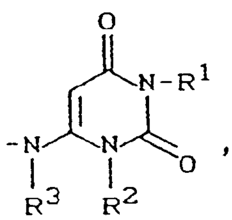 , 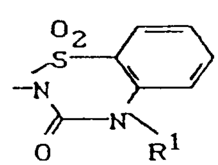 , 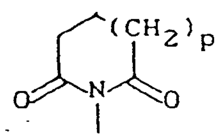 , 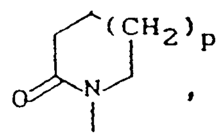 ,

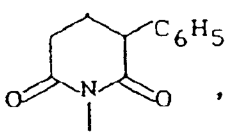 , 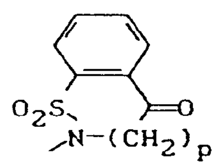 , 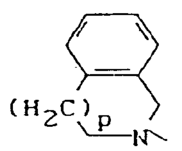 , 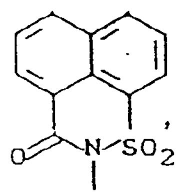 ,

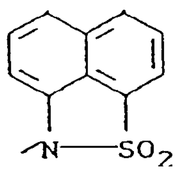 , 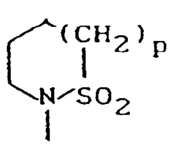 ou 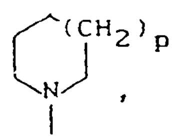 ,

où
p - est le nombre 0, 1 ou 2,
$R^1$, $R^2$ ou $R^3$ ont la définition indiquée ci-dessus
n - est un nombre de 1 à 10, et leurs sels.

**2.** Pyrrolidines disubstituées en positions 1,3 suivant la revendication 1, dans lesquelles

A - est un reste phényle ou un reste hétéroaryle monocyclique pentagonal ou hexagonal qui contient un ou deux atomes d'azote et/ou d'oxygène et/ou de soufre, auquel sont condensés le cas échéant un à trois hydrocarbures aromatiques, cycliques saturés ou non saturés ayant 5 à 8 atomes de carbone ou des hétérocycles pentagonaux ou hexagonaux ayant un ou deux atomes d'azote et/ou d'oxygène et/ou de soufre, ce reste étant éventuellement substitué une ou deux fois par un radical alkyle $C_1$ à $C_6$, alkoxy en $C_1$ à $C_6$, (alkoxy en $C_1$ à $C_6$)carbonyle, alcényloxy en $C_1$ à $C_6$, acyloxy, benzoyloxy, cyano, phényle, benzyle, fluoro, chloro, bromo, trifluorométhyle, trifluorométhoxy, difluorométhoxy ou un reste de formule -CO-NYZ, $-NH-SO_2-Y'$, $-SO_2NYZ$ ou -NH-CO-Y, où

Y, Z sont identiques ou différents et représentent l'hydrogène ou un groupe alkyle en $C_1$ à $C_6$ et

Y' est un groupe alkyle en $C_1$ à $C_6$ ou aryle ayant 6 à 12 atomes de carbone,
et dans le cas d'hétérocycles azotés, se présente éventuellement sous forme de N-oxyde,
X - représente un groupe $-CH_2-O-$, $-O-CH_2-$ ou -O-,
B - est un groupe cyano ou un groupe de formule $-COOR^1$, $CONR^2R^3$, $-SO_2NR^2R^3$, $-SO_mR^4$, $NR^5R^6$ ou $-C{\equiv}C-CH_2-NR^5R^6$ où
$R^1$ - est l'hydrogène, un groupe alkyle en $C_1$ à $C_6$ ou phényle,
$R^2$ et $R^3$ sont identiques ou différents et représentent
- l'hydrogène,un groupe alkyle en $C_1$ à $C_6$ ou phényle qui peut être substitué quant à lui par du fluor, du chlore, du brome, un radical alkoxy en $C_1$ à $C_6$ ou (alkoxy en $C_1$ à $C_6$)carbonyle,
$R^4$ - est un groupe alkyle en $C_1$ à $C_6$ ou
- un groupe phényle qui peut porter jusqu'à deux substituants, identiques ou différents, fluoro, chloro, bromo, alkyle en $C_1$ à $C_6$ ou alkoxy en $C_1$ à $C_6$, ou bien
- un groupe de formule

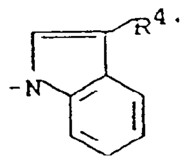

dans laquelle $R^{4'}$ représente l'hydrogène ou un groupe alkyle en $C_1$ à $C_6$,
m - est le nombre 0, 1 ou 2,
$R^5$ et $R^6$ sont identiques ou différents et représentent
- l'hydrogène, un groupe alkyle en $C_1$ à $C_6$, phényle ou benzyle, les restes phényle pouvant être substitués par du fluor, du chlore, du brome, un radical alkyle en $C_1$ à $C_6$, alkoxy en $C_1$ à $C_6$ ou trifluorométhyle, ou bien
- un groupe de formule $-COR^7$ ou $-SO_2R^8$, où
$R^7$
- représente l'hydrogène ou
- un groupe $NHR^9$ ou
- un groupe alkyle en $C_1$ à $C_6$ ou alkoxy en $C_1$ à $C_6$ ou
- un groupe phényle, benzyle, benzyloxy, thiényle, furyle, pyridyle, pyrimidyle, quinolyle, isoquinolyle, benzothiazolyle, benzoxazolyle, thiazolyle, oxazolyle, isoxazolyle ou isothiazolyle éventuellement substitué par un radical alkyle en $C_1$ à $C_6$, alkoxy en $C_1$ à $C_6$, fluoro, chloro, bromo, trifluorométhyle, diméthylamino ou diéthylamino,
$R^8$ - est un groupe cyclopropyle, cyclopentyle, cyclohexyle ou
- un groupe alkyle en $C_1$ à $C_6$ portant éventuellement un substituant cyano, chloro, chloro, bromo, trifluorométhyle ou (alkoxy en $C_1$ à $C_6$)-carbonyle, ou bien
- un groupe phényle, naphtyle, benzyle, thiényle, furyle, pyrimidyle, pyridyle, quinolyle, isoquinolyle, benzothiazolyle, benzoxazolyle, thiazolyle, oxazolyle, isoxazolyle ou isothiazolyle éventuellement substitué par un radical alkyle en $C_1$ à $C_6$, alkoxy en $C_1$ à $C_6$, fluoro, chloro, bromo, nitro, trifluorométhyle, diméthylamino ou diéthylamino, ou bien
- un groupe $NR^2R^3$,
dans lequel
$R^2$ et $R^3$ ont la définition indiquée ci-dessus et
$R^9$
- est un groupe alkyle en $C_1$ à $C_6$ éventuellement substitué par un radical cyano, fluoro, chloro ou bromo, ou bien
- un groupe phényle, benzyle, thiényle, furyle, pyridyle, pyrimidyle, quinolyle, isoquinolyle, benzothiazolyle, benzoxazolyle, thiazolyle, oxazolyle, isoxazolyle ou isothiazolyle portant éventuellement un substituant alkyle en $C_1$ à $C_6$, alkoxy en $C_1$ à $C_6$, fluoro, chloro, bromo, trifluorométhyle, diméthylamino ou diéthylamino, ou bien
$R^5$ et $R^6$ forment conjointement avec l'atome d'azote un noyau de la série

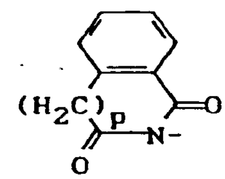 , 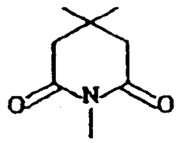 , 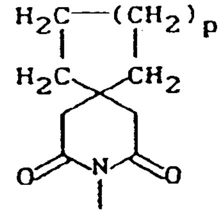 ,

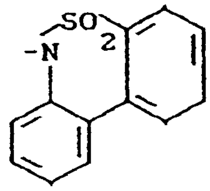 , 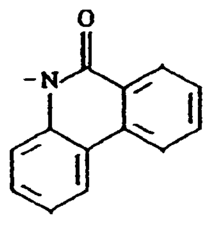 , 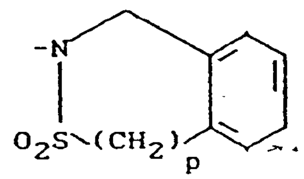 ,

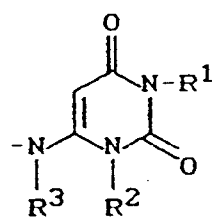 , 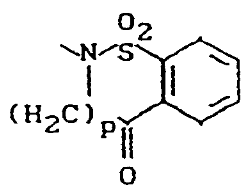 , 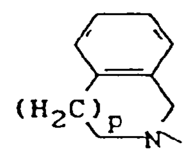 , 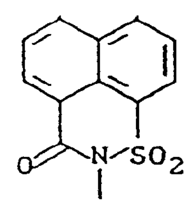

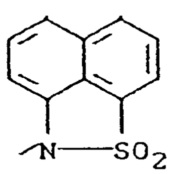 , 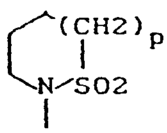 , 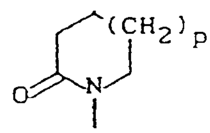 ,

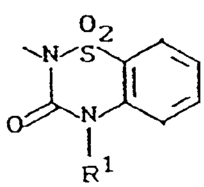 ou 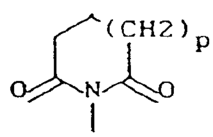

où
p - est le nombre 0, 1 ou 2,
$R^1$, $R^2$ et $R^3$ ont la définition indiquée ci-dessus et
n - est un nombre de 1 à 8, et leurs sels.

**3.** Pyrrolidines disubstituées en positions 1,3 suivant la revendication 1, dans lesquelles

A - représente un groupe phényle ou naphtyle portant éventuellement des substituants, identiques ou différents, alkyle en $C_1$ à $C_6$, alkoxy en $C_1$ à $C_6$, (alkoxy en $C_1$ à $C_6$)carbonyle, alcényloxy en $C_1$ à $C_6$, acyloxy, benzoyloxy, cyano, phényle, benzyle, sulfonylamino, sulfamoyle en $C_1$ à $C_6$, carbamoyle, carbonylamino, fluoro, chloro, bromo, trifluorométhyle, trifluorométhoxy, difluorométhoxy, un groupe furanne, thiophène, isoxazole, pyridine, pyrimidine, indole, indazole, benzofuranne, benzisoxazole, quinoléine, isoquinoléine, tétraline, indène, chromane, dihydrobenzodioxine, dihydroindole, tétrahydroquinoléine ou dihydrobenzofuranne,

X - représente $-O-CH_2-$, $-CH_2-O-$ ou -O-,

B - représente un groupe cyano ou

- un groupe de formule $-CONR^2R^3$, $-NR^5R^6$, $-SO_mR^4$, $-C{\equiv}C-CH_2-NR^5R^6$, $-SO_2NR^2R^3$,

$COOR^1$, où

$R^1$ - est l'hydrogène, un groupe méthyle, éthyle ou phényle,

$R^2$ et $R^3$ sont identiques ou différents et représentent

- l'hydrogène, un groupe méthyle, éthyle, propyle, isopropyle, butyle, isobutyle ou un groupe phényle qui peut être substitué par un radical méthoxycarbonyle

$R^4$

- est un groupe méthyle ou éthyle ou bien
- un groupe de formule

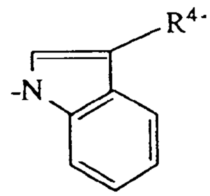

dans laquelle $R^{4'}$ est l'hydrogène ou un groupe méthyle,

$R^5$ et $R^6$ sont identiques ou différents et représentent

- l'hydrogène, un groupe méthyle, éthyle, propyle, isopropyle, butyle, isobutyle ou
- un groupe phényle portant éventuellement un substituant fluoro, chloro, méthyle ou méthoxy, ou bien
- un groupe $-COR^7$ ou $-SO_2R^8$, où

$R^7$

- est un groupe $NHR^9$, ou bien
- un groupe méthyle, éthyle, propyle, isopropyle, méthoxy, éthoxy, propoxy, isopropoxy, tertio-butoxy, ou bien
- un groupe phényle, benzyle, benzyloxy, thiényle, furyle, pyridyle, pyrimidyle, quinolyle ou isoquinolyle portant éventuellement un substituant méthyle, méthoxy, fluoro ou chloro,

$R^8$

- est un groupe méthyle, éthyle, propyle, isopropyle, butyle ou isobutyle portant éventuellement un substituant fluoro, chloro, méthoxycarbonyle, éthoxycarbonyle, propoxycarbonyle, isoproproxycarbonyle, butoxycarbonyle, isobutoxycarbonyle ou tertio-butoxycarbonyle, ou bien
- un groupe phényle, naphtyle, thiényle, furyle, pyridyle, pyrimidyle, quinolyle ou isoquinolyle portant le cas échéant un substituant méthyle, éthyle, propyle, isopropyle, méthoxy, fluoro, chloro, nitro, ou bien
- un groupe $NR^2R^3$, où

$R^2$ et $R^3$ ont la définition indiquée ci-dessus,

$R^9$

- est un groupe méthyle, éthyle, propyle, isopropyle, butyle, isobutyle, pentyle, isopentyle, hexyle ou isohexyle éventuellement substitué par du fluor ou du chlore, ou bien
- un groupe phényle qui peut être substitué par un radical fluoro, chloro, méthyle ou méthoxy, ou bien

$R^5$ et $R^6$ forment conjointement avec l'atome d'azote un noyau de la série

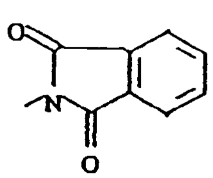 , 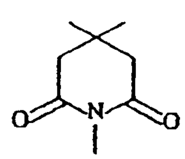 , 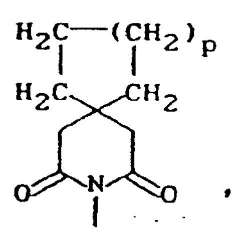 ,

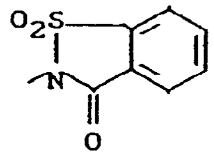 , 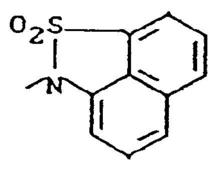 , 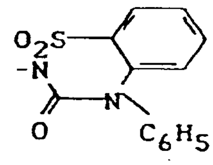 ,

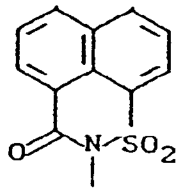 , 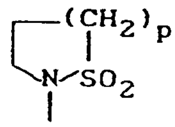 , 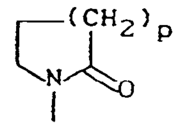 ,

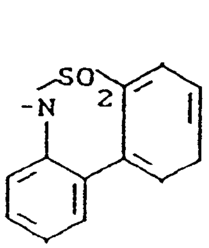 , 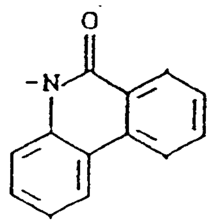 , 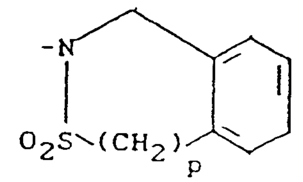 ,

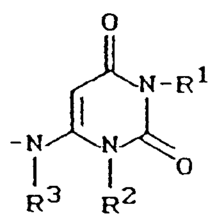 ou 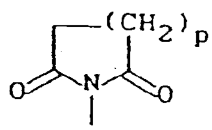

où
p - est le nombre 1 ou 2,
$R^1$, $R^2$ et $R^3$ ont la définition indiquée ci-dessus et
n - est un nombre de 1 à 6, et leurs sels.

4. Pyrrolidines disubstituées en positions 1,3 suivant les revendications 1 à 3, destinées à un traitement thérapeutique.

5. Procédé de production de pyrrolidines disubstituées en positions 1,3 de formule générale

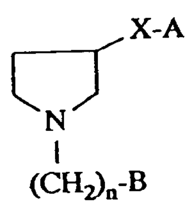 (I)

dans laquelle

A représente un reste phényle ou un reste hétéro-aryle monocyclique pentagonal ou hexagonal, qui comprend un ou deux atomes d'azote et/ou d'oxygène et/ou de soufre, avec lequel sont condensés le cas échéant un à trois hydrocarbures aromatiques cycliques saturés ou non saturés ayant 5 à 8 atomes de carbone ou des hétérocycles pentagonaux ou hexagonaux comportant un ou deux atomes d'azote et/ou d'oxygène et/ou de soufre, ce reste portant éventuellement un ou deux substituants alkyle en $C_1$ à

$C_6$, alkoxy en $C_1$ à $C_6$, (alkoxy en $C_1$ à $C_6$)carbonyle, alcényloxy en $C_1$ à $C_6$, acyloxy, benzoyloxy, cyano, phényle, benzyle, fluoro, chloro, bromo, trifluorométhyle, trifluorométhoxy, difluorométhoxy ou étant substitué le cas échéant par un reste de formule -CO-NYZ, $-NH-SO_2-Y'$, $-SO_2NYZ$ ou -NH-CO-Y, où

Y,Z sont identiques ou différents et représentent l'hydrogène ou un groupe alkyle en $C_1$ à $C_6$ et

Y' est un groupe alkyle en $C_1$ à $C_6$ ou aryle ayant 6 à 12 atomes de carbone,

et se présentant le cas échéant sous forme de N-oxyde dans le cas d'hétérocycles azotés

X - représente un groupe $-CH_2-O-$, $O-CH_2-$ ou -O-,

B - est un groupe cyano ou un groupe de formule $-COOR^1$, $CONR^2R^3$, $-SO_2NR^2R^3$, $-SO_mR^4$, $NR^5R^6$ ou $-C{\equiv}C-CH_2-NR^5R^6$ où

$R^1$ - est l'hydrogène, un groupe alkyle en $C_1$ à $C_{11}$, cycloalkyle en $C_5$ à $C_8$, alcényle en $C_2$ à $C_{12}$, aryle en $C_6$ à $C_{12}$ ou aralkyle en $C_7$ à $C_{14}$,

$R^2$ et $R^3$ sont identiques ou différents et représentent

- l'hydrogène, un groupe alkyle en $C_1$ à $C_{12}$, cycloalkyle en $C_5$ à $C_8$, aryle en $C_6$ à $C_{12}$ ou aralkyle en $C_7$ à $C_{14}$, les restes aryle pouvant être substitués par un halogène, un radical alkoxy en $C_1$ à $C_6$ ou (alkoxy en $C_1$ à $C_6$)-carbonyle,

$R^4$ - est un groupe alkyle en $C_1$ à $C_{12}$, cycloalkyle en $C_5$ à $C_8$, aryle en $C_6$ à $C_{12}$ ou aralkyle en $C_7$ à $C_{14}$, les restes aryle pouvant porter jusqu'à 3 substituants, identiques ou différents, halogéno, cyano, alkyle en $C_1$ à $C_6$, alkoxy en $C_1$ à $C_6$, trifluorométhyle ou trifluoromèthoxy, ou bien

un groupe de formule

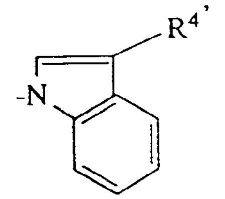

dans laquelle $R^{4'}$ est l'hydrogène ou un radical alkyle en $C_1$ à $C_6$,

m - a la valeur 0, 1 ou 2,

$R^5$ et $R^6$ sont identiques ou différents et représentent

- l'hydrogène, un groupe alkyle en $C_1$ à $C_{12}$, cycloalkyle en $C_5$ à $C_8$, aryle en $C_6$ à $C_{12}$ ou aralkyle en $C_7$ à $C_{14}$, les restes aryle pouvant être substitués par un halogène, un radical cyano, alkyle en $C_1$ à $C_6$, alkoxy en $C_1$ à $C_6$, ou trifluorométhyle, ou bien

- un groupe de formule $-COR^7$ ou $-SO_2R^8$, dans laquelle

$R^7$

- représente l'hydrogène ou
- un groupe $NHR^9$ ou
- un groupe alkyle en $C_1$ à $C_{11}$, cycloalkyle en $C_5$ à $C_8$ ou alkoxy en $C_1$ à $C_{12}$, ou
- un groupe aryle en $C_6$ à $C_{12}$, aryloxy en $C_6$ à $C_{12}$, aralkyle en $C_7$ à $C_{14}$, aralkoxy en $C_6$ à $C_{12}$ ou un noyau hétéroaryle pentagonal ou hexagonal portant un ou deux atomes d'oxygène et/ou d'azote et/ou de soufre, les restes mentionnés pouvant porter jusqu'à 3 substituants, identiques ou diffèrents, alkyle en $C_1$ à $C_6$, alkoxy en $C_1$ à $C_6$, alkylthio en $C_1$ à $C_6$, halogéno, cyano, trifluorométhyle, trifluorométhoxy, trifluorométhylthio, amino, alkylamino en $C_1$ à $C_6$ ou di(alkyle en $C_1$ à $C_6$)amino,

$R^8$

- est un groupe cycloalkyle en $C_5$ à $C_8$ ou
- alkyle en $C_1$ à $C_{12}$ qui peut être substitué par un radical cyano, halogéno, alkoxy en $C_1$ à $C_{12}$ ou (alkoxy en $C_1$ à $C_{12}$)-carbonyle, ou bien
- un groupe aryle en $C_6$ à $C_{12}$, aralkyle en $C_7$ à $C_{14}$ ou un noyau hétéroaryle pentagonal ou hexagonal portant un ou deux atomes d'oxygène et/ou d'azote et/ou de soufre, les restes mentionnés pouvant porter jusqu'à 3 substituants, identiques ou différents, alkyle en $C_1$ à $C_6$, alkoxy en $C_1$ à $C_6$, alkylthio en $C_1$ à $C_6$, halogéno, cyano, trifluorométhyle, trifluorométhoxy, trifluorométhylthio, nitro, amino, (alkyle en $C_1$ à $C_6$)-amino ou di(alkyle en $C_1$ à $C_6$)-amino,

ou

- un groupe $NR^2R^3$, dans lequel $R^2$ et $R^3$ ont la définition indiquée ci-dessus et

$R^9$

- représente l'hydrogène ou
- un groupe cycloalkyle en $C_5$ à $C_8$ ou
- un groupe alkyle en $C_1$ à $C_{12}$ portant facultativement un substituant cyano, fluoro, chloro ou bromo, ou
- un groupe aryle en $C_6$ à $C_{12}$, aralkyle en $C_7$ à $C_{14}$, un noyau hétéroaryle pentagonal ou hexagonal comportant 1 ou 2 atomes d'oxygène et/ou d'azote et/ou de soufre, les restes aryle pouvant porter jusqu'à 3 substituants, identiques ou différents, alkyle en $C_1$ à $C_6$, alkoxy en $C_1$ à $C_6$, alkylthio en $C_1$ à $C_6$, halogéno, cyano, trifluorométhyle, trifluorométhoxy, trifluorométhylthio, nitro, amino,

ou bien

$R^5$ et $R^6$ forment conjointement avec l'atome d'azote un noyau de la série

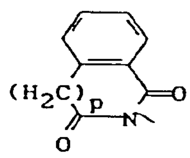 , 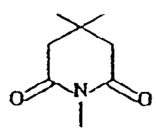 , 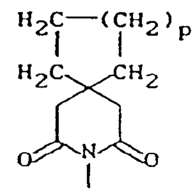 ,

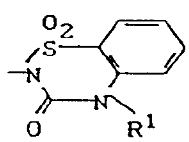 , 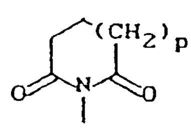 , 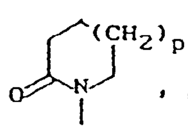 , 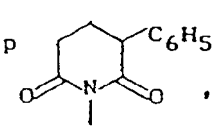 ,

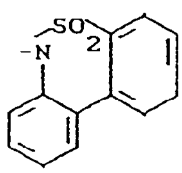 , 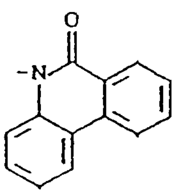 , 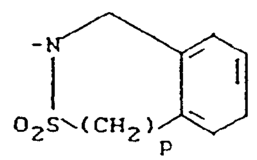 ,

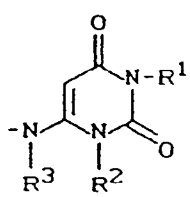 , 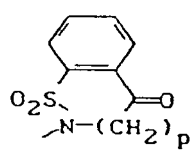 , 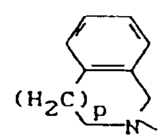 ,

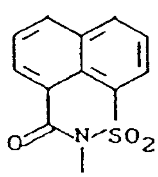 , 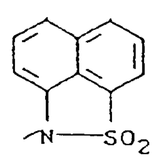 , 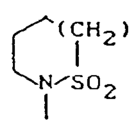 ou 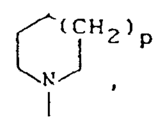 ,

où

p - est le nombre 0, 1 ou 2,

$R^1$, $R^2$ et $R^3$ ont la définition indiquée ci-dessus

n - est un nombre de 1 à 10,

et de leurs sels, caractérisé en ce qu'on fait réagir des pyrrolidines substituées en position 3 de formule générale (II)

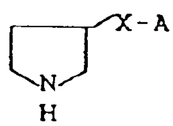

(II)

dans laquelle A et X ont la définition indiquée ci-dessus, ou leurs sels dans une première étape avec des dérivés alkyliques de formule générale (III)

$$R\text{-}(CH_2)_n\text{-}B' \qquad (III)$$

dans laquelle

n - a la définition indiquée ci-dessus

B' - correspond au cadre de définition de B, toutefois $R^5$ et $R^6$ ne représentent pas simultanément l'hydrogène ou simultanément l'hydrogène et un groupe alkyle ou aryle,

R - représente le chlore, le brome, l'iode, un groupe méthylsulfonyloxy, phénylsulfonyloxy, tolylsulfonyloxy, trifluoracétoxy ou trifluorométhylsulfonyloxy,

dans des solvants inertes, éventuellement en présence de bases,

puis on transforme le cas échéant des groupes fonctionnels par réduction, oxydation, hydrolyse ou réaction avec des réactifs nucléophiles ou électrophiles, en d'autres groupes fonctionnels, après quoi dans le cas de la production des sels, on les fait réagir le cas échéant avec un acide correspondant.

**6.** Procédé suivant la revendication 5, caractérisé en ce qu'on conduit la réaction dans la plage de températures de -20°C à +150°C.

**7.** Procédé de production de pyrrolidines disubstituées en positions 1,3 de formule générale

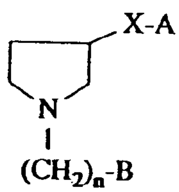

(I)

dans laquelle

A représente un reste phényle ou un reste hétéroaryle monocyclique pentagonal ou hexagonal, qui comprend un ou deux atomes d'azote et/ou d'oxygène et/ou de soufre, auquel sont condensés le cas échéant un à trois hydrocarbures aromatiques cycliques saturés ou non saturés ayant 5 à 8 atomes de carbone ou des hétérocycles pentagonaux ou hexagonaux comportant un ou deux atomes d'azote et/ou d'oxygène et/ou de soufre, ce reste étant substitué le cas échéant une ou deux fois par un radical alkyle en $C_1$ à $C_6$, alkoxy en $C_1$ à $C_6$, (alkoxy en $C_1$ à $C_6$)carbonyle, alcényloxy en $C_1$ à $C_6$, acyloxy, benzoyloxy, cyano, phényle, benzyle, fluoro, chloro, bromo, trifluorométhyle, trifluorométhoxy, difluorométhoxy ou un reste de formule -CO-NYZ, $-NH-SO_2-Y'$, $-SO_2NYZ$ ou -NH-CO-Y, où

Y,Z sont identiques ou différents et représentent l'hydrogène ou un groupe alkyle en $C_1$ à $C_6$ et

Y' est un groupe alkyle en $C_1$ à $C_6$ ou aryle ayant 6 à 12 atomes de carbone,

et se présentant le cas échéant sous forme de N-oxyde dans le cas d'hétérocycles azotés

X - est un groupe $-CH_2-O-$, $O-CH_2-$ ou -O-,

et

B - est un groupe cyano ou un groupe de formule $-SO_2NR^2R^3$ ou $-SO_mR^4$,

où

$R^2$, $R^3$, $R^4$ et m ont la définition indiquée dans la revendication 1,

caractérisé en ce qu'on fait réagir les pyrrolidines substituées en position 3 de formule générale (II)

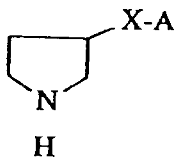

(II)

dans laquelle

A et X ont la définition indiquée ci-dessus, avec l'acrylonitrile ou un composé de formule $CH_2=CH-SO_2NR^2R^3$ ou $-CH_2=CH_2-SO_mR^4$ en présence de catalyseurs.

**8.** Procédé suivant la revendication 7, caractérisé en ce que la réaction est conduite dans la plage de températures de +50°C à +150°C.

**9.** Médicament contenant des pyrrolidines disubstituées en positions 1,3 selon les revendications 1 à 3.

**10.** Médicament suivant la revendication 9, contenant 0,5 à 90 % en poids de pyrrolidines disubstituées en positions 1,3, par rapport au mélange total.

**11.** Utilisation de pyrrolidines disubstituées en positions 1,3 selon les revendications 1 à 3 pour la préparation de médicaments.

**12.** Utilisation suivant la revendication 11, pour la préparation de médicaments destinés au traitement de maladies du système nerveux central.

**Revendications pour l'Etat contractant suivant : ES**

**1.** Procédé de production de pyrrolidines disubstituées en positions 1,3, de formule générale

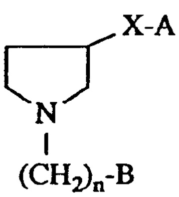

(I)

dans laquelle

A représente un reste phényle ou un reste hétéroaryle monocyclique pentagonal ou hexagonal, qui comprend un ou deux atomes d'azote et/ou d'oxygène et/ou de soufre, auquel sont condensés le cas échéant un à trois hydrocarbures aromatiques cycliques saturés ou non saturés ayant 5 à 8 atomes de carbone ou des hétérocycles pentagonaux ou hexagonaux comportant un ou deux atomes d'azote et/ou d'oxygène et/ou de soufre, ce reste portant éventuellement un ou deux substituants alkyle en $C_1$ à $C_6$, alkoxy en $C_1$ à $C_6$, (alkoxy en $C_1$ à $C_6$)carbonyle, alcényloxy en $C_1$ à $C_6$, acyloxy, benzoyloxy, cyano, phényle, benzyle, fluoro, chloro, bromo, trifluorométhyle, trifluorométhoxy, difluorométhoxy ou étant substitué le cas échéant par un reste de formule -CO-NYZ, $-NH-SO_2-Y'$, $-SO_2NYZ$ ou -NH-CO-Y, où

Y,Z sont identiques ou différents et représentent l'hydrogène ou un groupe alkyle en $C_1$ à $C_6$ et

Y' est un groupe alkyle en $C_1$ à $C_6$ ou aryle ayant 6 à 12 atomes de carbone,

et se présentant le cas échéant sous forme de N-oxyde dans le cas d'hétérocycles azotés

X - représente un groupe $-CH_2-O-$, $-O-CH_2-$ ou -O-,

B - est un groupe cyano ou un groupe de formule $-COOR^1$, $CONR^2R^3$, $-SO_2NR^2R^3$, $-SO_mR^4$, $NR^5R^6$ ou $-C{\equiv}C-CH_2-NR^5R^6$ où

$R^1$ - est l'hydrogène, un groupe alkyle en $C_1$ à $C_{11}$, cycloalkyle en $C_5$ à $C_8$, alcényle en $C_2$ à $C_{12}$, aryle en $C_6$ à $C_{12}$ ou aralkyle en $C_7$ à $C_{14}$,

$R^2$ et $R^3$ sont identiques ou différents et représentent

- l'hydrogène, un groupe alkyle en $C_1$ à $C_{12}$, cycloalkyle en $C_5$ à $C_8$, aryle en $C_6$ à $C_{12}$ ou

aralkyle en $C_7$ à $C_{14}$, les restes aryle pouvant être substitués par un halogène, un radical alkoxy en $C_1$ à $C_6$ ou (alkoxy en $C_1$ à $C_6$)-carbonyle,

$R^4$ - est un groupe alkyle en $C_1$ à $C_{12}$, cycloalkyle en $C_5$ à $C_8$, aryle en $C_6$ à $C_{12}$ ou aralkyle en $C_7$ à $C_{14}$, les restes aryle pouvant porter jusqu'à 3 substituants, identiques ou différents, halogéno, cyano, alkyle en $C_1$ à $C_6$, alkoxy en $C_1$ à $C_6$, trifluorométhyle ou trifluorométhoxy, ou bien

- un groupe de formule

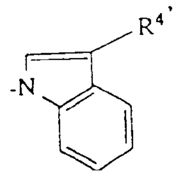

dans laquelle $R^{4'}$ est l'hydrogène ou un radical alkyle en $C_1$ à $C_6$,

m - est le nombre 0, 1 ou 2,

$R^5$ et $R^6$ sont identiques ou différents et représentent

- l'hydrogène, un groupe alkyle en $C_1$ à $C_{12}$, cycloalkyle en $C_5$ à $C_8$, aryle en $C_6$ à $C_{12}$ ou aralkyle en $C_7$ à $C_{14}$, les restes aryle pouvant être substitués par un halogène, un radical cyano, alkyle en $C_1$ à $C_6$, alkoxy en $C_1$ à $C_6$, ou trifluorométhyle, ou bien

- un groupe de formule $-COR^7$ ou $-SO_2R^8$, dans laquelle

$R^7$

- représente l'hydrogène ou
- un groupe $NHR^9$ ou
- un groupe alkyle en $C_1$ à $C_{11}$, cycloalkyle en $C_5$ à $C_8$ ou alkoxy en $C_1$ à $C_{12}$, ou
- un groupe aryle en $C_6$ à $C_{12}$, aryloxy en $C_6$ à $C_{12}$, aralkyle en $C_7$ à $C_{14}$, aralkoxy en $C_6$ à $C_{12}$ ou un noyau hétéroaryle pentagonal ou hexagonal portant un ou deux atomes d'oxygène et/ou d'azote et/ou de soufre, les restes mentionnés pouvant porter 1 à 3 substituants, identiques ou différents, alkyle en $C_1$ à $C_6$, alkoxy en $C_1$ à $C_6$, alkylthio en $C_1$ à $C_6$, halogéno, cyano, trifluorométhyle, trifluorométhoxy, trifluorométhylthio, amino, alkylamino en $C_1$ à $C_6$ ou di(alkyle en $C_1$ à $C_6$)amino,

$R^8$

- est un groupe cycloalkyle en $C_5$ à $C_8$ ou
- alkyle en $C_1$ à $C_{12}$ qui peut être substitué par un radical cyano, halogéno, alkoxy en $C_1$ à $C_{12}$ ou (alkoxy en $C_1$ à $C_{12}$)-carbonyle, ou bien
- un groupe aryle en $C_6$ à $C_{12}$, aralkyle en $C_7$ à $C_{14}$ ou un noyau hétéroaryle pentagonal ou hexagonal portant un ou deux atomes d'oxygène et/ou d'azote et/ou de soufre, les restes mentionnés pouvant porter jusqu'à 3 substituants, identiques ou différents, alkyle en $C_1$ à $C_6$, alkoxy en $C_1$ à $C_6$, alkylthio en $C_1$ à $C_6$, halogéno, cyano, trifluorométhyle, trifluorométhoxy, trifluorométhylthio, nitro, amino, (alkyle en $C_1$ à $C_6$)-amino, di(alkyle en $C_1$ à $C_6$)-amino,

ou

- un groupe $NR^2R^3$, dans lequel

$R^2$ et $R^3$ ont la définition indiquée ci-dessus et

$R^9$

- représente l'hydrogène ou
- un groupe cycloalkyle en $C_5$ à $C_8$ ou
- un groupe alkyle en $C_1$ à $C_{12}$ portant facultativement un substituant cyano, fluoro, chloro ou bromo, ou
- un groupe aryle en $C_6$ à $C_{12}$, aralkyle en $C_7$ à $C_{14}$, un noyau hétéroaryle pentagonal ou hexagonal comportant 1 ou 2 atomes d'oxygène et/ou d'azote et/ou de soufre, les restes aryle pouvant porter jusqu'à 3 substituants, identiques ou différents, alkyle en $C_1$ à $C_6$, alkoxy en $C_1$ à $C_6$, alkylthio en $C_1$ à $C_6$, halogéno, cyano, trifluorométhyle, trifluorométhoxy, trifluorométhylthio, nitro, amino, alkylamino en $C_1$ à $C_6$, ou di(alkyle en $C_1$ à $C_6$)-amino,

ou bien

$R^5$ et $R^6$ forment conjointement avec l'atome d'azote un noyau de la série

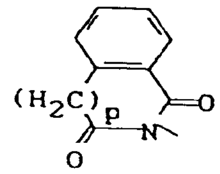 , 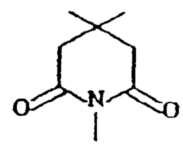 , 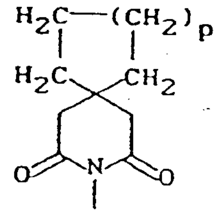 ,

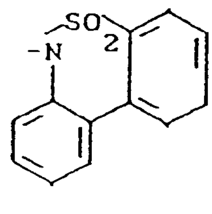 , 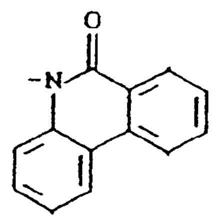 , 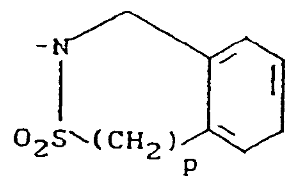 ,

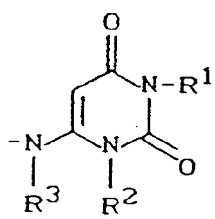 , 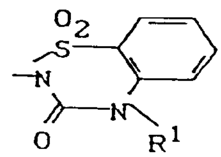 , 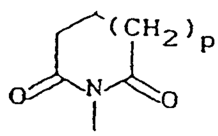 , 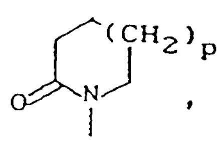 ,

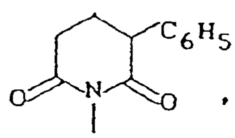 , 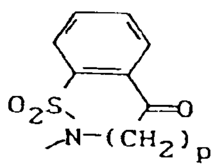 , 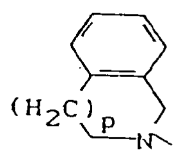 , 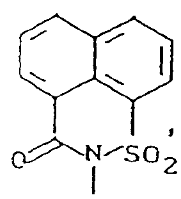

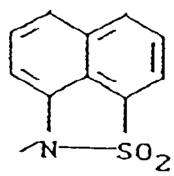 , 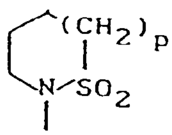 ou 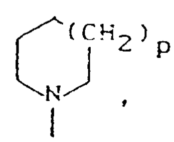 ,

où

p - est le nombre 0, 1 ou 2,

$R^1$, $R^2$ ou $R^3$ ont la définition indiquée ci-dessus

n - est un nombre de 1 à 10, et de leurs sels, caractérisé en ce qu'on fait réagir des pyrrolidines substituées en position 3 de formule générale (II)

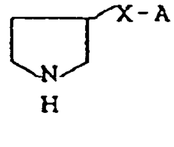 (II)

dans laquelle A et X ont la définition indiquée ci-dessus, ou leurs sels

dans une première étape, avec des dérivés alkyliques de formule générale (III)

$$R\text{-}(CH_2)_n\text{-}B' \qquad (III)$$

dans laquelle

n - a la définition indiquée ci-dessus

B' - correspond au cadre de définition de B, toutefois $R^5$ et $R^6$ ne représentent pas simultanément l'hydrogène ou simultanément l'hydrogène et un groupe alkyle ou aryle,

R - représente le chlore, le brome, l'iode, un groupe méthylsulfonyloxy, phénylsulfonyloxy, tolylsulfonyloxy, trifluoracétoxy ou trifluorométhylsulfonyloxy

dans des solvants inertes, éventuellement en présence de bases,

puis on transforme le cas échéant des groupes fonctionnels par réduction, oxydation, hydrolyse ou réaction avec des réactifs nucléophiles ou électrophiles, en d'autres groupes fonctionnels, après quoi dans le cas de la production des sels, on les fait réagir le cas échéant avec un acide correspondant.

**2.** Procédé suivant la revendication 1, pour la production de composés de formule (I), dans laquelle

A - est un reste phényle ou un reste hétéroaryle monocyclique pentagonal ou hexagonal qui contient un ou deux atomes d'azote et/ou d'oxygène et/ou de soufre, auquel sont condensés le cas échéant un à trois hydrocarbures aromatiques cycliques saturés ou non saturés ayant 5 à 8 atomes de carbone ou des hétérocycles pentagonaux ou hexagonaux ayant un ou deux atomes d'azote et/ou d'oxygène et/ou de soufre, ce reste étant éventuellement substitué une ou deux fois par un radical alkyle $C_1$ à $C_6$, alkoxy en $C_1$ à $C_6$, (alkoxy en $C_1$ à $C_6$)carbonyle, alcényloxy en $C_1$ à $C_6$, acyloxy, benzoyloxy, cyano, phényle, benzyle, fluoro, chloro, bromo, trifluorométhyle, trifluorométhoxy, difluorométhoxy ou un reste de formule -CO-NYZ, $-NH-SO_2-Y'$, $-SO_2NYZ$ ou -NH-CO-Y,

Y, Z sont identiques ou différents et représentent l'hydrogène ou un groupe alkyle en $C_1$ à $C_6$ et

Y' est un groupe alkyle en $C_1$ à $C_6$ ou aryle ayant 6 à 12 atomes de carbone,

et dans le cas d'hétérocycles azotés, se présente éventuellement sous forme de N-oxyde,

X - représente un groupe $-CH_2-O-$, $-O-CH_2-$ ou -O-,

B - est un groupe cyano ou un groupe de formule $-COOR^1$, $CONR^2R^3$, $-SO_2NR^2R^3$, $-SO_mR^4$, $NR^5R^6$ ou $-C{\equiv}C-CH_2-NR^5R^6$ où

$R^1$ - est l'hydrogène, un groupe alkyle en $C_1$ à $C_6$ ou phényle,

$R^2$ et $R^3$ sont identiques ou différents et représentent

- l'hydrogène un groupe alkyle en $C_1$ à $C_6$ ou phényle qui peut être substitué quant à lui par du fluor, du chlore, du brome, un radical alkoxy en $C_1$ à $C_6$ ou (alkoxy en $C_1$ à $C_6$)carbonyle,

$R^4$ - est un groupe alkyle en $C_1$ à $C_6$ ou

- un groupe phényle qui peut porter jusqu'à deux substituants, identiques ou différents, fluoro, chloro, bromo, alkyle en $C_1$ à $C_6$, alkoxy en $C_1$ à $C_6$, ou bien

- un groupe de formule

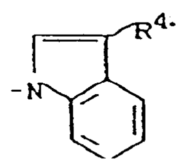

dans laquelle $R^{4\cdot}$ représente l'hydrogène ou un groupe alkyle en $C_1$ à $C_6$,

m - est le nombre 0, 1 ou 2,

$R^5$ et $R^6$ sont identiques ou différents et représentent

- l'hydrogène, un groupe alkyle en $C_1$ à $C_6$, phényle ou benzyle, les restes phényle pouvant être substitués par du fluor, du chlore, du brome, un radical alkyle en $C_1$ à $C_6$, alkoxy en $C_1$ à $C_6$ ou trifluorométhyle, ou bien

- un groupe de formule $-COR^7$ ou $-SO_2R^8$,

où

$R^7$

- représente l'hydrogène ou
- un groupe $NHR^9$ ou
- un groupe alkyle en $C_1$ à $C_6$ ou alkoxy en $C_1$ à $C_6$ ou
- un groupe phényle, benzyle, benzyloxy, thiényle, furyle, pyridyle, pyrimidyle, quinolyle, isoquinolyle, benzothiazolyle, benzoxazolyle, thiazolyle, oxazolyle, isoxazolyle ou isothiazolyle éventuellement substitué par un radical alkyle en $C_1$ à $C_6$, alkoxy en $C_1$ à $C_6$, fluoro, chloro, bromo, trifluorométhyle, diméthylamino ou diéthylamino,

$R^8$

- est un groupe cyclopropyle, cyclopentyle, cyclohexyle ou

- un groupe alkyle en $C_1$ à $C_6$ portant éventuellement un substituant cyano, fluoro, chloro, bromo, trifluorométhyle ou (alkoxy en $C_1$ à $C_6$)-carbonyle, ou bien
- un groupe phényle, naphtyle, benzyle, thiényle, furyle, pyrimidyle, pyridyle, quinolyle, isoquinolyle, benzothiazolyle, benzoxazolyle, thiazolyle, oxazolyle, isoxazolyle ou isothiazolyle éventuellement substitué par un radical alkyle en $C_1$ à $C_6$, alkoxy en $C_1$ à $C_6$, fluoro, chloro, bromo, nitro, trifluorométhyle, diméthylamino ou diéthylamino, ou bien
- un groupe $NR^2R^3$, dans lequel

$R^2$ et $R^3$ ont la définition indiquée ci-dessus et

$R^9$

- est un groupe alkyle en $C_1$ à $C_6$ éventuellement substitué par un radical cyano, fluoro, chloro ou bromo, ou bien
- un groupe phényle, benzyle, thiényle, furyle, pyridyle, pyrimidyle, quinolyle, isoquinolyle, benzothiazolyle, benzoxazolyle, thiazolyle, oxazolyle, isoxazolyle ou isothiazolyle portant éventuellement un substituant alkyle en $C_1$ à $C_6$, alkoxy en $C_1$ à $C_6$, fluoro, chloro, bromo, trifluorométhyle, diméthylamino ou diéthylamino,

ou bien

$R^5$ et $R^6$ forment conjointement avec l'atome d'azote un noyau de la série

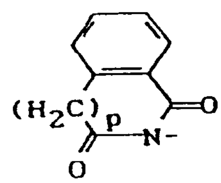 , 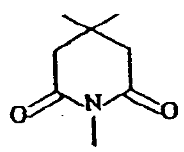 , 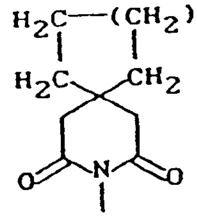 ,

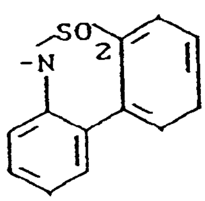 , 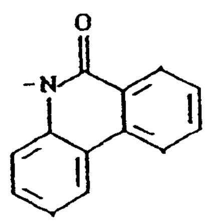 , 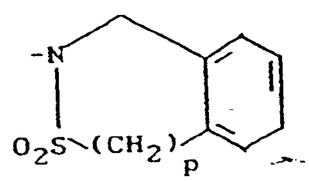 ,

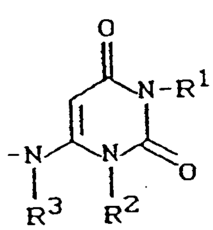 , 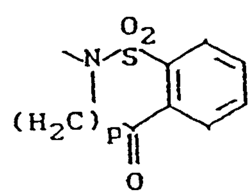 , 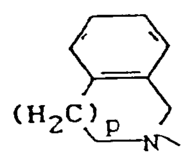 , 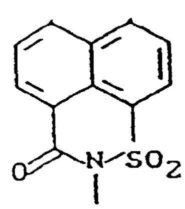

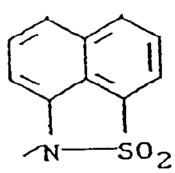 , 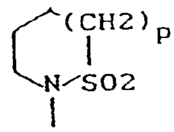 , 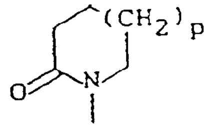 ,

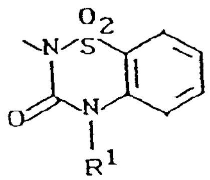

ou

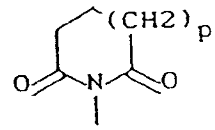

où
p - est le nombre 0, 1 ou 2,
$R^1$, $R^2$ et $R^3$ ont la définition indiquée ci-dessus et
n - est un nombre de 1 à 8, et de leurs sels.

3. Procédé suivant la revendication 1 pour la production de composés de formule (I), dans laquelle
A - représente un groupe phényle ou naphtyle portant éventuellement des substituants, identiques ou différents, alkyle en $C_1$ à $C_6$, alkoxy en $C_1$ à $C_6$, (alkoxy en $C_1$ à $C_6$)carbonyle, alcényloxy en $C_1$ à $C_6$, acyloxy, benzoyloxy, cyano, phényle, benzyle, sulfonylamino, sulfamoyle (en $C_1$ à $C_6$), carbamoyle, carbonylamino, fluoro, chloro, bromo, trifluorométhyle, trifluorométhoxy, difluorométhoxy, un groupe furanne, thiophène, isoxazole, pyridine, pyrimidine, indole, indazole, benzofuranne, benzisoxazole, quinoléine, isoquinoléine, tétraline, indène, chromane, dihydrobenzodioxine, dihydroindole, tétrahydroquinoléine ou dihydrobenzofuranne,
X - représente $-O-CH_2-$, $-CH_2-O-$ ou -O-,
B
- représente un groupe cyano ou
- un groupe de formule
$-CONR^2R^3$, $-NR^5R^6$, $-SO_mR^4$, $-C{\equiv}C-CH_2-NR^5R^6$, $-SO_2NR^2R^3$, $COOR^1$, où
$R^1$ - est l'hydrogène, un groupe méthyle, éthyle ou phényle,
$R^2$ et $R^3$ sont identiques ou différents et représentent
- l'hydrogène, un groupe méthyle, éthyle, propyle, isopropyle, butyle, isobutyle ou un groupe phényle qui peut être substitué par un radical méthoxycarbonyle,
$R^4$ - est un groupe méthyle ou éthyle ou bien
- un groupe de formule

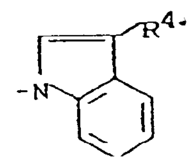

dans laquelle $R^{4\cdot}$ est l'hydrogène ou un groupe méthyle,
$R^5$ et $R^6$ sont identiques ou différents et représentent
- l'hydrogène, un groupe méthyle, éthyle, propyle, isopropyle, butyle, isobutyle ou
- un groupe phényle portant éventuellement un substituant fluoro, chloro, méthyle ou méthoxy, ou bien
- un groupe $-COR^7$ ou $-SO_2R^8$, où
$R^7$
- est un groupe $NHR^9$, ou bien
- un groupe méthyle, éthyle, propyle, isopropyle, méthoxy, éthoxy, propoxy, isopropoxy, tertio-butoxy, ou bien
- un groupe phényle, benzyle, benzyloxy, thiényle, furyle, pyridyle, pyrimidyle, quinolyle ou isoquinolyle portant éventuellement un substituant méthyle, méthoxy, fluoro ou chloro,
$R^8$
- est un groupe méthyle, éthyle, propyle, isopropyle, butyle ou isobutyle portant éventuellement un substituant fluoro, chloro, méthoxycarbonyle, éthoxycarbonyle, propoxycarbonyle, isoproproxycarbonyle, butoxycarbonyle, isobutoxycarbonyle ou tertio-butoxycarbonyle, ou bien
- un groupe phényle, naphtyle, thiényle, furyle, pyridyle, pyrimidyle, quinolyle, ou isoquinolyle portant le cas échéant un substituant méthyle, éthyle, propyle, isopropyle, méthoxy, fluoro, chloro, nitro,
- ou bien un groupe $NR^2R^3$, où

$R^2$ et $R^3$ ont la définition indiquée ci-dessus,

$R^9$

- est un groupe méthyle, éthyle, propyle, isopropyle, butyle, isobutyle, pentyle, isopentyle, hexyle ou isohexyle éventuellement substitué par du fluor ou du chlore, ou bien
- un groupe phényle qui peut être substitué par un radical fluoro, chloro, méthyle ou méthoxy,

$R^5$ et $R^6$ forment conjointement avec l'atome d'azote un noyau de la série

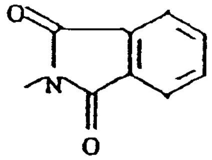
,
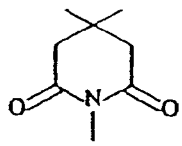
,
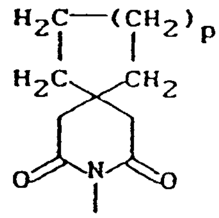
,

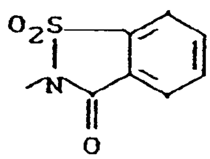
,
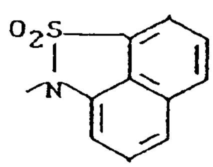
,
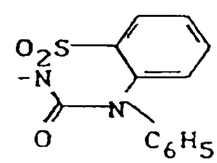
,

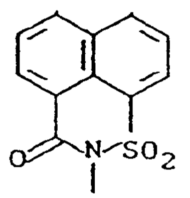
,
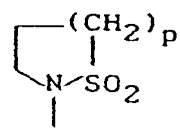
,
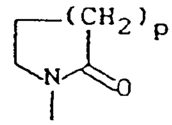
,

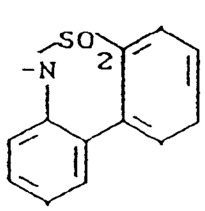
,
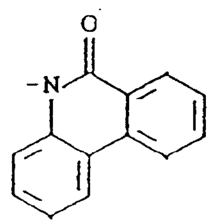
,
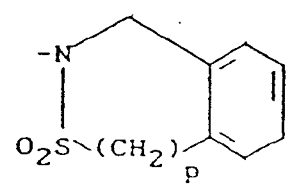
,

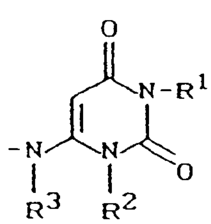
ou
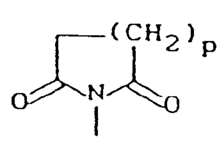

p - a la valeur 1 ou 2,

$R^1$, $R^2$ et $R^3$ ont la définition indiquée ci-dessus et

n - est un nombre de 1 à 6,

et de leurs sels.

4. Procédé suivant la revendication 1, caractérisé en ce qu'on opère dans une plage de températures de -20°C

à +150°C.

**5.** Procédé de production de pyrrolidines disubstituées en positions 1,3 de formule générale

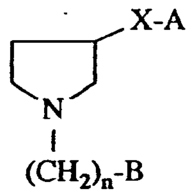

(I)

dans laquelle

A est un reste phényle ou un reste hétéroaryle monocyclique pentagonal ou hexagonal qui comporte un ou deux atomes de carbone et/ou d'oxygène et/ou de soufre, auquel sont condensés le cas échéant 1 à 3 hydrocarbures aromatiques cycliques saturés ou non saturés ayant 5 à 8 atomes de carbone ou hétérocycles pentagonaux ou hexagonaux ayant 1 ou 2 atomes d'azote et/ou d'oxygène et/ou de soufre, ce reste étant substitué le cas échéant une ou deux fois par un radical alkyle en $C_1$ à $C_6$, alkoxy en $C_1$ à $C_6$, (alkoxy en $C_1$ à $C_6$)carbonyle, alcényloxy en $C_1$ à $C_6$, acyloxy, benzoyloxy, cyano, phényle, benzyle, fluoro, chloro, bromo, trifluorométhyle, trifluorométhoxy, fluorométhoxy ou un reste de formule -CO-NYZ, $-NH-SO_2-Y'$, $-SO_2NYZ$ ou -NH-CO-Y,

Y, Z sont identiques ou différents et représentent l'hydrogène ou un groupe alkyle en $C_1$ à $C_6$ et

Y' est un groupe alkyle en $C_1$ à $C_6$ ou aryle ayant 6 à 12 atomes de carbone,

et se présente éventuellement sous forme du N-oxyde dans le cas d'hétérocycles azotés,

X - est un groupe $-CH_2-O-$, $O-CH_2-$ ou -O-,

et

B - est un groupe cyano ou un groupe de formule $-SO_2NR^2R^3$, ou $-SO_mR^4$,

où

$R^2$, $R^3$, $R^4$ et m ont la définition indiquée dans la revendication 1,

caractérisé en ce qu'on fait réagir les pyrrolidines substituées en position 3 de formule générale (II)

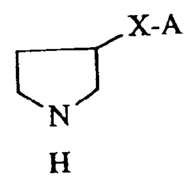

(II)

dans laquelle

A et X ont la définition indiquée ci-dessus, avec l'acrylonitrile ou un composé de formule $CH_2=CH-SO_2NR^2R^3$ ou $-CH_2=CH_2-SO_mR^4$ en présence de catalyseurs.

**6.** Procédé suivant la revendication 5, caractérisé en ce qu'on conduit la réaction dans un intervalle de températures de +50°C à +150°C.